# EUROPEAN PATENT SPECIFICATION

(11) **EP 2 326 734 B1**
(45) Date of publication and mention of the grant of the patent: **09.11.2016**
(21) Application number: 09812193.2
(22) Date of filing: 03.09.2009
(51) Int. Cl.: C12Q 1/68

(54) **PATHWAYS UNDERLYING PANCREATIC TUMORIGENESIS AND AN HEREDITARY PANCREATIC CANCER GENE**
SIGNALWEGE DER PANKREASTUMORGENESE UND HEREDITÄRES PANKREASKREBSGEN
VOIES À L ORIGINE DE LA TUMORIGENÈSE PANCRÉATIQUE ET GÈNE HÉRÉDITAIRE DU CANCER PANCRÉATIQUE

(30) Priority: 03.09.2008 US 93844 P; 05.03.2009 US 157700 P
(43) Date of publication of application: 01.06.2011
(73) Proprietor: The Johns Hopkins University, Baltimore, MD 23218 (US)
(72) Inventor: VOGELSTEIN, Bert, Baltimore Maryland 21208 (US); KINZLER, Kenneth, W., Baltimore Maryland 21015 (US); PARSONS, Donald William, Bellaire, TX 77401 (US); JONES, Sian, Maryland 21201 (US); ZHANG, Xiaosong, Maryland 21234 (US); LIN, Jimmy Chang-Ho, Baltimore Maryland 21205 (US); LEARY, Rebecca J., Maryland 21201 (US); ANGENENDT, Philipp, 22523 Hamburg (DE); PAPADOPOULOS, Nickolas, Towson Maryland 21204 (US); VELCULESCU, Victor, Dayton Maryland 21036 (US); PARMIGIANI, Giovanni, Baltimore Maryland 21230 (US); KARCHIN, Rachel, Towson Maryland 21204 (US); KERN, Scott, Hunt Valley Maryland 21030 (US); HRUBAN, Ralph, Baltimore Maryland 21212 (US); ESHLEMAN, James R., Lutherville Maryland 21093 (US); GOGGINS, Michael, Baltimore Maryland 21210 (US); KLEIN, Alison, Baltimore Maryland 21212 (US)
(74) Representative: Tombling, Adrian George
(86) International application number: PCT/US2009/055802
(87) International publication number: WO 2010/028098

(56) References cited:
- WO-A1-2007/148997
- WO-A2-2005/053512
- WO-A2-2008/047128
- US-A1- 2005 158 718
- SARAH REID ET AL: "Biallelic mutations in PALB2 cause Fanconi anemia subtype FA-N and predispose to childhood cancer", NATURE GENETICS, vol. 39, no. 2, 1 February 2007 (2007-02-01), pages 162-164, XP55027643, ISSN: 1061-4036, DOI: 10.1038/ng1947
- HAHN STEPHAN A ET AL: "BRCA2 germline mutations in familial pancreatic carcinoma.", JOURNAL OF THE NATIONAL CANCER INSTITUTE (BETHESDA), vol. 95, no. 3, 5 February 2003 (2003-02-05), pages 214-221, XP002676404, ISSN: 0027-8874
- COUCH FERGUS J ET AL: "Germ line Fanconi anemia complementation group C mutations and pancreatic cancer", CANCER RESEARCH, vol. 65, no. 2, 15 January 2005 (2005-01-15), pages 383-386, XP002676535, ISSN: 0008-5472
- S. JONES ET AL: "Core Signaling Pathways in Human Pancreatic Cancers Revealed by Global Genomic Analyses", SCIENCE, vol. 321, no. 5897, 26 September 2008 (2008-09-26), pages 1801-1806, XP55015968, ISSN: 0036-8075, DOI: 10.1126/science.1164368
- MCWILLIAMS ROBERT R ET AL: "Polymorphic variants in hereditary pancreatic cancer genes are not associated with pancreatic cancer risk.", CANCER EPIDEMIOLOGY, BIOMARKERS & PREVENTION : A PUBLICATION OF THE AMERICAN ASSOCIATION FOR CANCER RESEARCH, COSPONSORED BY THE AMERICAN SOCIETY OF PREVENTIVE ONCOLOGY SEP 2009 LNKD- PUBMED:19690177, vol. 18, no. 9, September 2009 (2009-09), pages 2549-2552, XP002676405, ISSN: 1538-7755
- JONES SIAN ET AL: "Exomic Sequencing Identifies PALB2 as a Pancreatic Cancer Susceptibility Gene", SCIENCE (WASHINGTON D C), vol. 324, no. 5924, April 2009 (2009-04), page 217, XP002676406, ISSN: 0036-8075
- SJOBLOM ET AL.: 'The Consensus Coding Sequences of Human Breast and Colorectal Cancers' SCIENCE vol. 314, no. 5797, 13 October 2006, pages 268 - 274, XP007901261
- ERKKO ET AL.: 'A recurrent mutation in PALB2 in Finnish cancer families' NATURE vol. 446, no. 7133, 15 March 2007, pages 316 - 319, XP008145272
- MALKIN ET AL.: 'Germ Line p53 Mutations in a Familial Syndrome of Breast Cancer, Sarcomas and Other Neoplasms' SCIENCE vol. 250, no. 4985, 30 November 1990, pages 1233 - 1238, XP008145273
- DATABASE EMBL [Online] 16 April 2005 'S216P61126FH2.T0 Masuku Pan troglodytes troglodytes STS genomic, sequence tagged site.' Retrieved from EBI, accession no. EM_STS:BV617345 Database accession no. BV617345
- DATABASE EMBL [Online] 16 March 2007 '1095515635721 Global-Ocean-Sampling_GS-32-01-01-1P3-1P6KB marine metagenome genomic clone 1061005927269 3', genomic survey sequence.' Retrieved from EBI, accession no. EM_GSS:EK524042 Database accession no. EK524042

### SEQUENCE LISTING

<110> VOGELSTEIN, Bert KINZLER, Kenneth W. PARSONS, D. Williams JONES, Sian ESHLEMAN, James VELCULESCU, Victor GOGGINS, Michael HRUBAN, Ralph KERN, Scott ZHANG, Xiaosong LIN, Jimmy Chang-Ho LEARY, Rebecca J. ANGENENDT,Philipp PAPADOPOULOS, Nickolas PARMIGIANI, Giovanni KARCHIN, Rachel KLEIN, Alison
<120> PATHWAYS UNDERLYING PANCREATIC
   TUMORIGENESIS AND AN HEREDITARY PANCREATIC CANCER GENE
<130> 001107.00787
<150> 61/157700
   <151> 2009-03-05
<150> 61/093844
   <151> 2008-09-03
<160> 1
<170> FastSEQ for Windows Version 4.0
<210> 1
   <211> 17
   <212> DNA
   <213> Bacteriophage M13
<400> 1
   gtaaaacgac ggccagt 17

## Description

This invention was made using funds from the United States government. The U.S. government retains certain rights in the invention according to the terms of NIH grants CA 43460, CA 57345, CA 62924, CA123483, RO1CA97075, and CA 121113.

### TECHNICAL FIELD OF THE INVENTION

This invention is related to the area of pancreatic cancer. In particular, it relates to diagnosis, treatment, characterization, monitoring, detection, and stratification of pancreatic cancers.

### BACKGROUND OF THE INVENTION

Worldwide, 213,000 patients will develop pancreatic cancer in 2008 and nearly all will die of their disease (*1*). The mortality is so high in part because the disease is generally not detected until it has already spread locally or metastasized to the liver, peritoneum, or other organs. This tumor strikes men and women relatively equally and the overall survival rate is less than 5% even with the aggressive treatments used in the western world (*2, 3*). Though there are modest associations with cigarette smoking, long-standing chronic pancreatitis and certain diets, little is known about the mechanisms through which environmental factors lead to pancreatic neoplasia. Similarly,~ 10% of pancreatic cancer patients appear to have familial predispositions to the disease. Though a small fraction of these patients harbor germline mutations of *BRCA2, CDKN2A, LKB1, PRSS1, STK11 or MSH2,* the gene(s) responsible for the vast majority of patients with familial predispositions to pancreatic cancers have not yet been discovered (*4*).

Pancreatic tumors appear to proceed through several intermediate stages, much like those of colorectal tumors. The non-invasive stages that precede invasive cancer are called pancreatic intraepithelial neoplasias (PanINs) and are associated with progressive dysplasia evident upon histopathological examination (*5*). Several genetic alterations have been identified in these lesions as well as in the fully invasive carcinomas that eventually develop from them *(6-10).* The genes altered include the *CDKN2A, SMAD4* and *TP53* tumor suppressor genes as well as the *KRAS* oncogene, each of which has been found to be mutated in a substantial fraction of late stage cancers and in variable fractions of pre-invasive neoplasms. The discoveries of these genes have provided unique insights into the natural history of the disease and have spurred efforts to develop improved diagnostic and therapeutic agents (*11*).

There is a continuing need in the art to understand the genetic make-up of pancreatic cancers in detail. There is a continuing need for additional genes and pathways that are associated with and important for pancreatic cancers.

### SUMMARY OF THE INVENTION

Disclosed herein is a method of identifying an individual who has a predisposition to a disease. One performs sequencing reactions upon a plurality of exons of protein coding genes of template nucleic acid derived from a tissue of the individual. One compares sequences of the plurality of exons of the individual to sequences of individuals without the disease to identify a mutant allele in a protein coding gene of the individual that is not present in individuals without the disease. Presence of the mutant allele indicates that the individual is predisposed to the disease.

Also disclosed is a method of identifying genes which are involved in hereditary cancers. One performs sequencing reactions on template nucleic acid of at least the exons of protein coding genes. The template nucleic acid is derived from a tumor of a first human individual who has a familial cancer. One identifies a protein coding gene in the tumor for which no wild-type allele is present. One performs sequencing reactions on template nucleic acid of the protein coding gene in a plurality of human individuals who have a familial cancer of the same organ as the first human individual. One identifies one or more mutant alleles in the protein coding gene in the plurality which are distinct from alleles in the first human individual, thereby confirming the protein coding gene as conferring susceptibility to the familial cancer.

Specifically, one aspect of the invention is a method of determining susceptibility to pancreatic cancer, comprising:
testing a sample obtained from an individual for the presence of a mutation in the *PALB2* gene found in a family member of the individual;
identifying the individual as being at increased risk of developing pancreatic cancer when the mutation is present and identifying the individual as being at normal risk when the mutation is not present, and wherein the mutation is a truncating mutation.

An aspect of the invention is a method of determining susceptibility to pancreatic cancer in an individual. One performs sequencing reactions of PALB2 gene sequences on template nucleic acid from the individual. One identifies a mutation in the PALB2 sequences, whereby one identifies the individual as at increased susceptibility to pancreatic cancer, and wherein the mutation is a truncating mutation.

Another aspect of the invention is a method of determining susceptibility to pancreatic cancer in an individual. One hybridizes a nucleic acid primer or probe comprising a PALB2 sequence of at least 18 nucleotides wherein the sequence comprises a mutation selected from the group consisting of: del TTGT 172-175, G>T at IVS5-1, del A at 3116, and C>T at 3256, to PALB2 gene sequences in nucleic acid from the individual. One identifies one of said mutations in the PALB2 sequences of the individual, whereby one identifies the individual as at increased susceptibility to pancreatic cancer.

These and other embodiments which will be apparent to those of skill in the art upon reading the specification provide the art with tools and methods for characterizing, treating, prognosing, diagnosing, and stratifying pancreatic cancers.

### BRIEF DESCRIPTION OF THE DRAWINGS

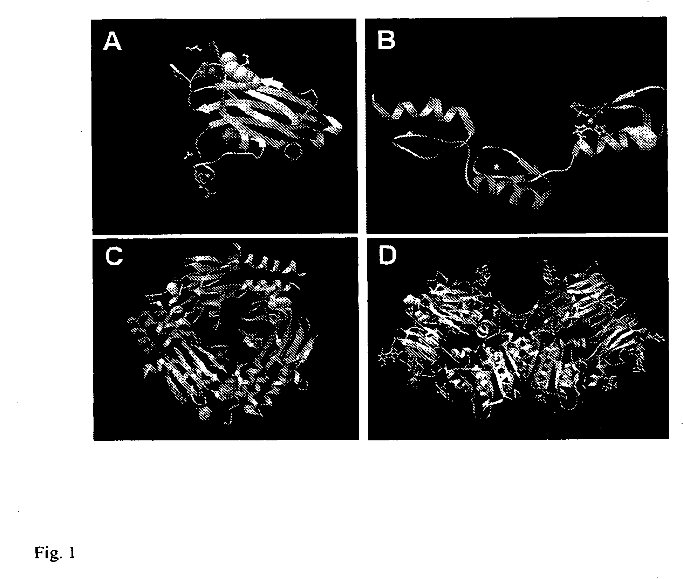

Fig. 1A-1D. Examples of structural models of mutations. (Fig. IA). The X-ray crystal structure of the C2 domain of Protein Kinase C (PKC) gamma (PDBID: 2UZP). Arg252 is shown as large space-fills, Ca2+ ions are shown as smaller spheres. The ligands 1,2-Ethanediol and Pyridoxal-5'-phosphate are shown as ball and stick representations. The R252H mutation could reduce the membrane binding of the C2 domain of PRKCG and thereby affect function. (Fig. 1 B) The NMR solution structure of the three tandem repeats of zf-C2H2 domains from human Kruppel-like factor 5 (KLF5) (PDBID: 2EBT). His389 is shown as space-fills, Zn2+ ions are shown as small spheres. The residues comprising the C2H2 group that coordinate the nearby Zn2+ ion, H393 and H397 are shown as ball and stick representations, and Cys380 and Cys375 are shown as ball and stick representations. The mutation at position 389 (H389N) may disrupt the structure of the zinc finger or nearby zinc coordination site. (Fig. 1C) The X-ray crystal structure of the heterotrimer of SMAD3 (two subunits shown as almost vertical ribbons) and SMAD4 (one subunit shown as horizontal ribbons) (PDBID: 1U7F). The residues corresponding to two of the mutant positions (F260S and S422F, shown as space-fills, in chain A), are located at interfaces and could perturb Smad3-Smad3 or Smad3-Smad4 interactions. In chain B, F260 and S422 are shown as space-fills. (Fig. ID) The X-ray crystal structure of the extracellular domain of human DPP6 as a homodimer (PDBID: 1XFD). Two of the mutated residues found in this study, T409I and D475N (shown as space-fills) are in spatial proximity and are close to one of the glycosylation sites, Asn471 (shown as space-fills). These mutations fall in the β-propeller domain of the protein (residues 142-322 and 351-581) thought to be involved in protein-protein interactions. The A778T mutation (shown as space-fills) falls in the σ/β hydrolase domain (residues 127-142 and 581 to 849) and is close to the homodimer region of the protein and could perturb the homodimer association. Carbohydrates with glycosylation sites are shown in stick representation.

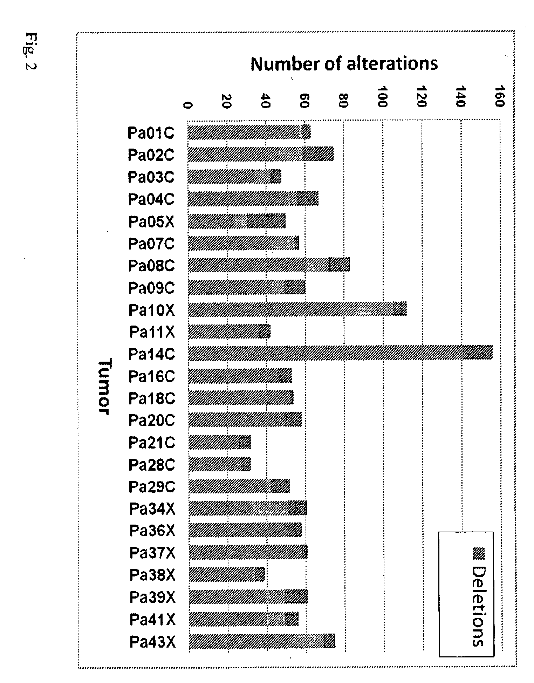

Fig. 2. Number of genetic alterations detected through sequencing and copy number analyses of each of the 24 cancers. Bottom of bar represent mutations, middle of bar, represents amplifications, and top of bar represents deletions.

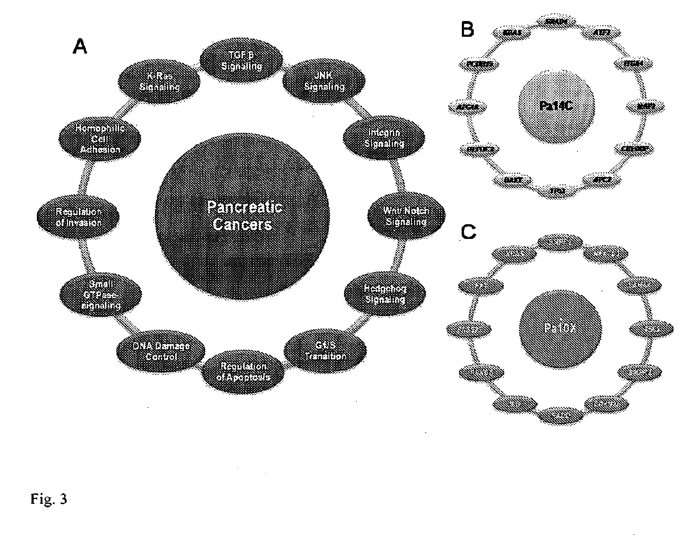

Fig. 3A-3C. Pathways and regulatory processes. (Fig. 3A) The 12 pathways and processes whose component genes were genetically altered in most pancreatic cancers. (Fig. 3B, Fig. 3C) Two pancreatic cancers (Pal4C and Pa10X) and the specific genes that are mutated in them. The positions around the circles in (Fig. 3B) and (Fig. 3C) correspond to the pathways and processes in (Fig. 3A). Several pathway components overlapped, as illustrated by the BMPR2 mutation that presumably disrupted both the SMAD4 and Hedgehog signaling pathways in Pa10X. Additionally, not all 12 processes and pathways were altered in every pancreatic cancer, as exemplified by the fact that no mutations known to affect DNA damage control were observed in Pa10X (N.O., not observed).

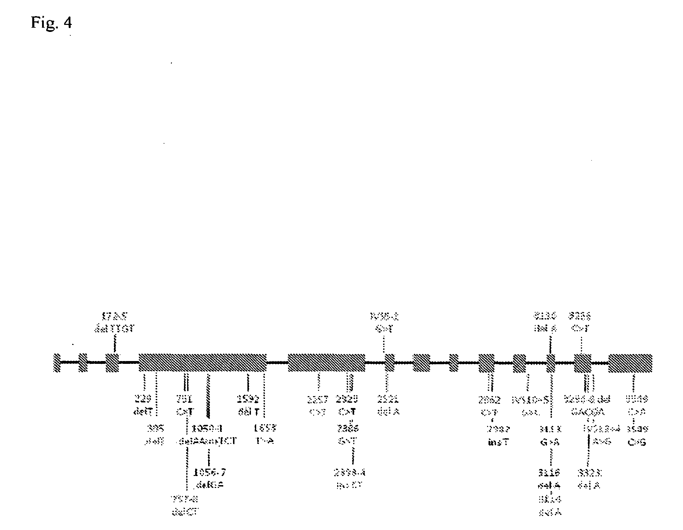

Fig. 4. Location of mutations in the ***PALB2*** gene. Exons are represented as boxes and introns as black lines (not to scale). Mutations previously identified in patients with familial breast cancer or Fanconi Anemia are shown below the gene. Germline mutations identified in patients with familial pancreatic cancer are shown above the gene.

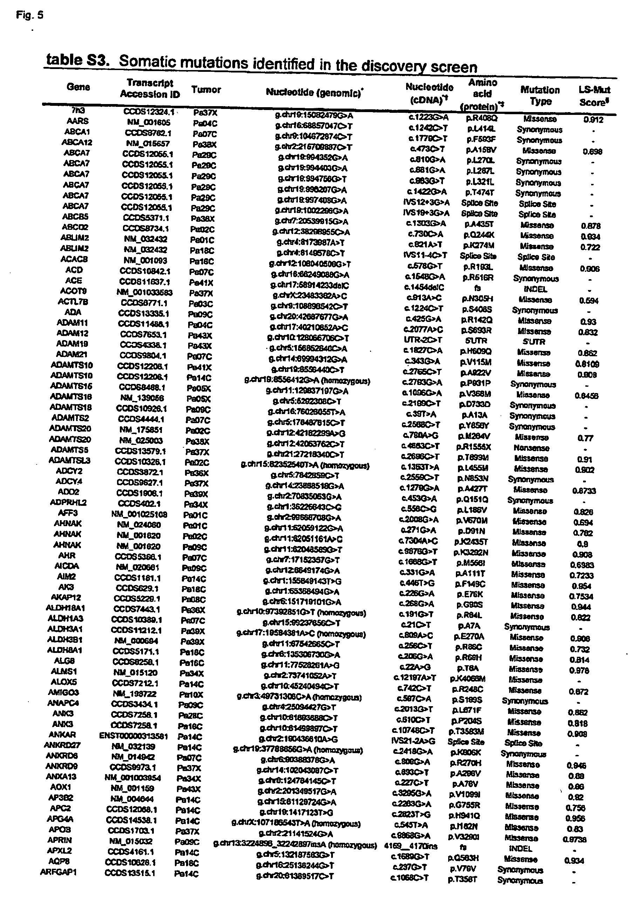

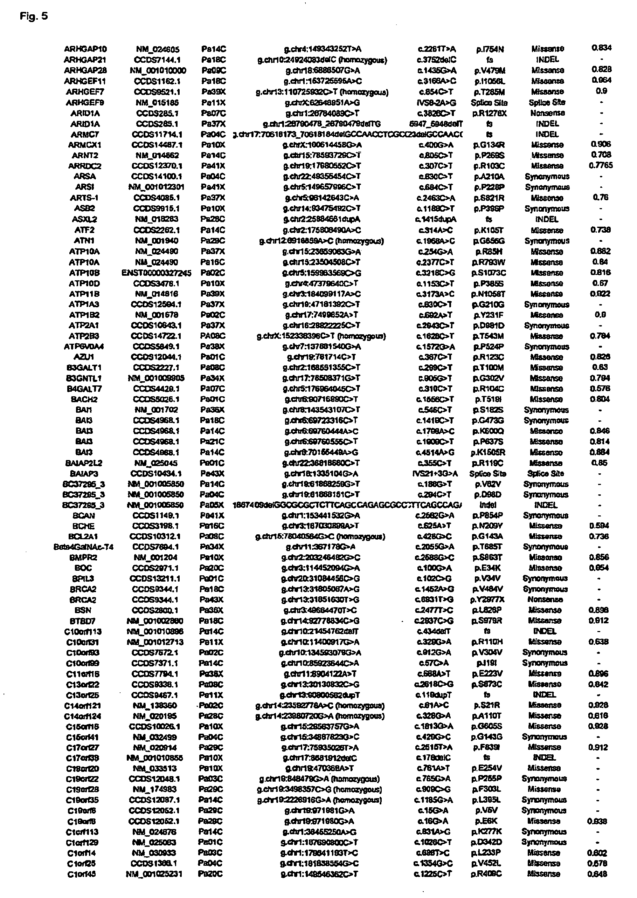

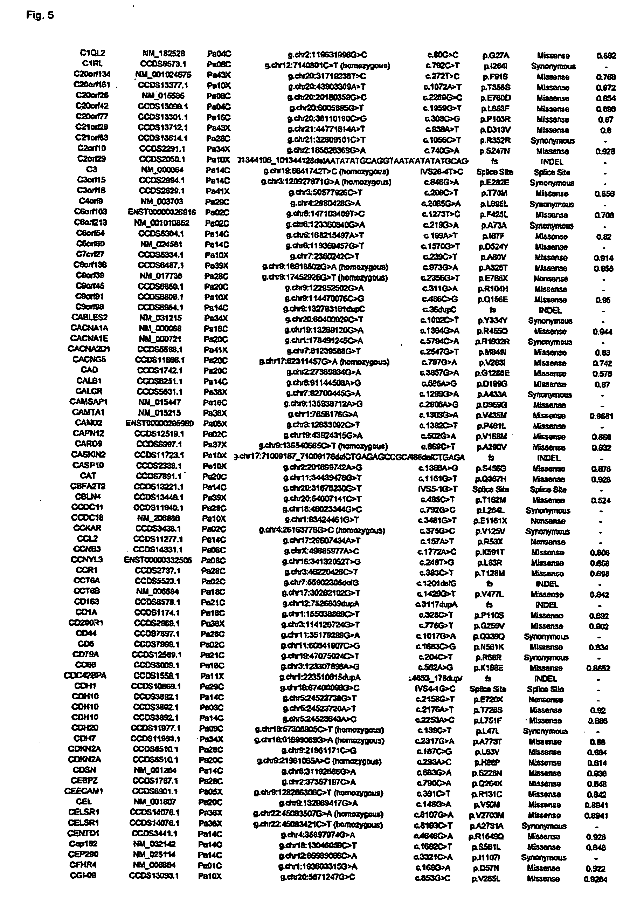

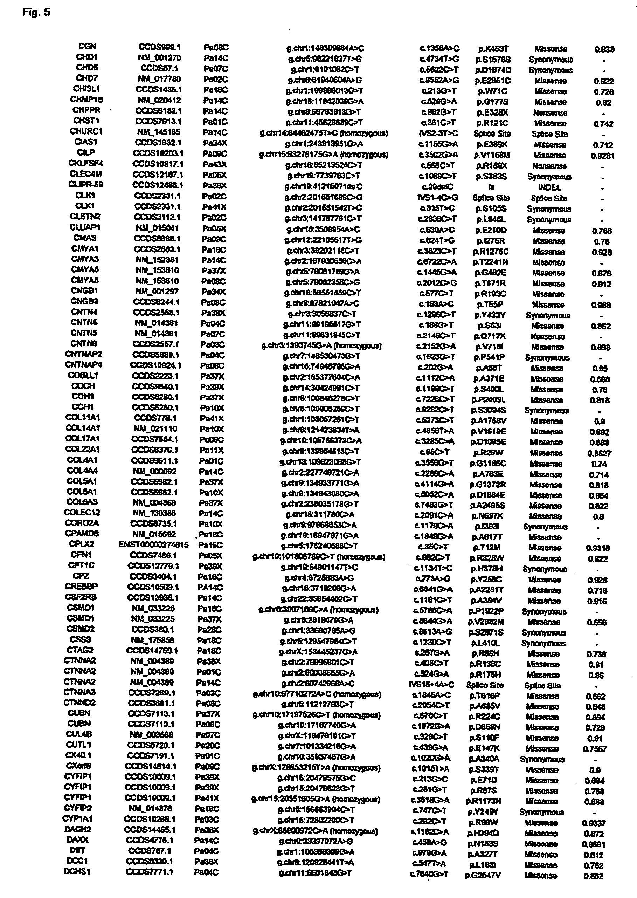

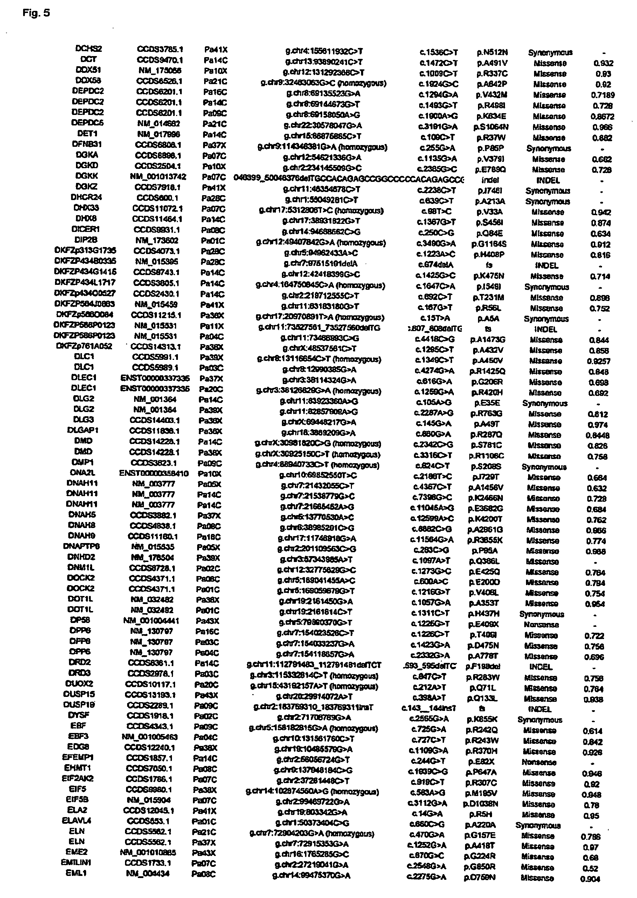

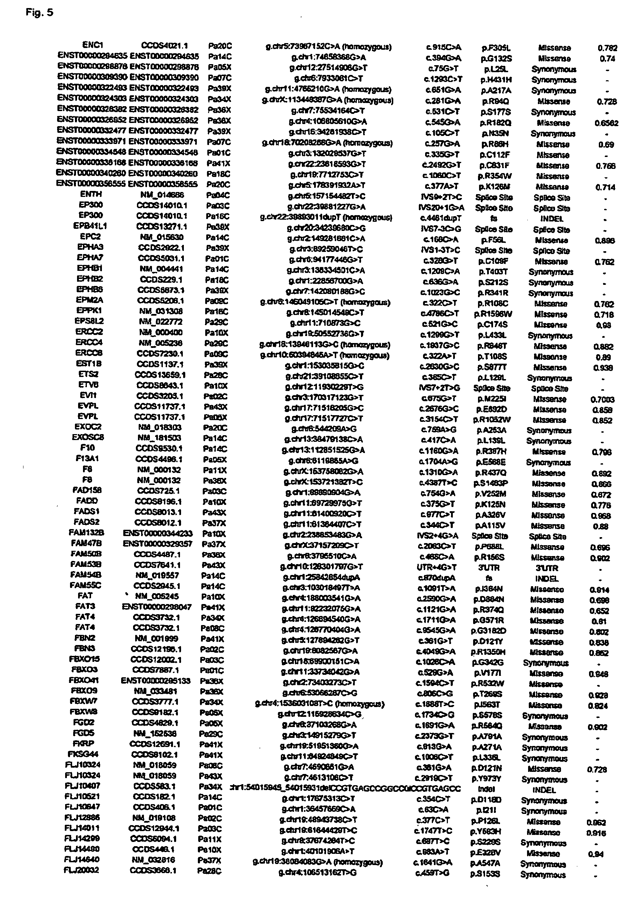

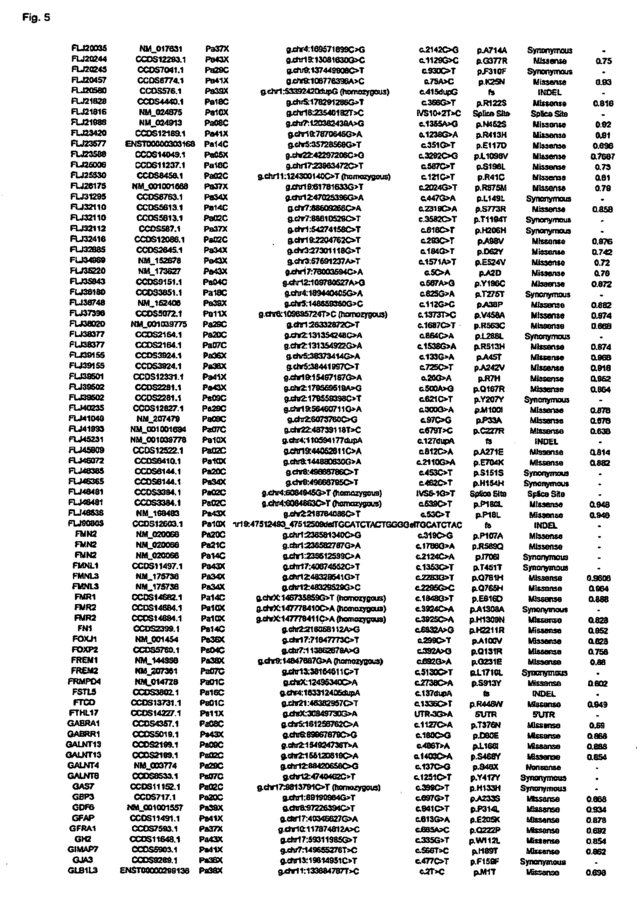

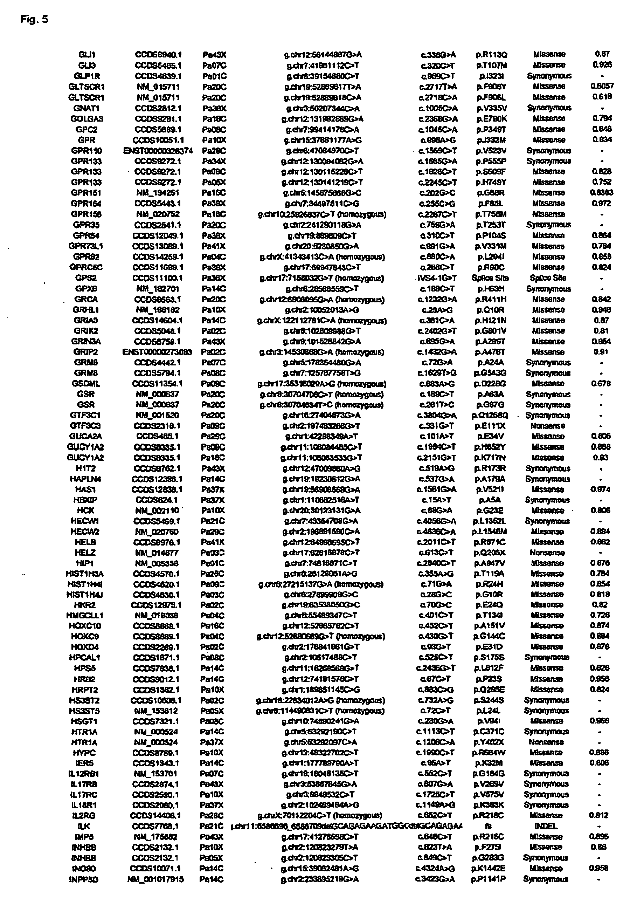

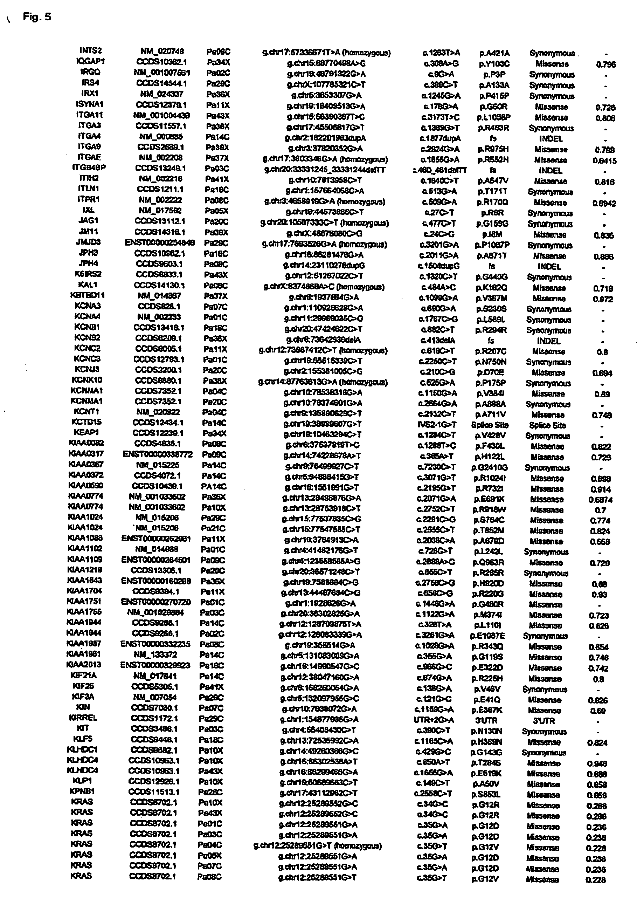

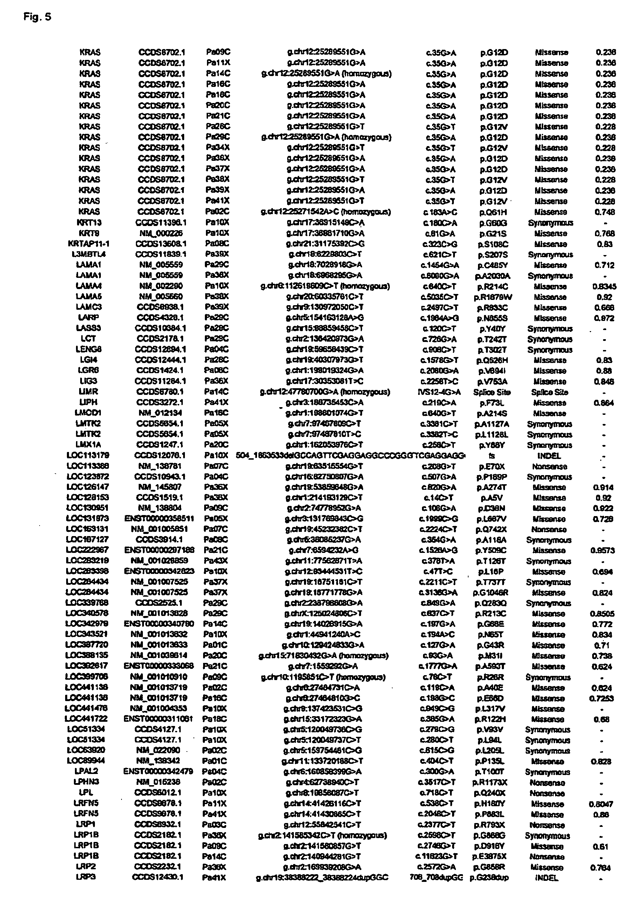

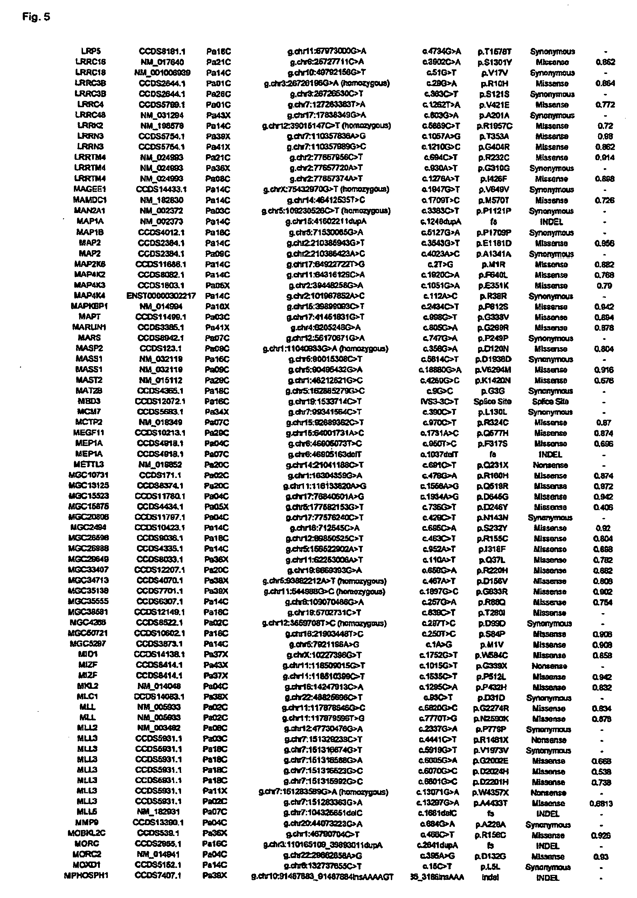

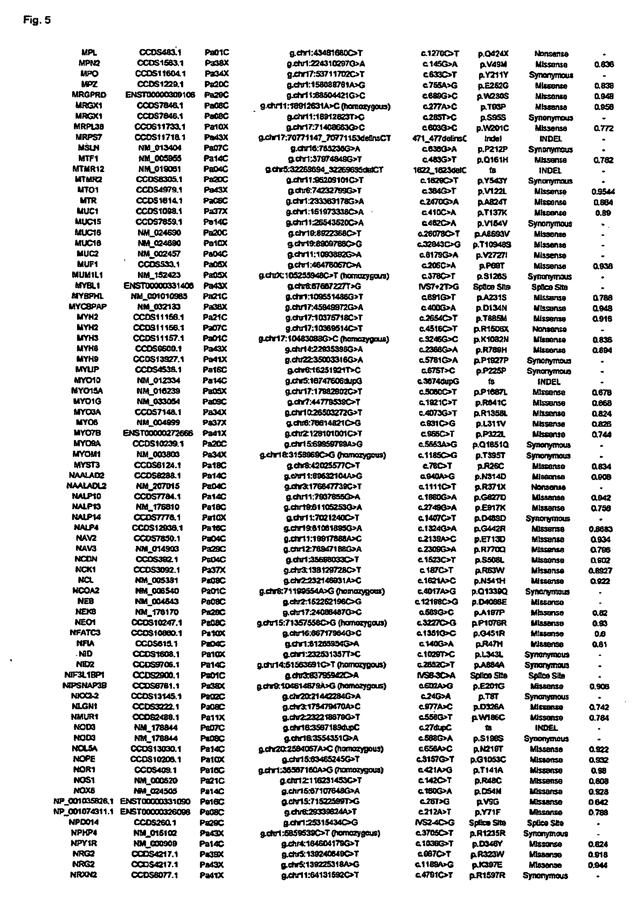

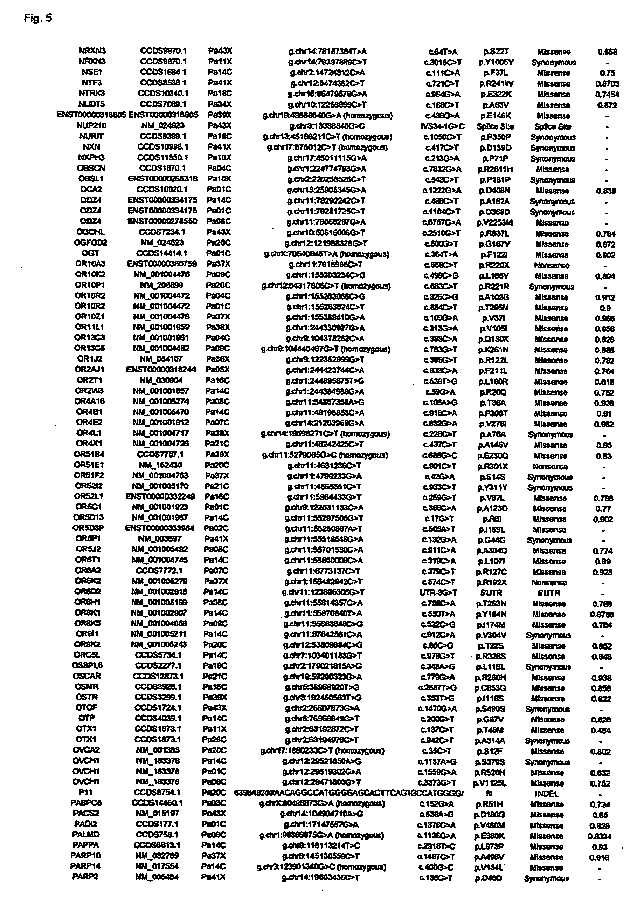

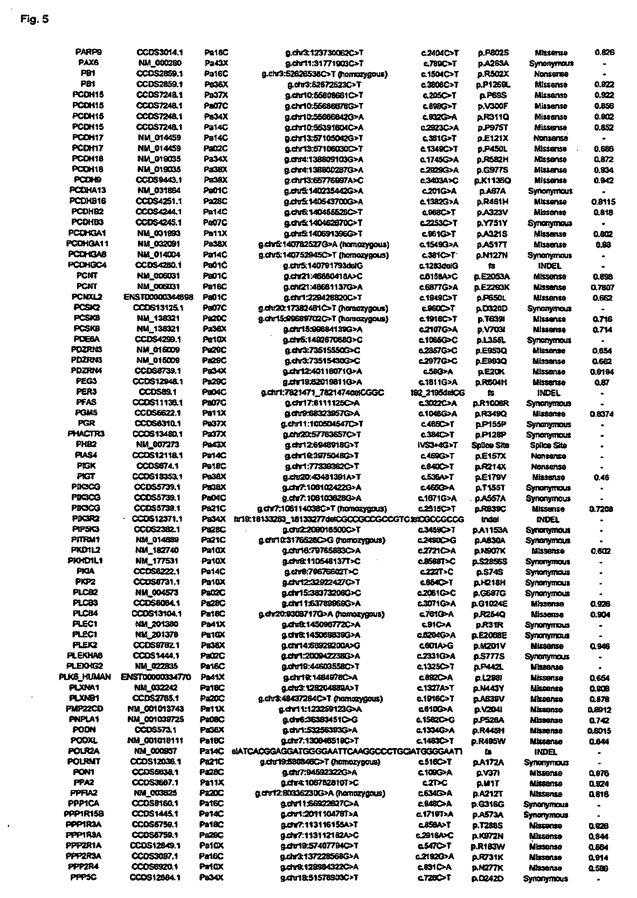

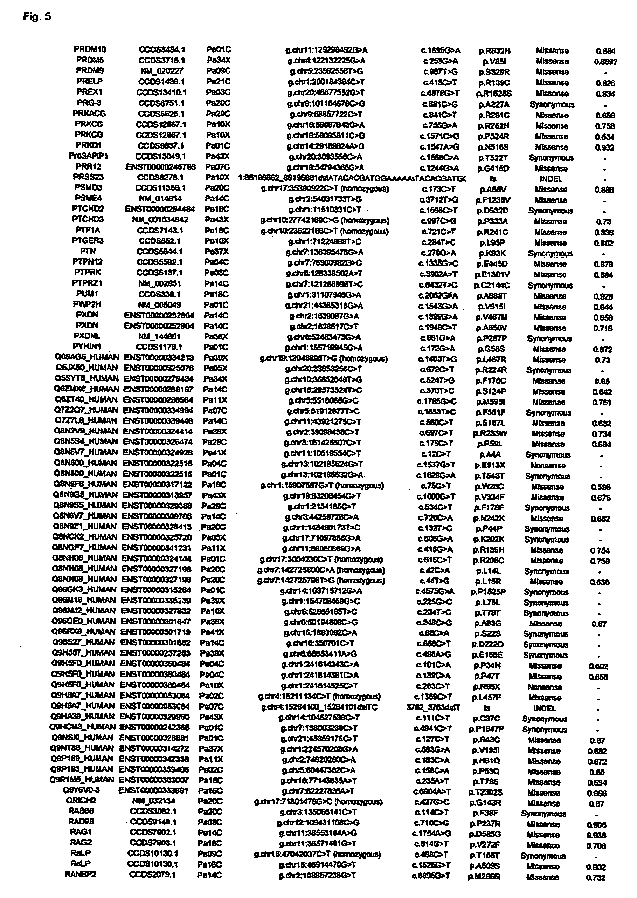

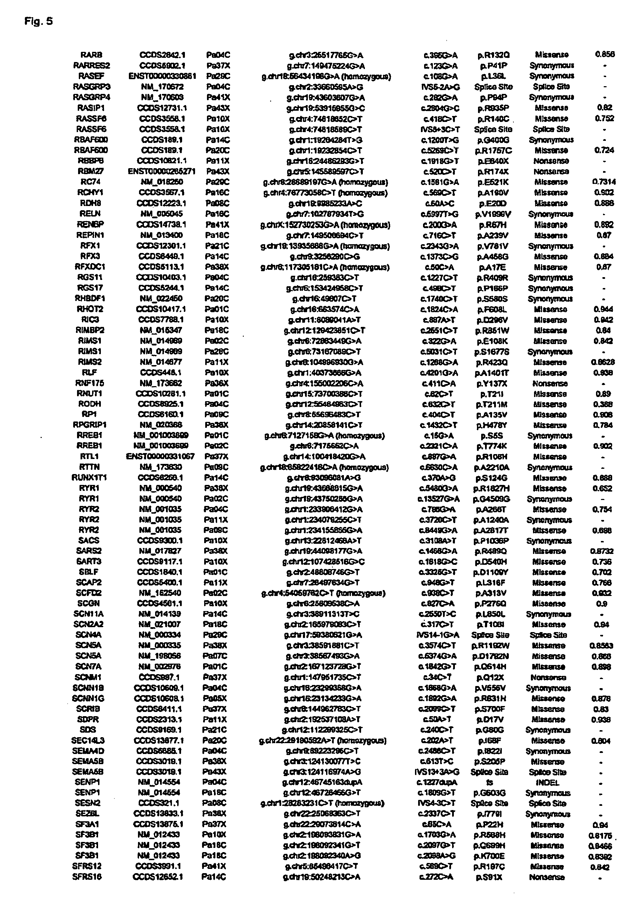

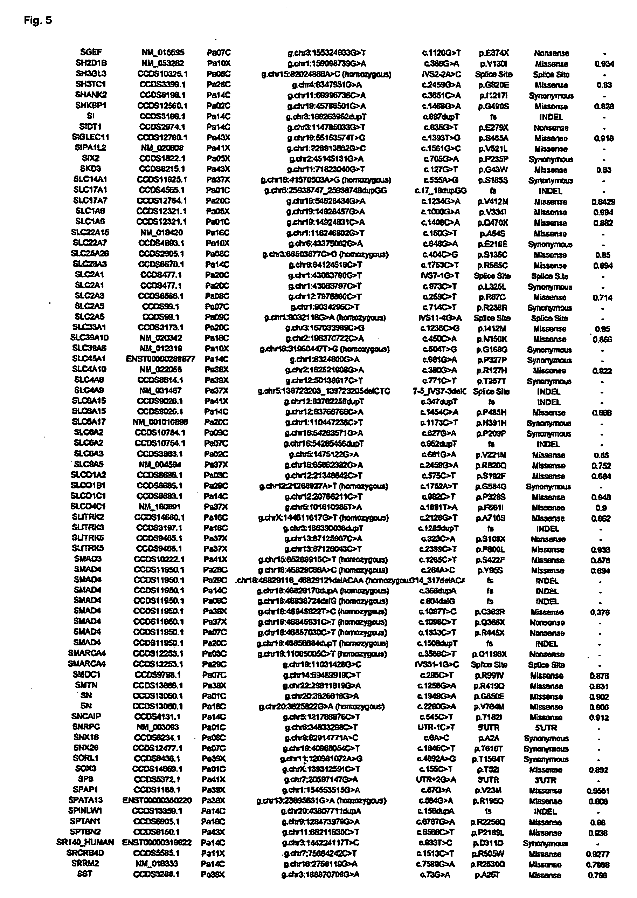

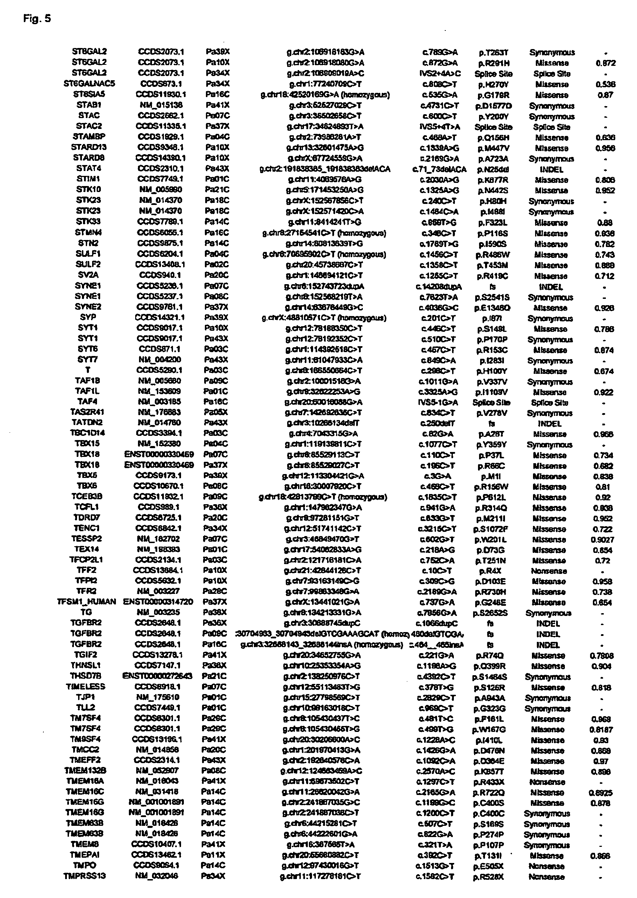

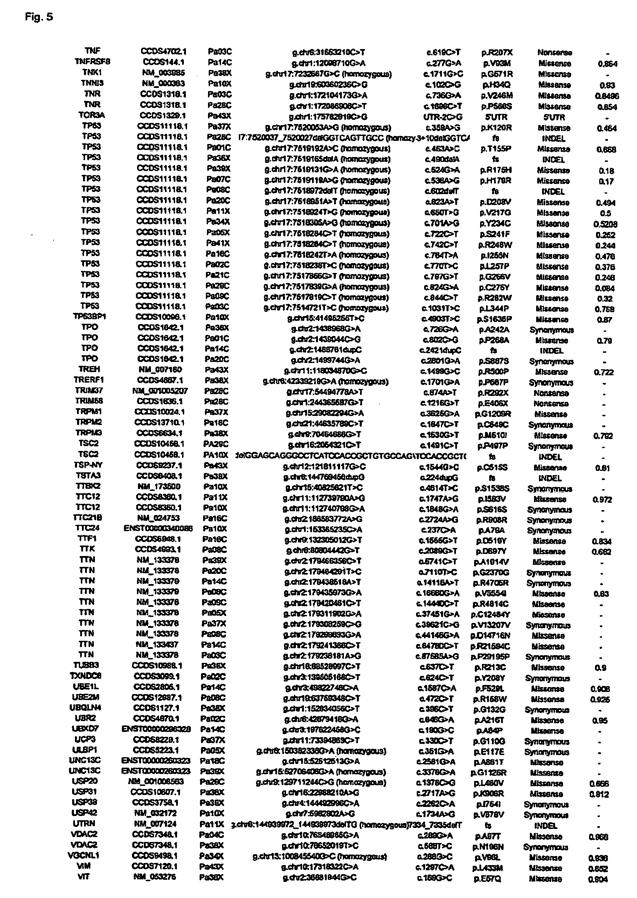

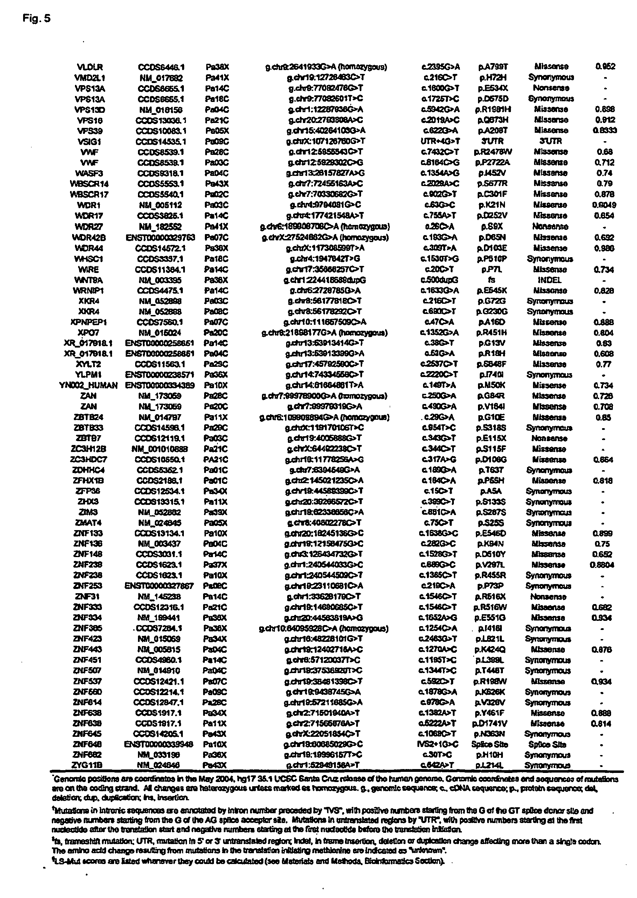

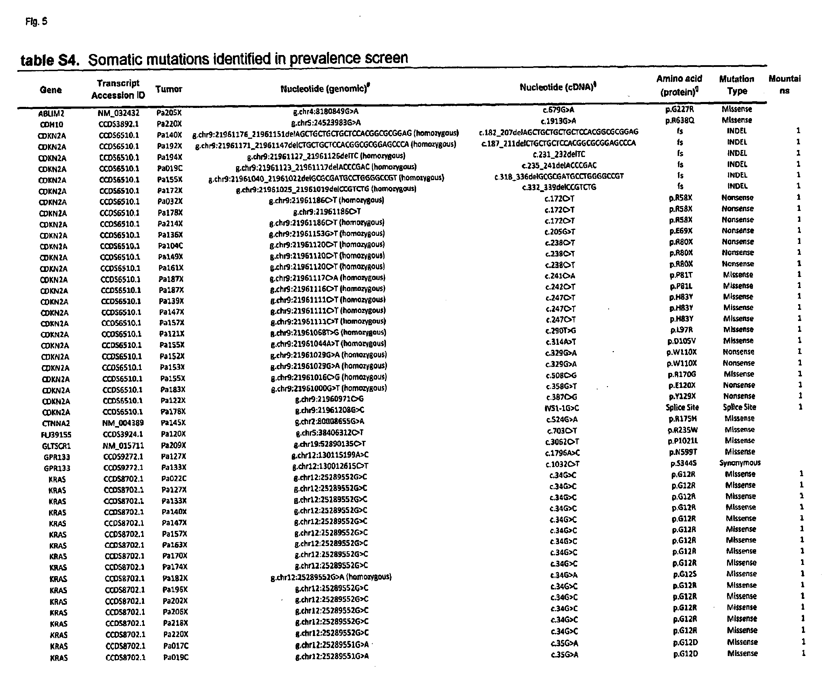

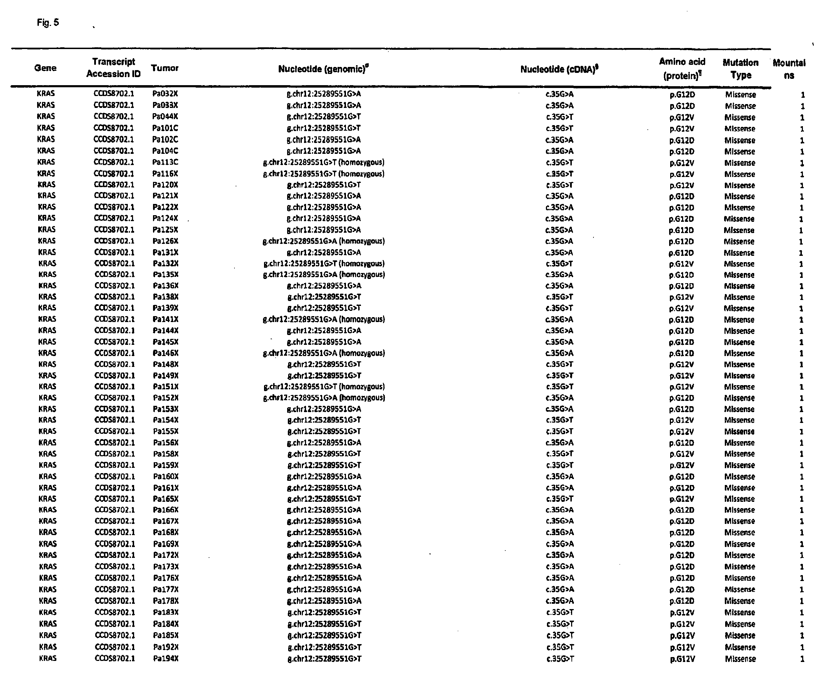

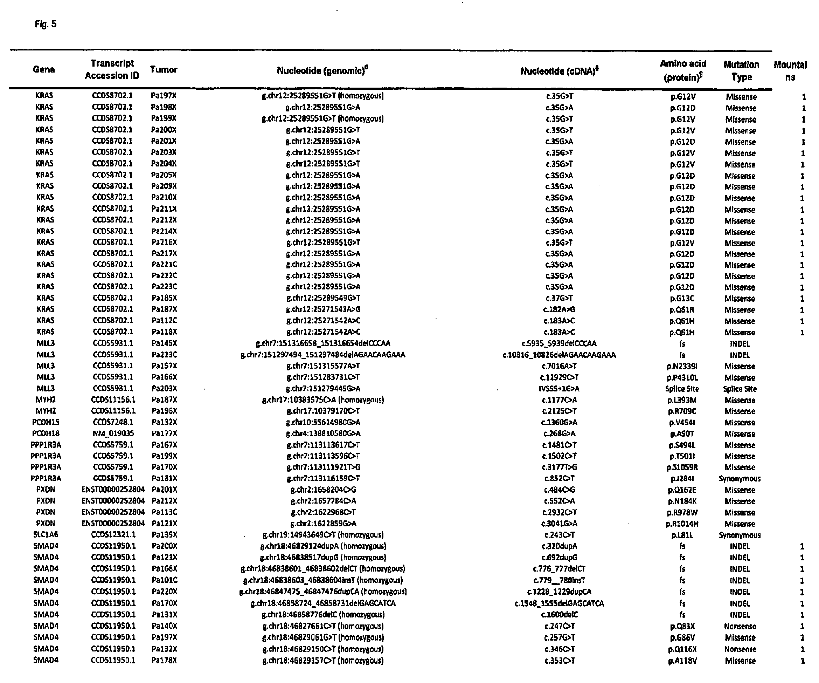

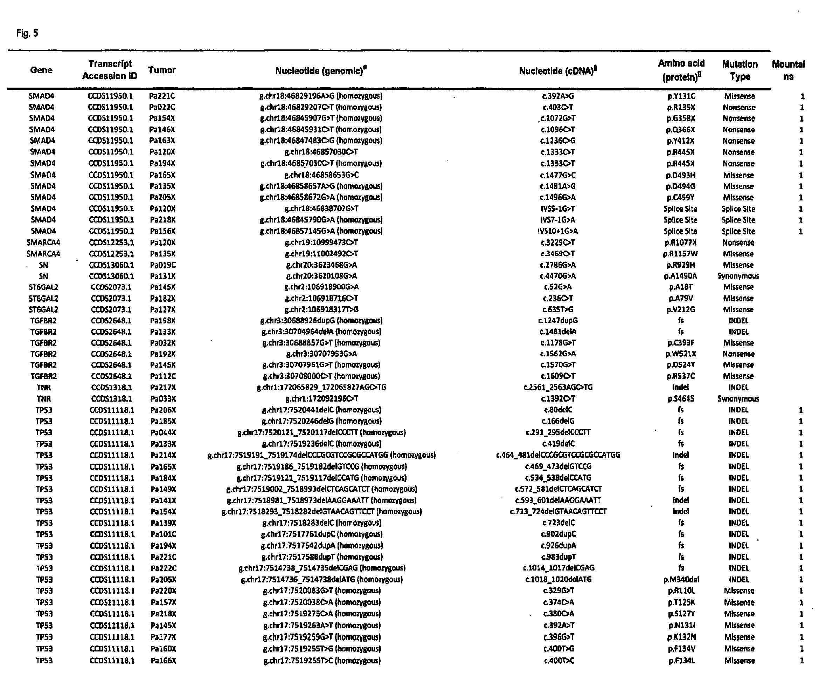

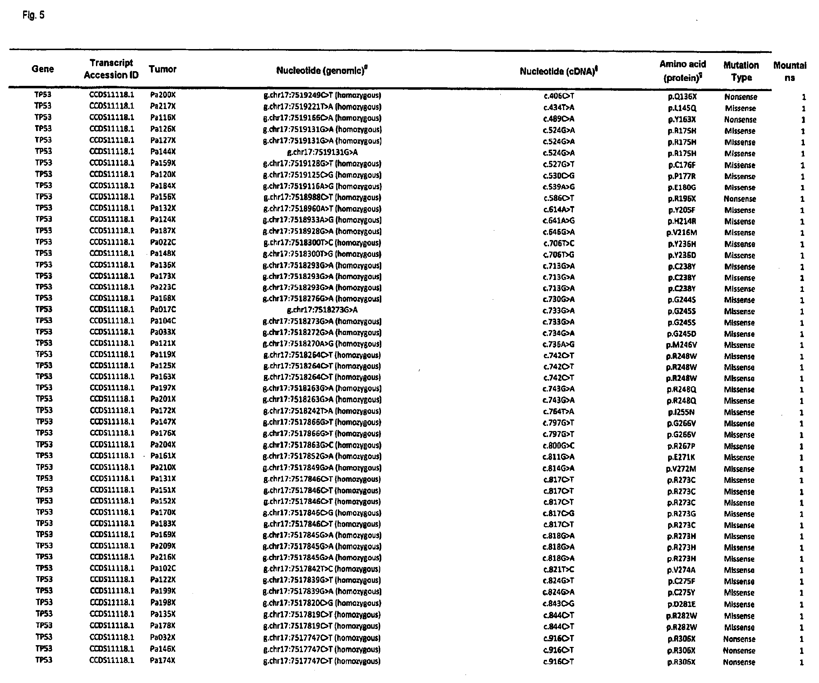

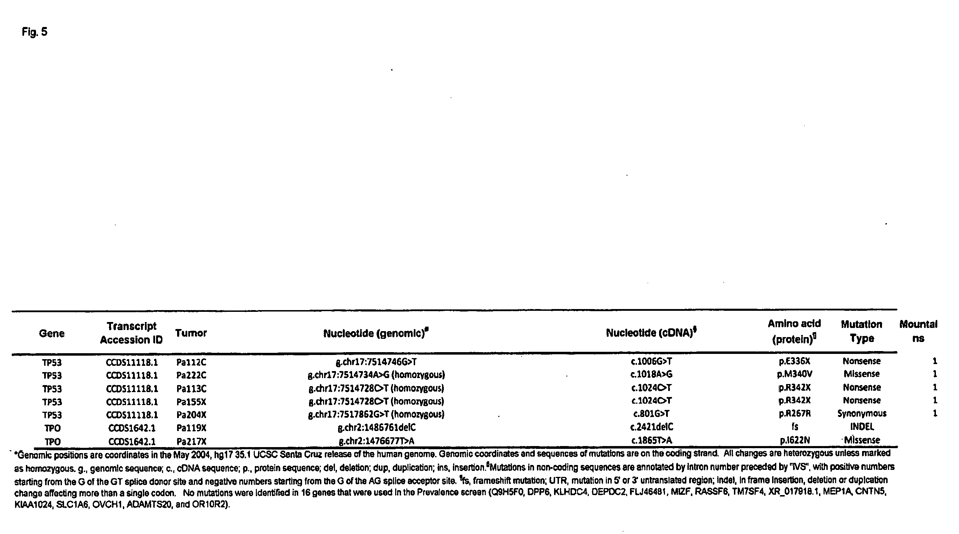

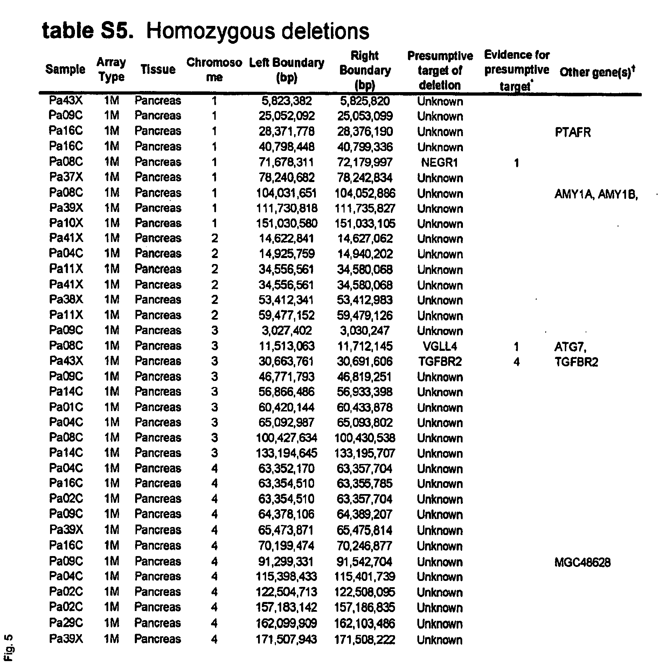

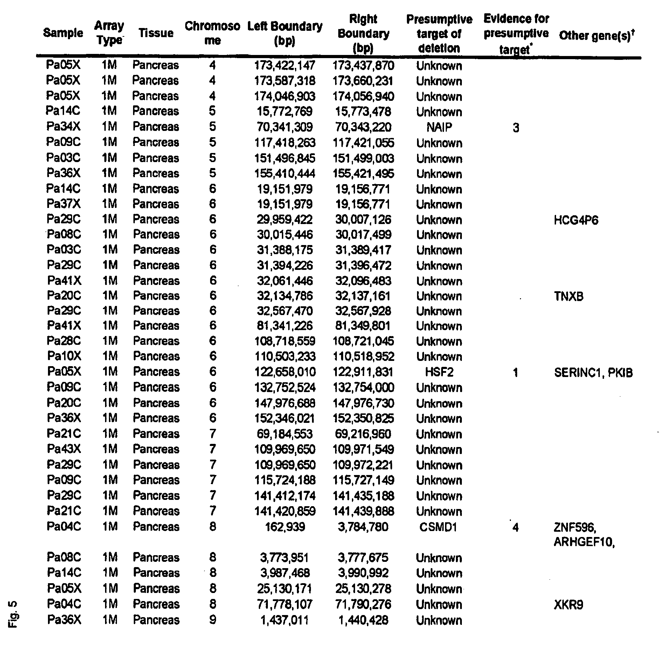

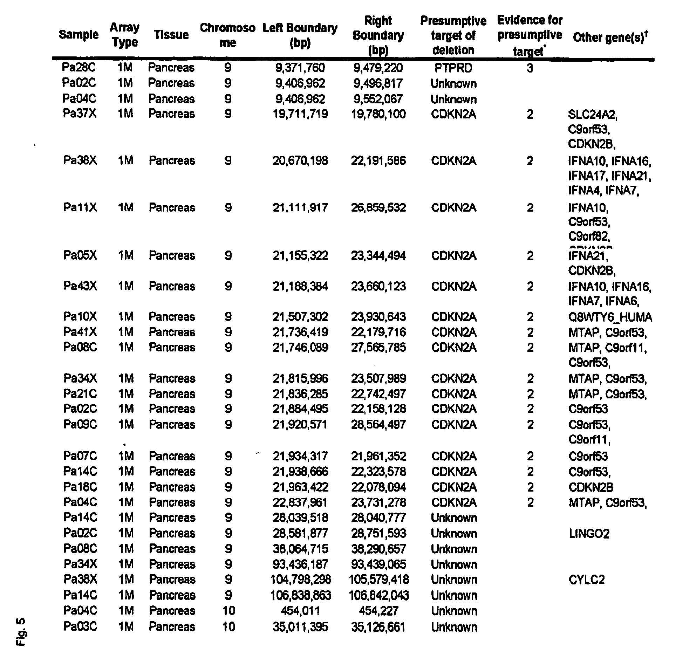

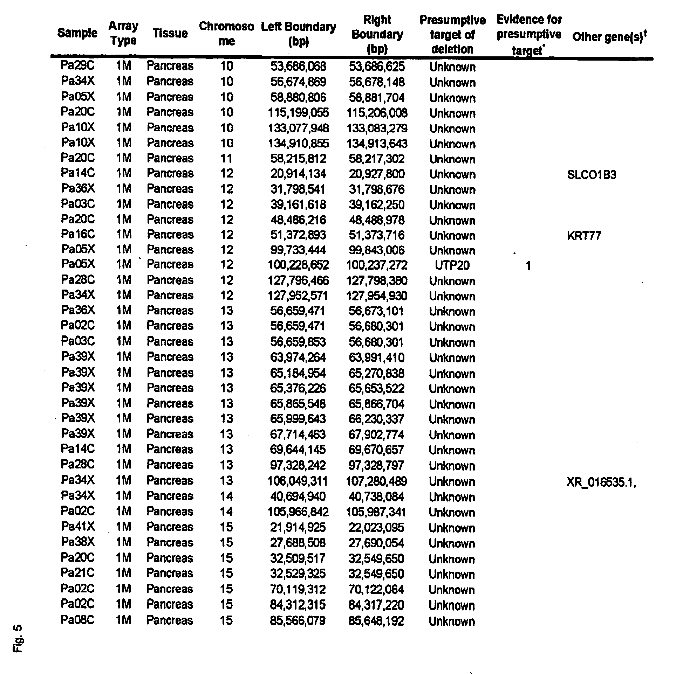

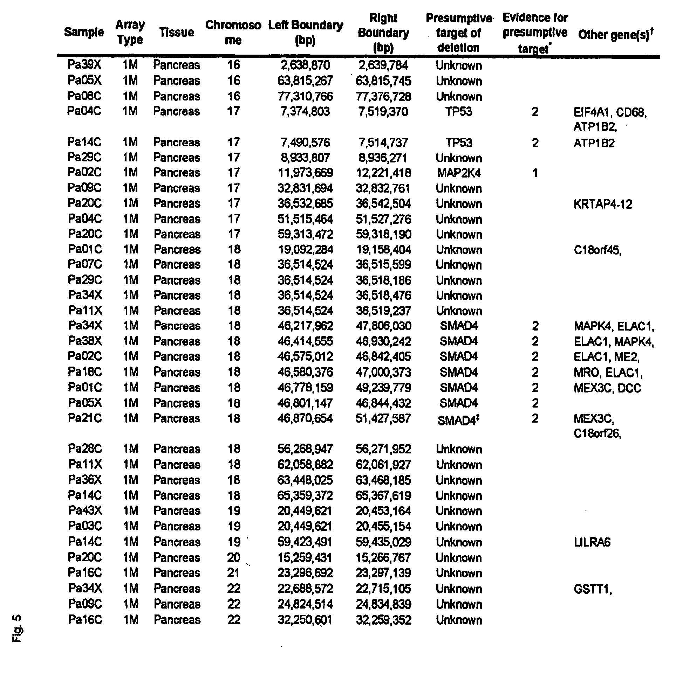

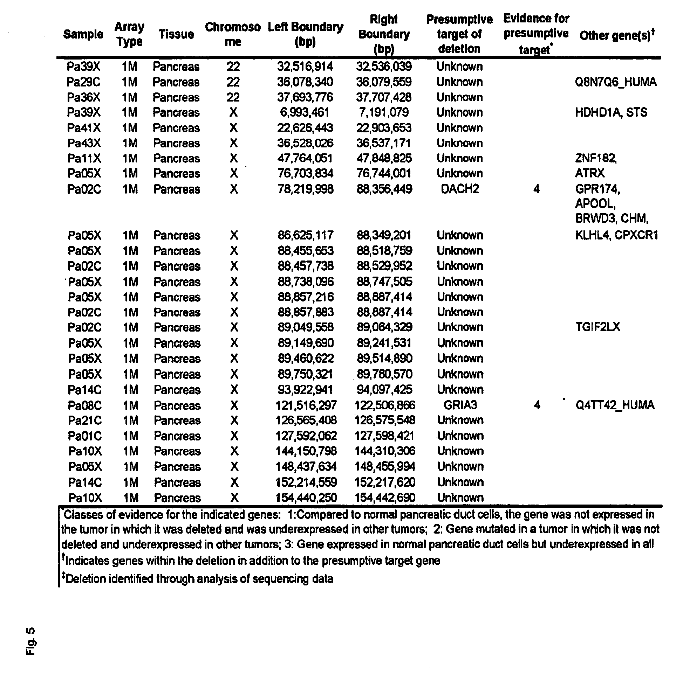

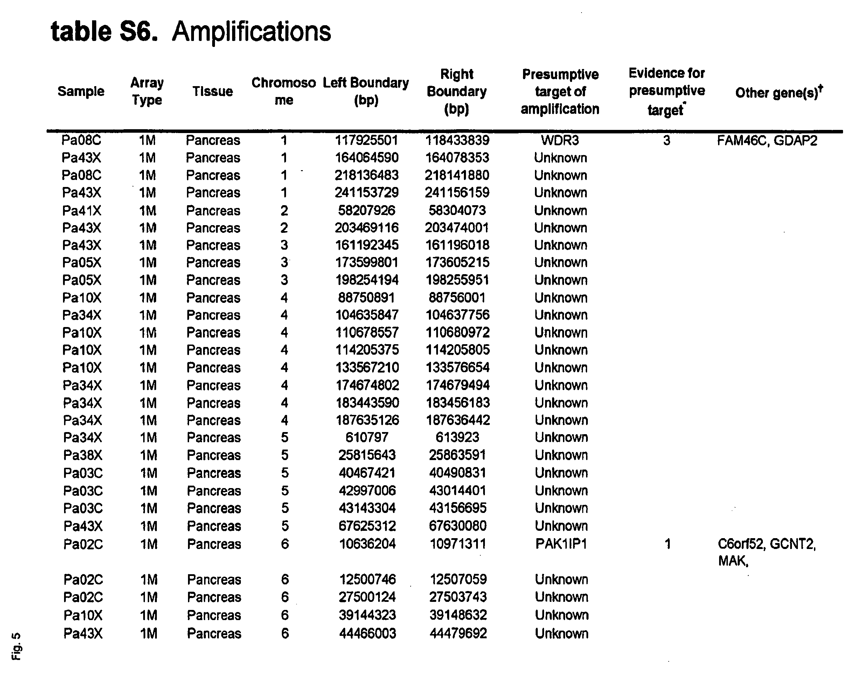

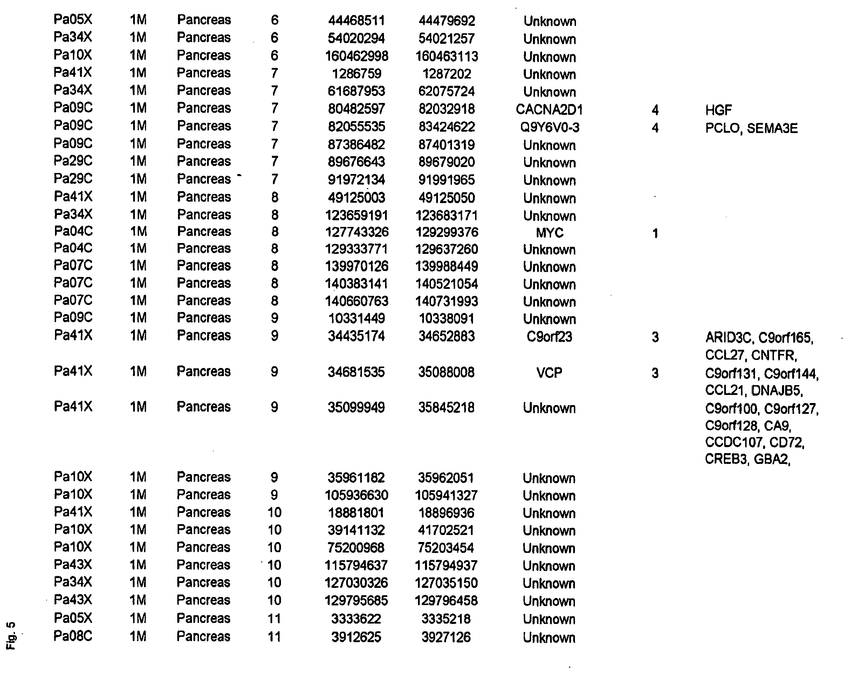

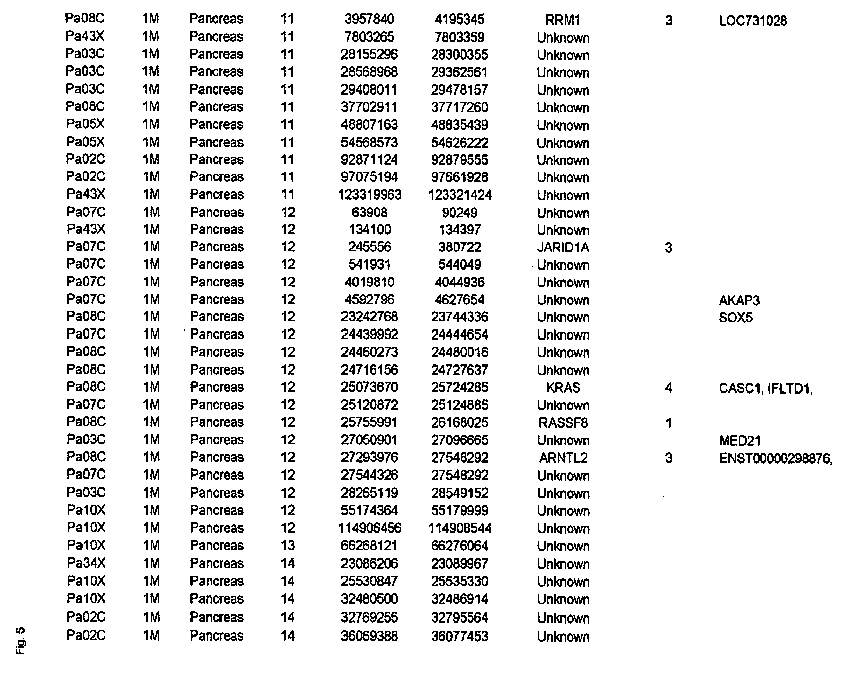

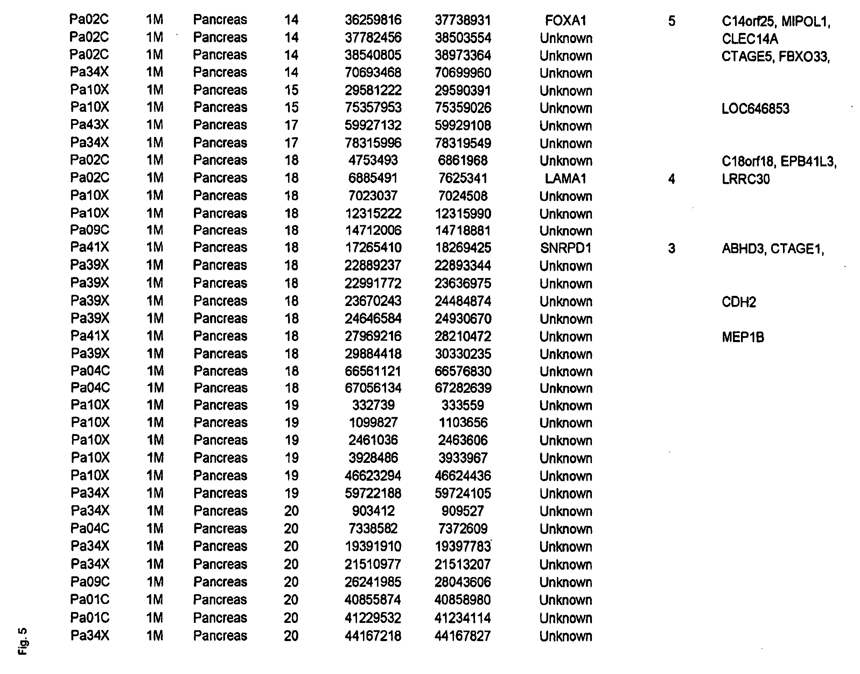

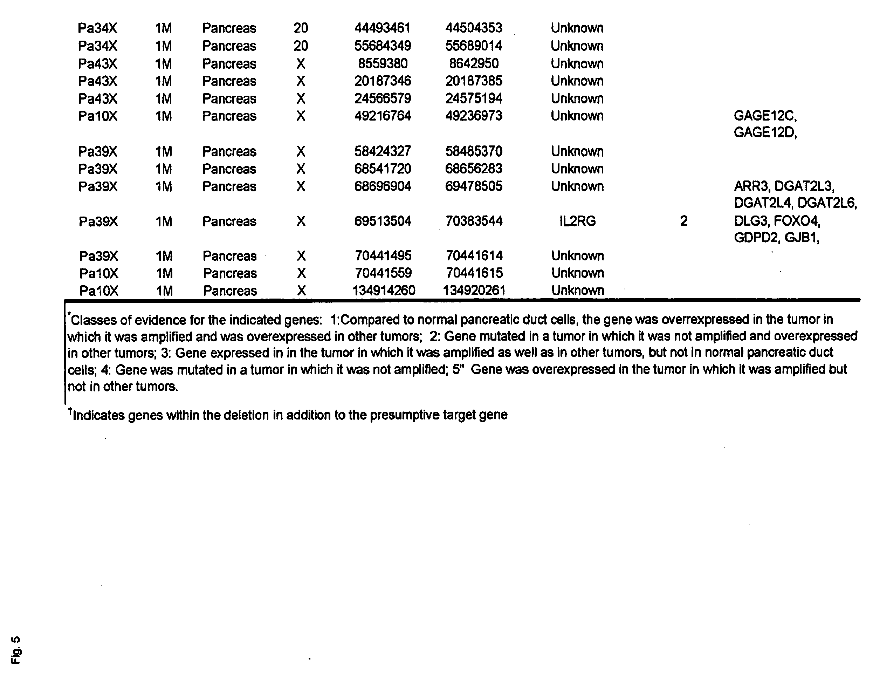

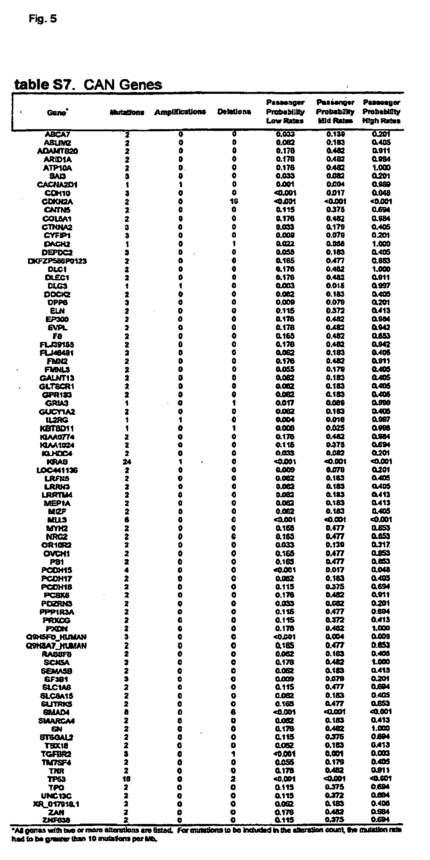

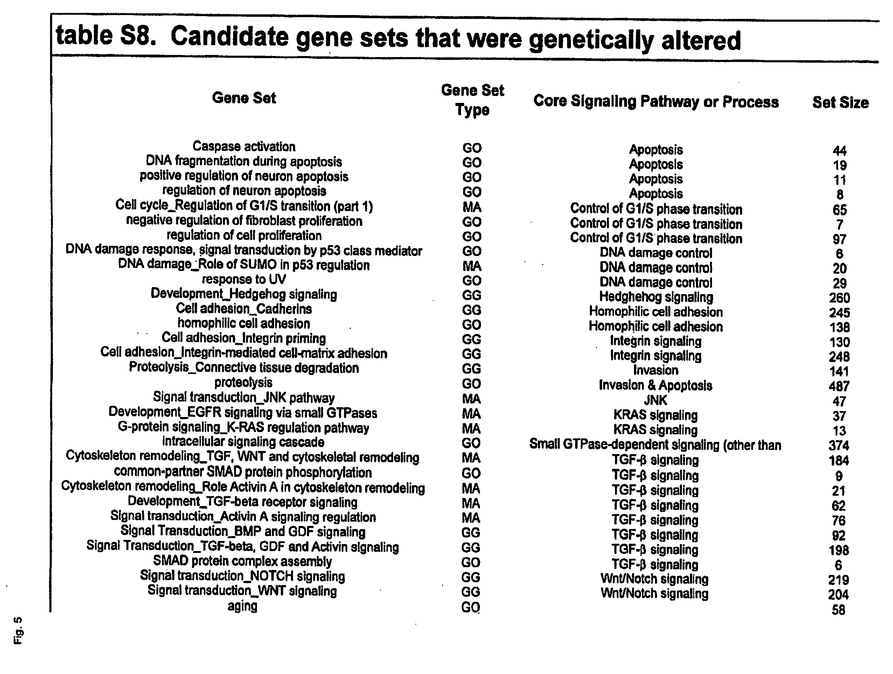

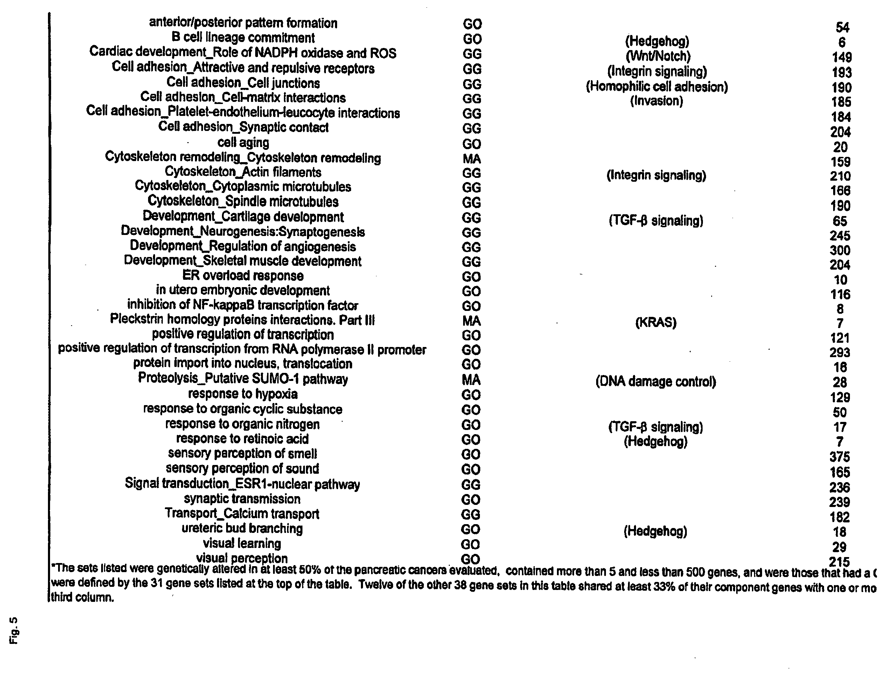

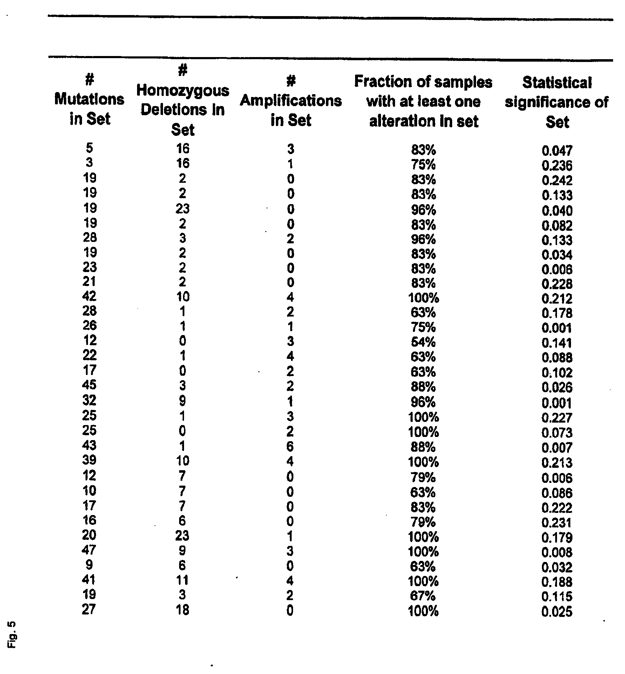

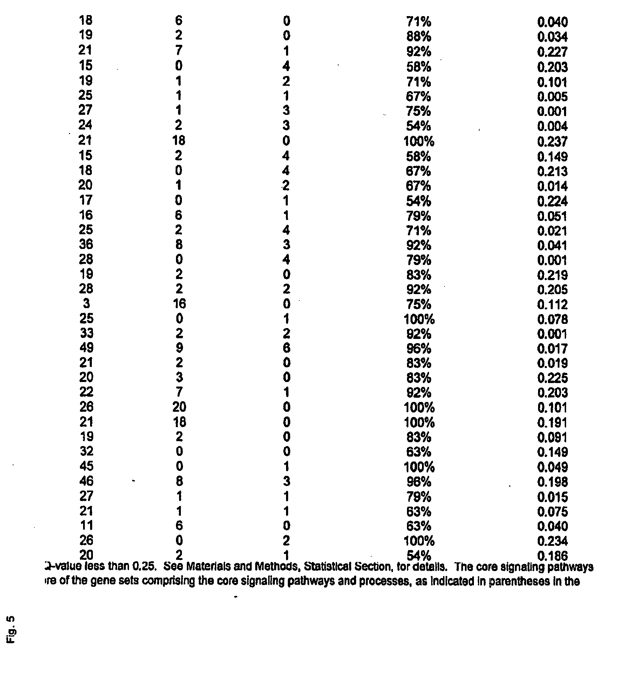

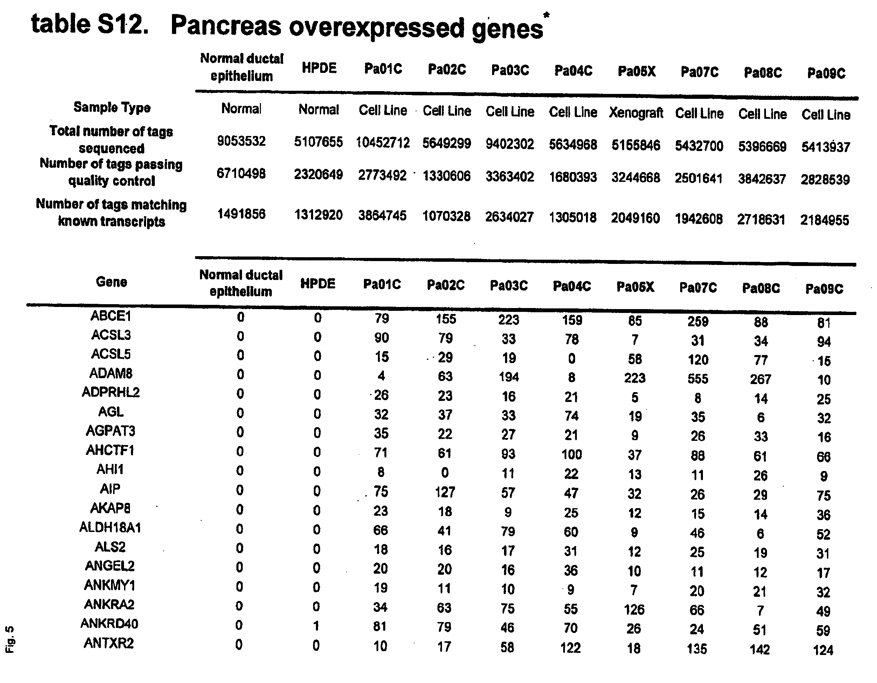

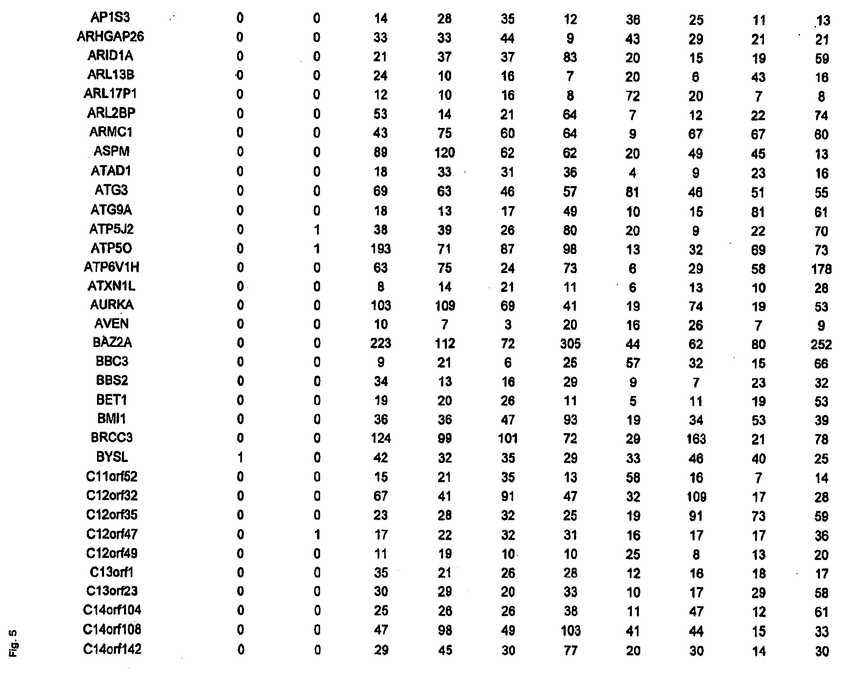

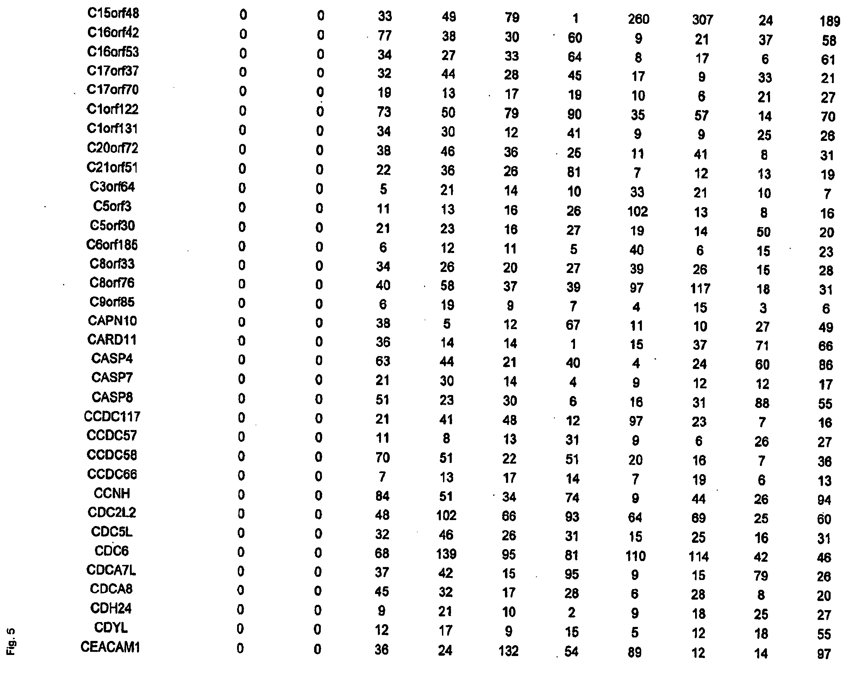

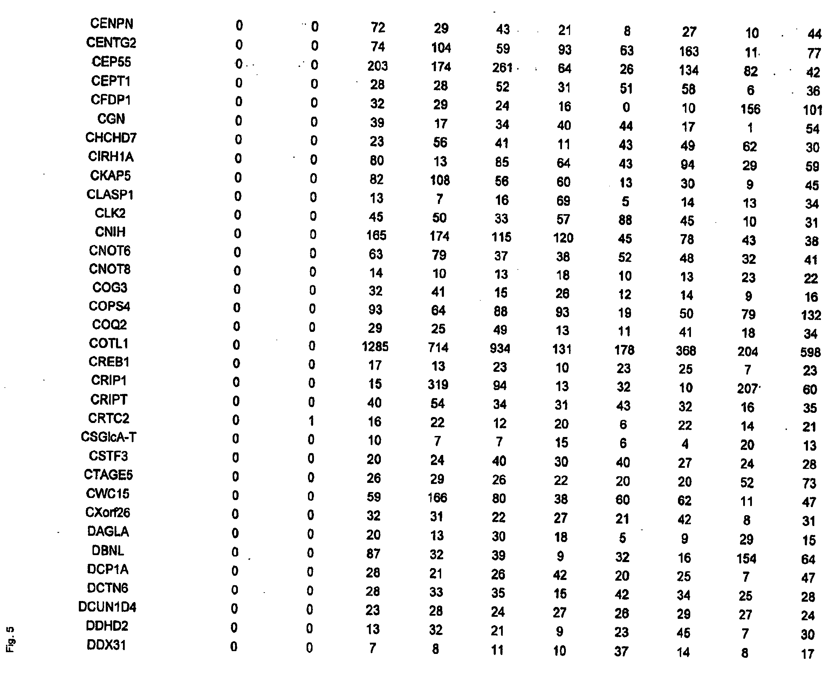

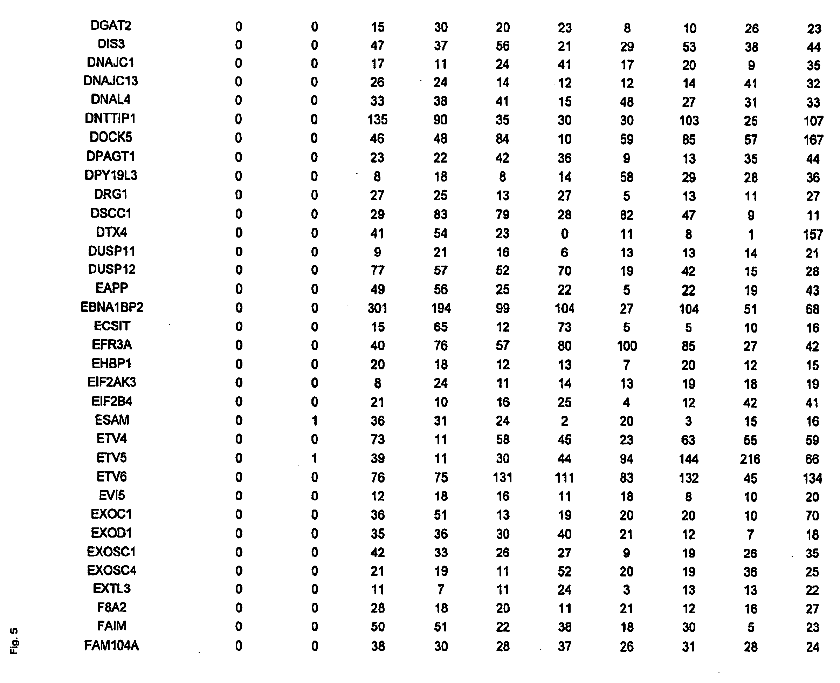

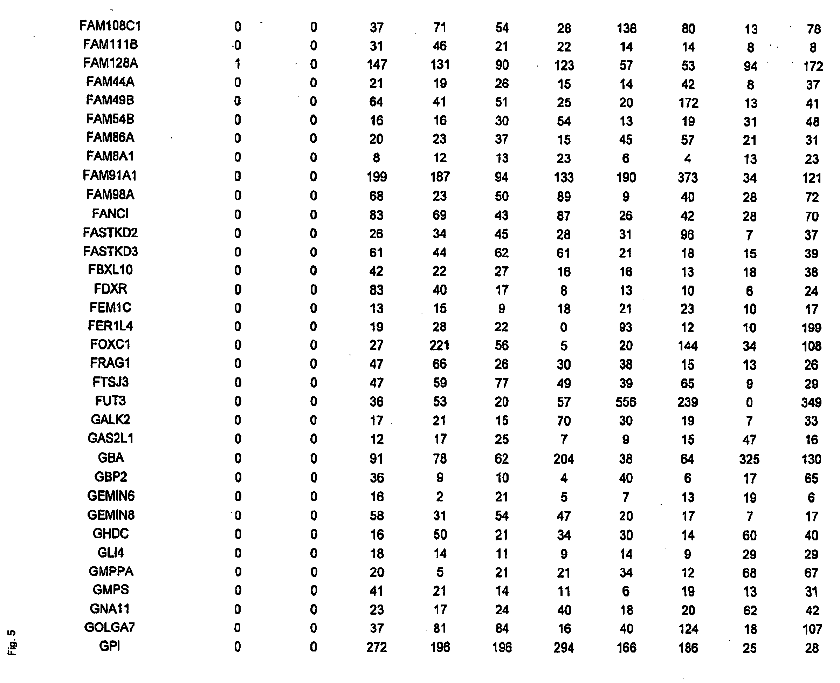

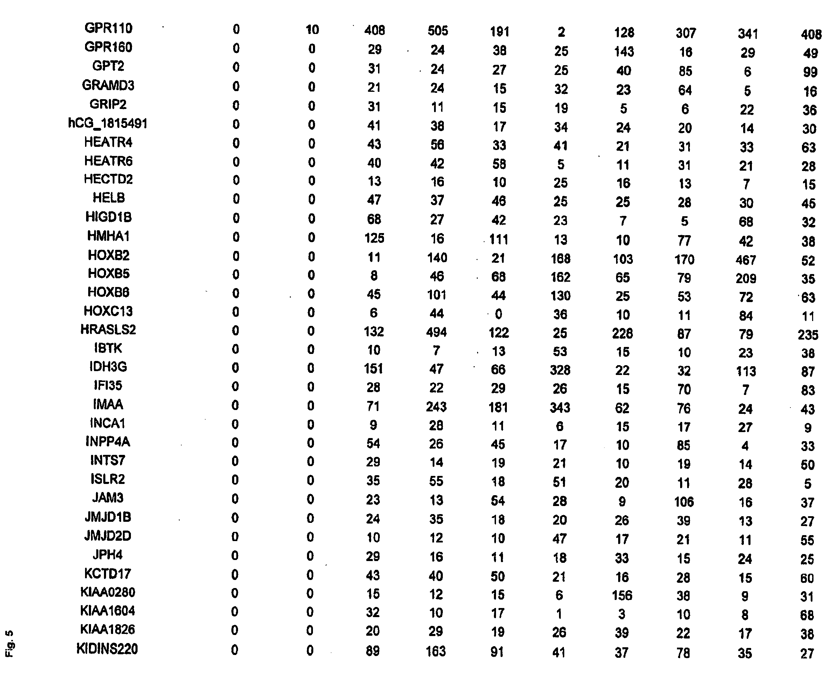

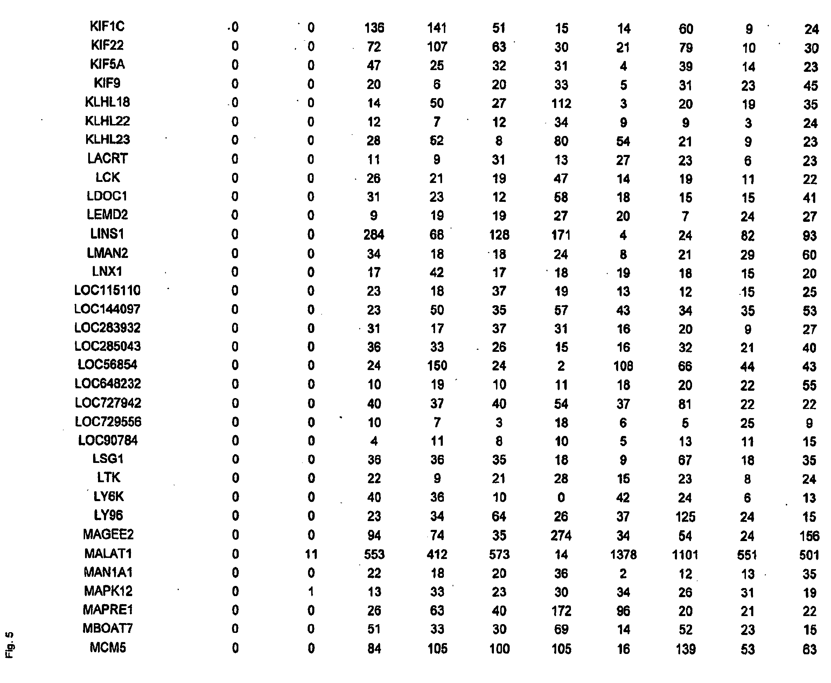

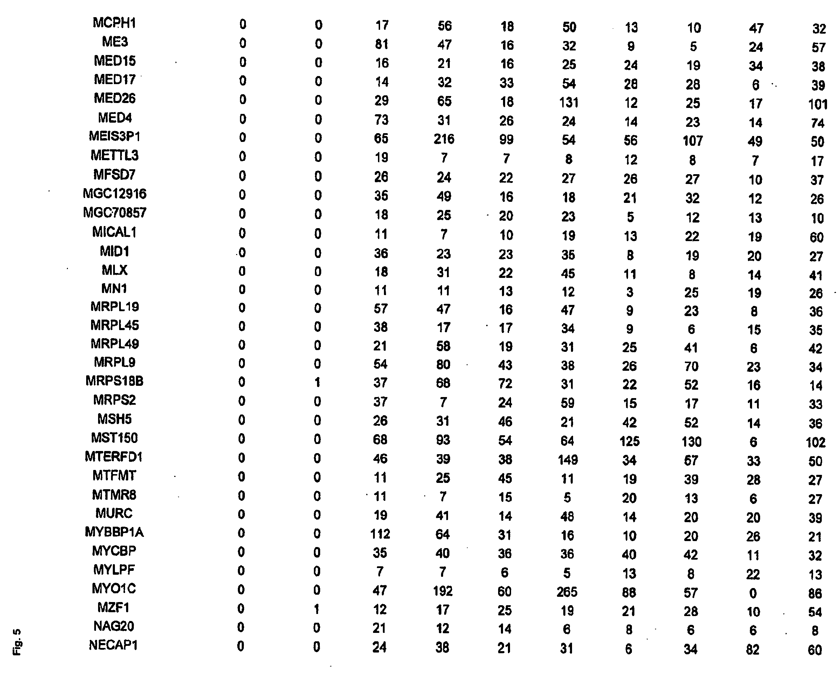

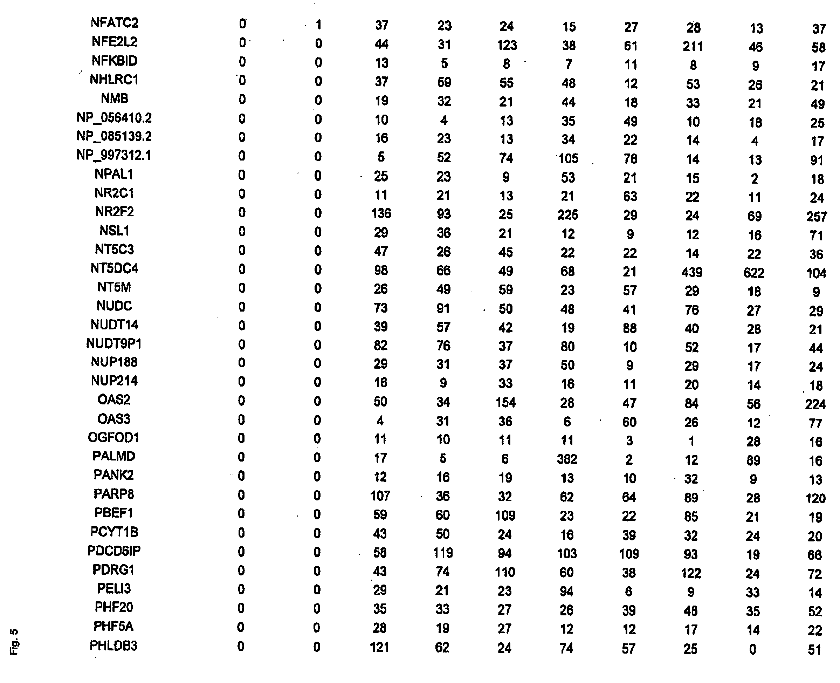

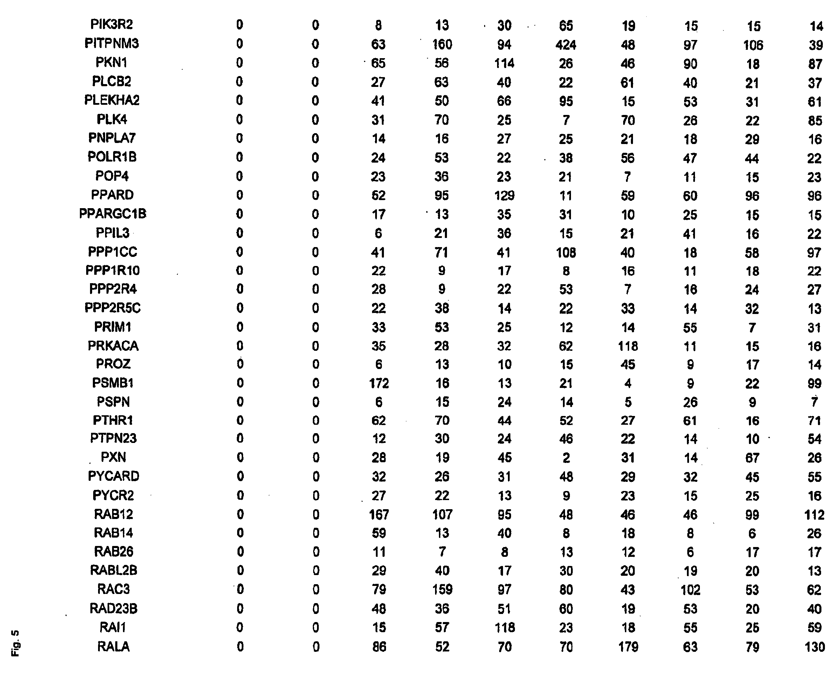

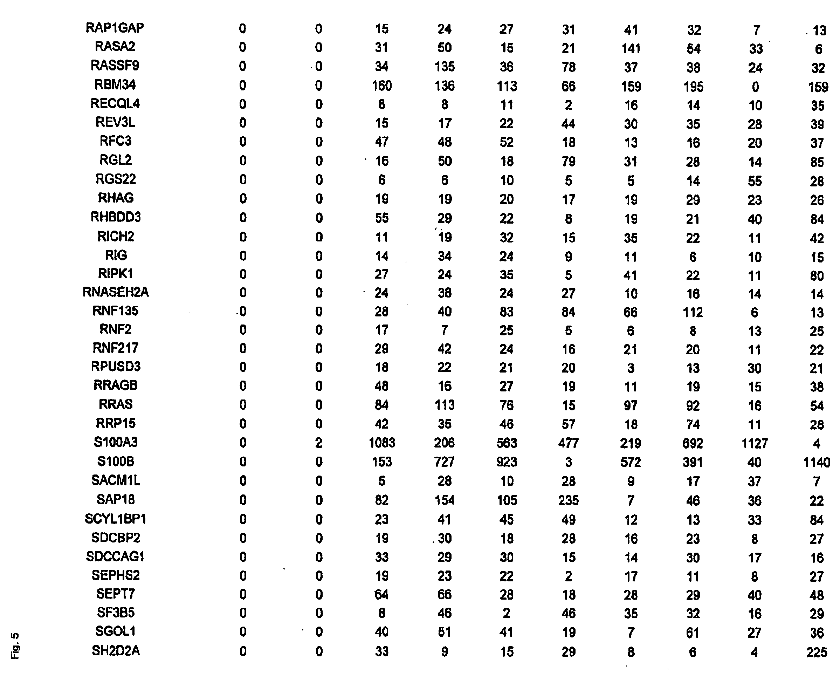

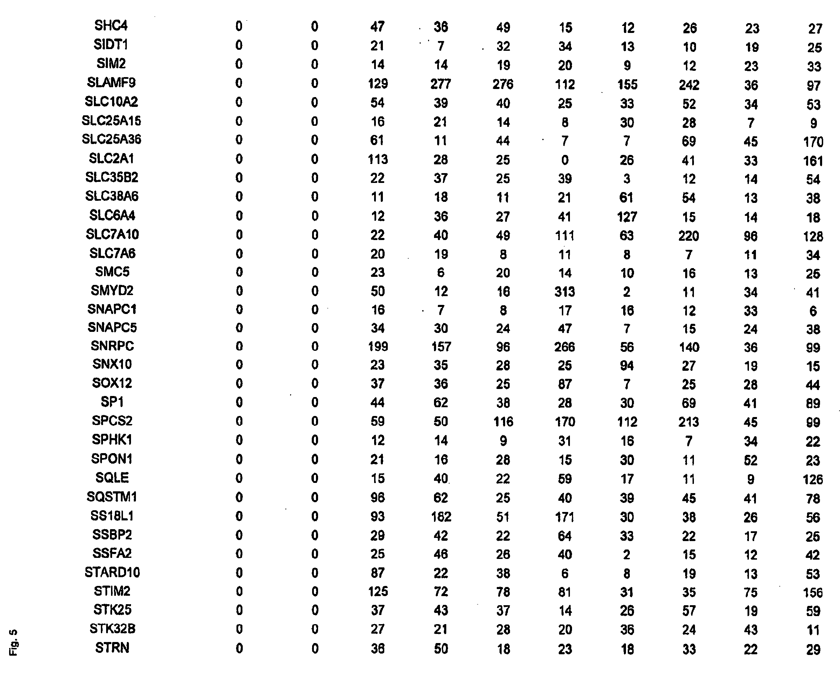

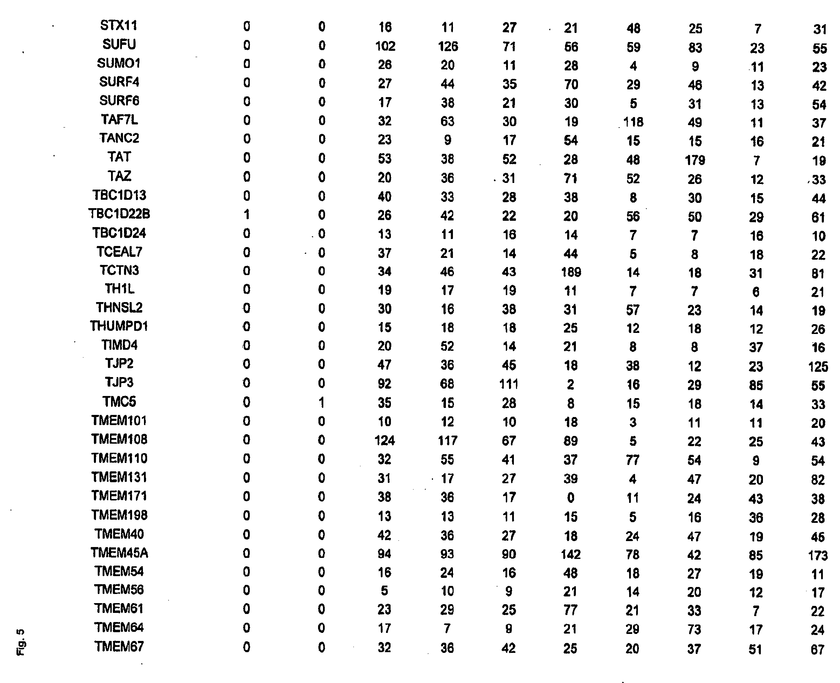

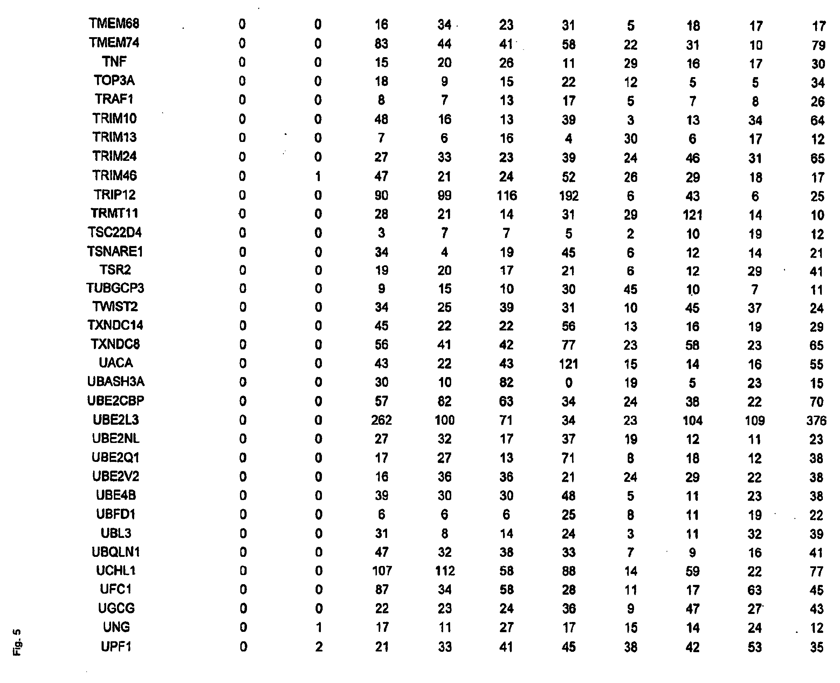

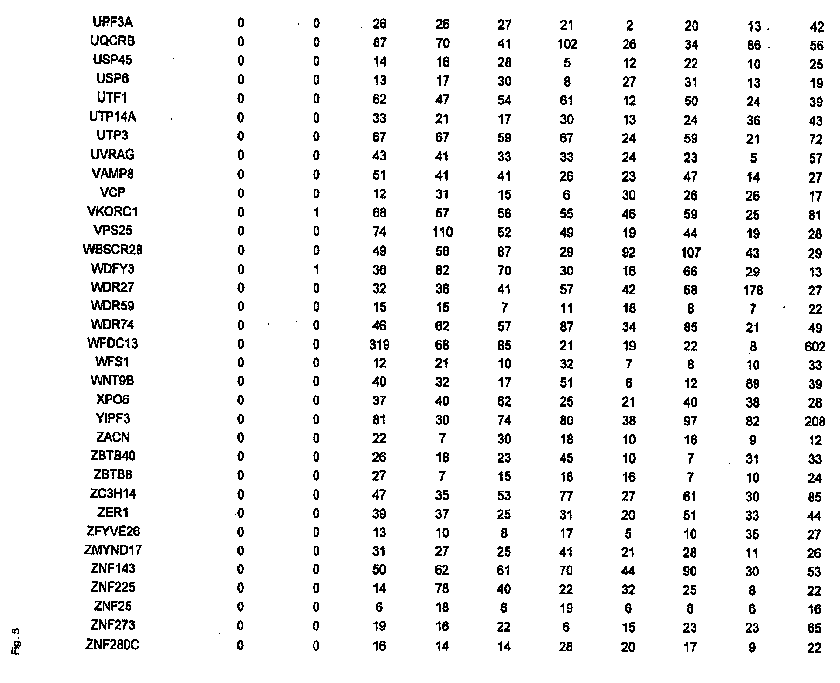

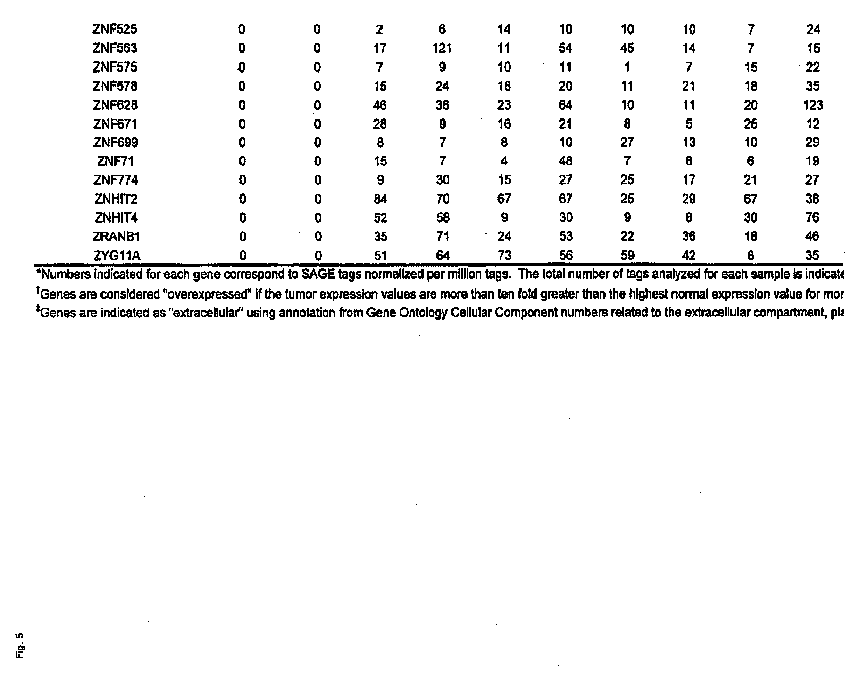

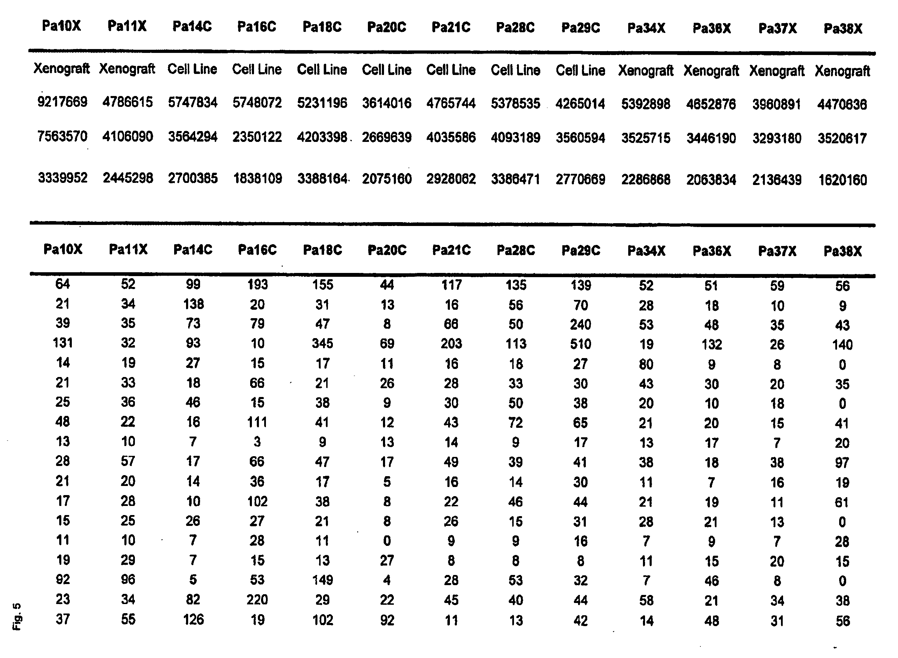

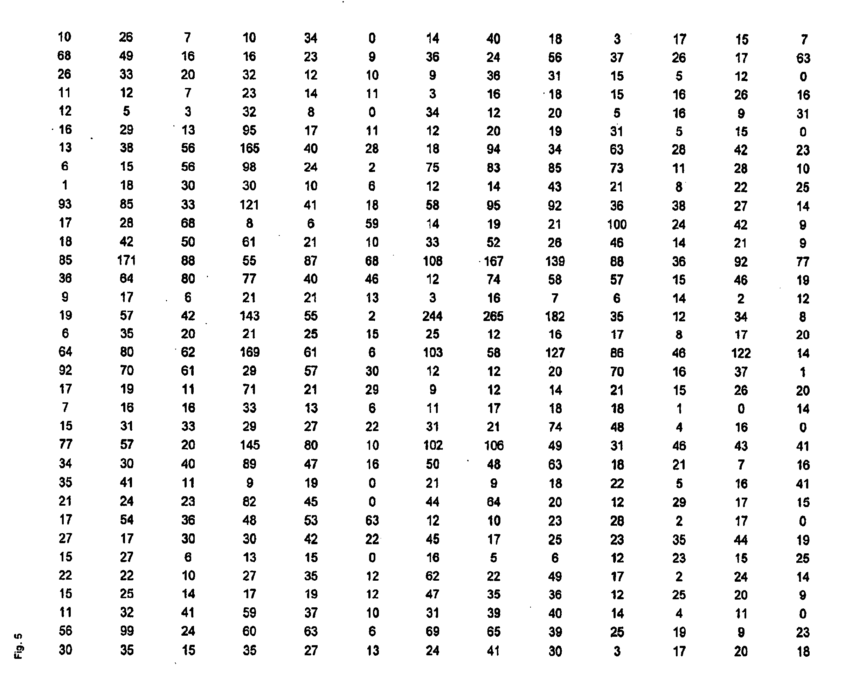

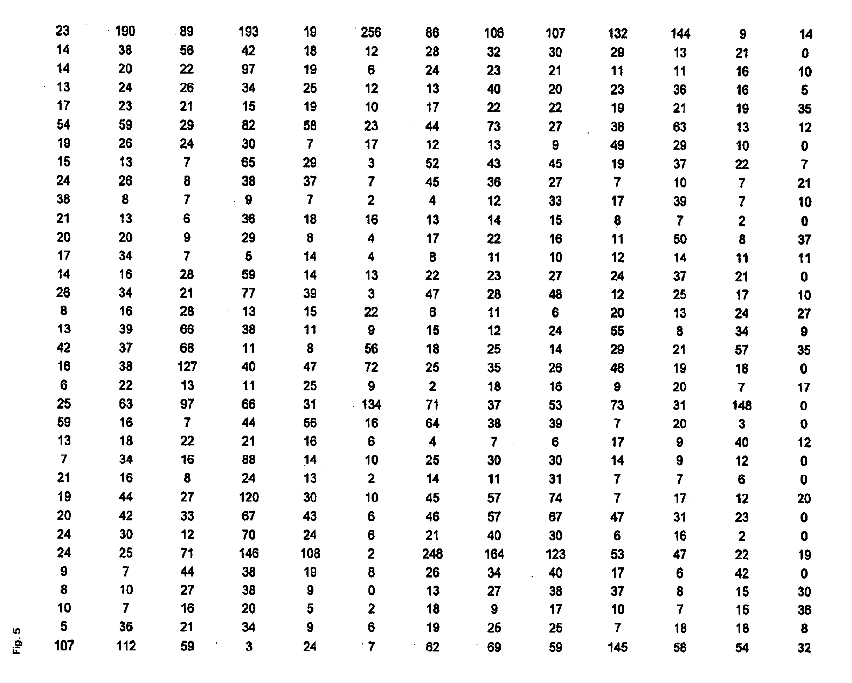

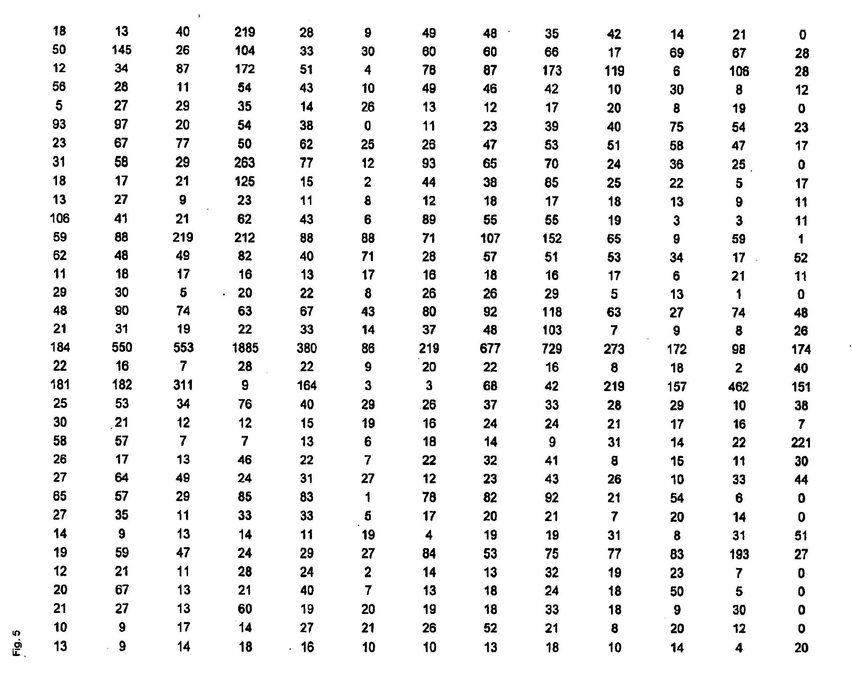

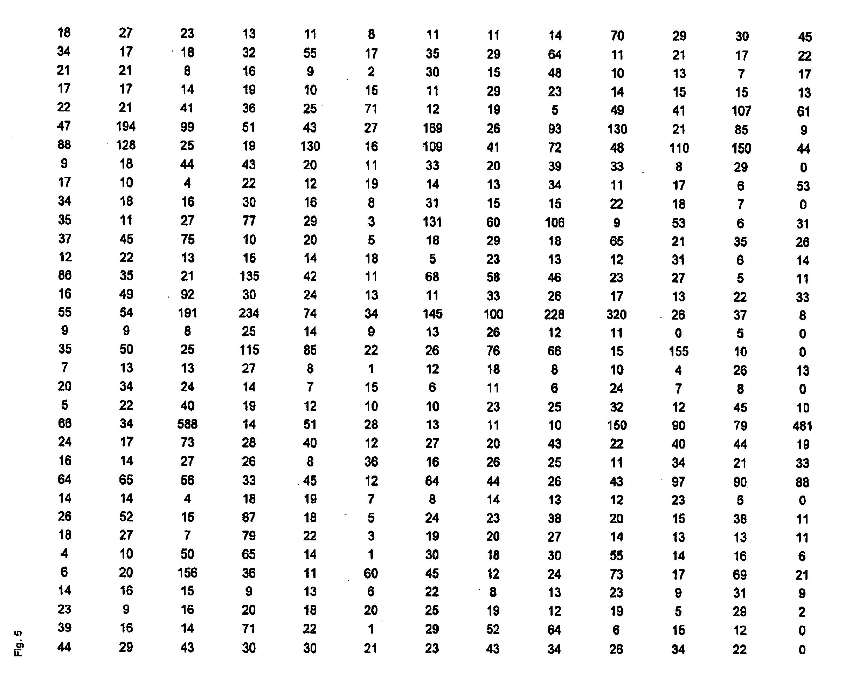

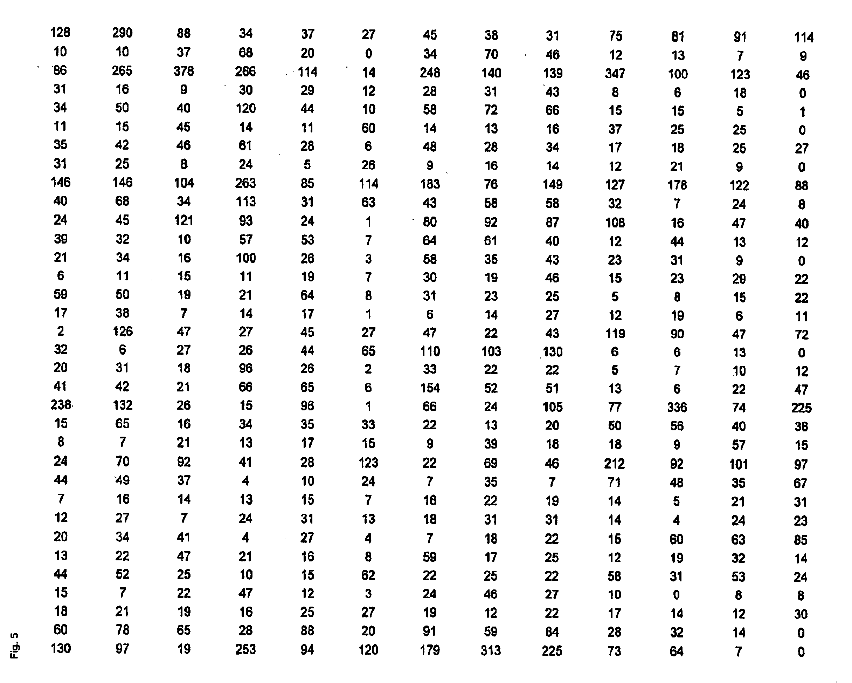

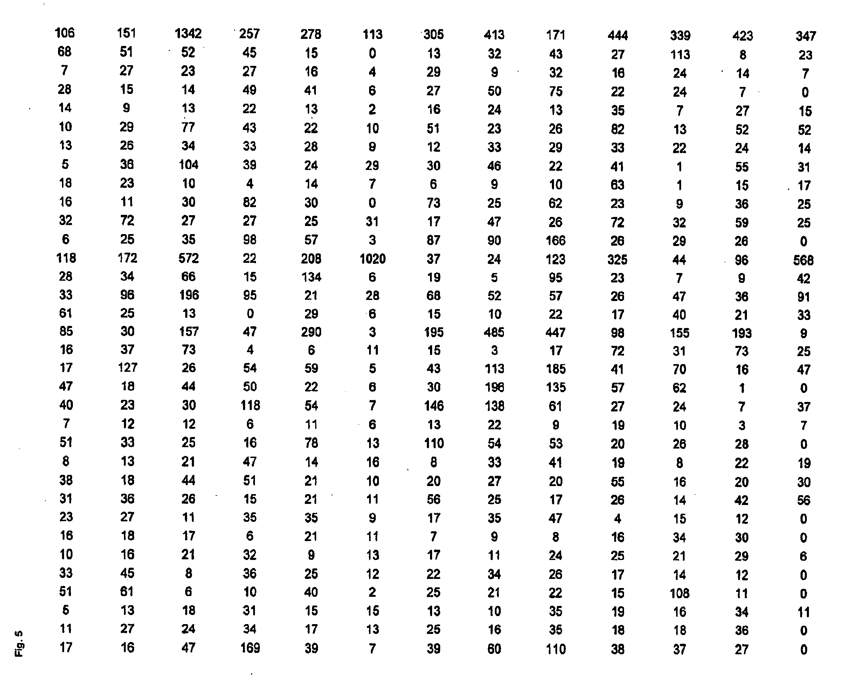

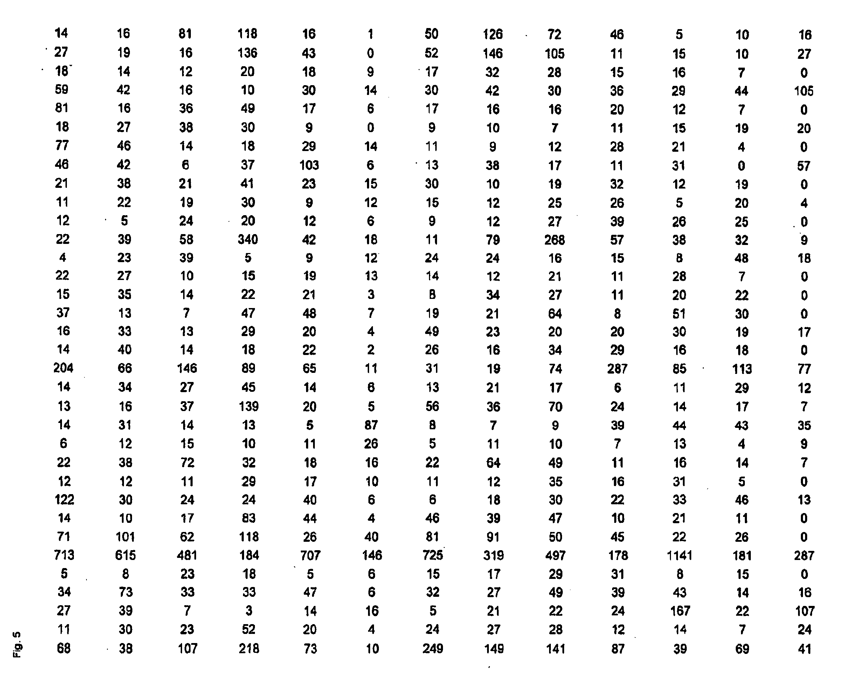

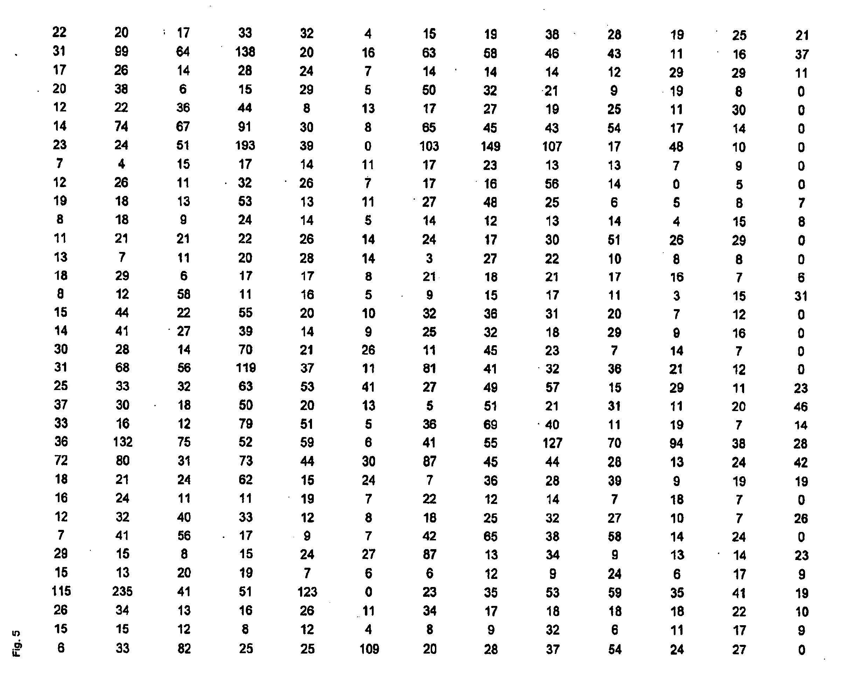

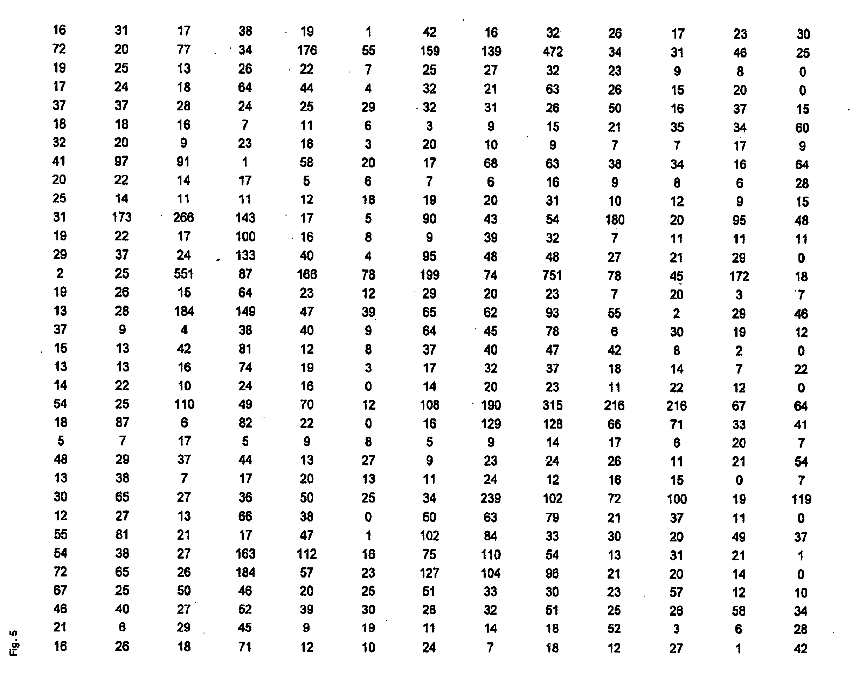

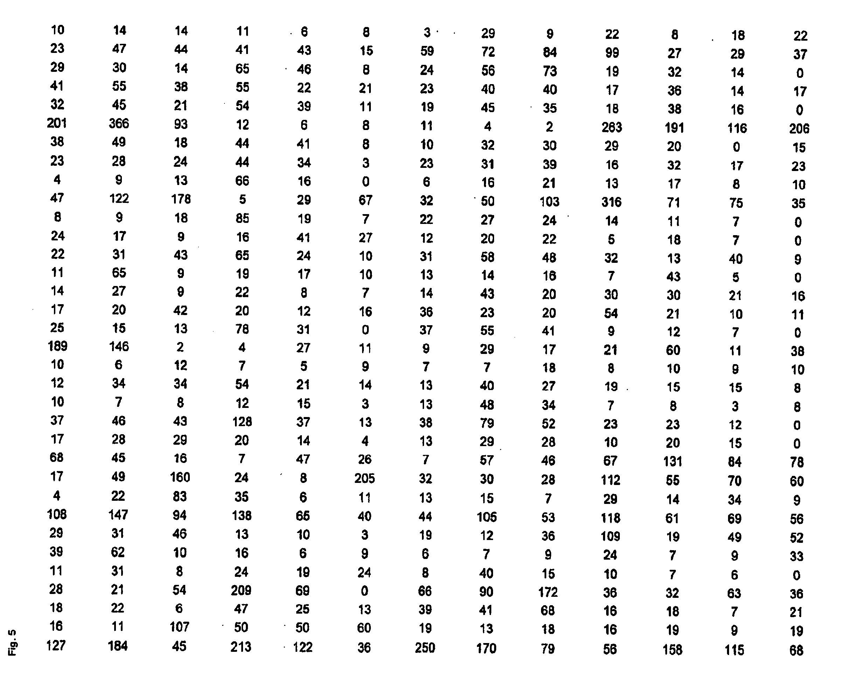

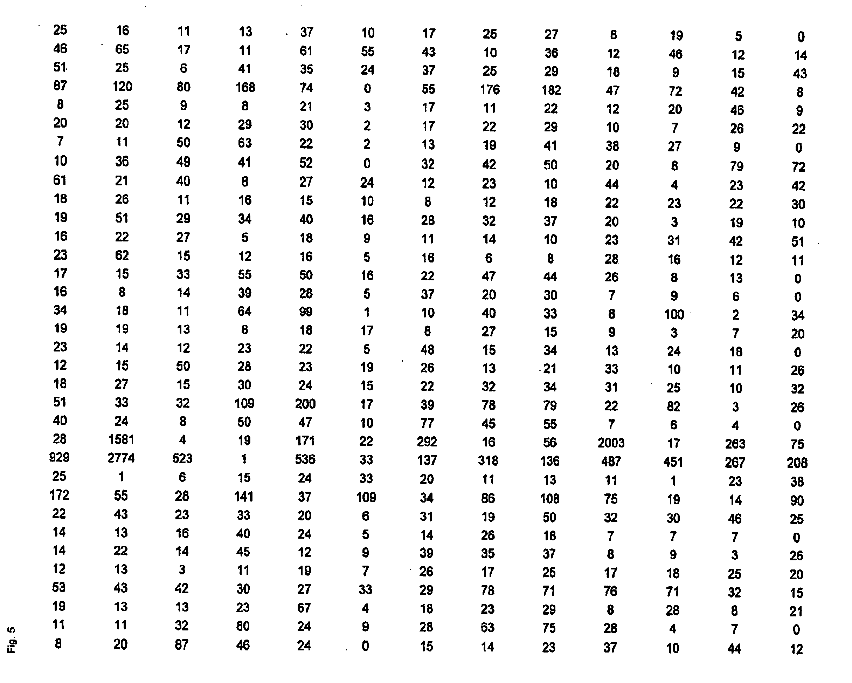

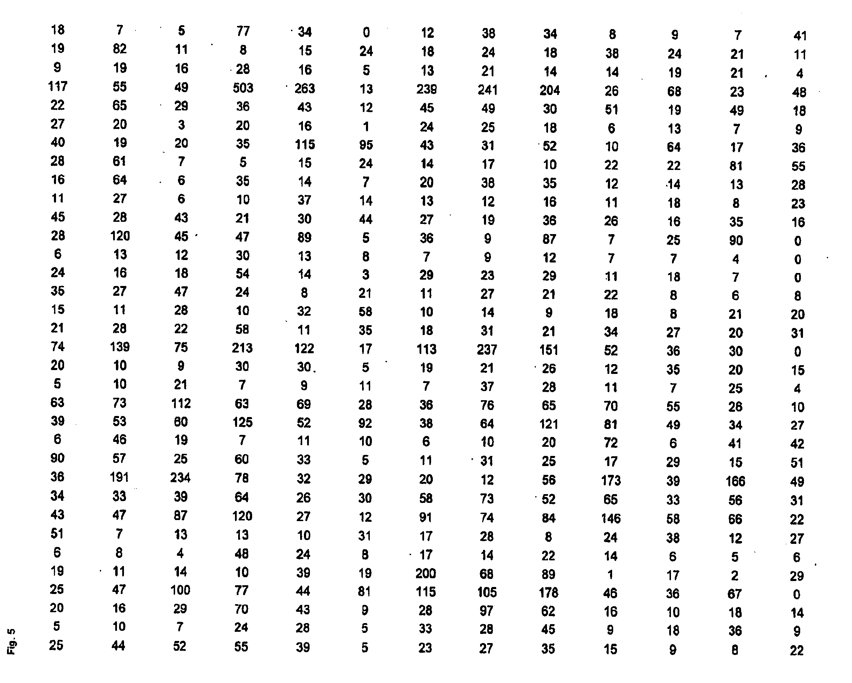

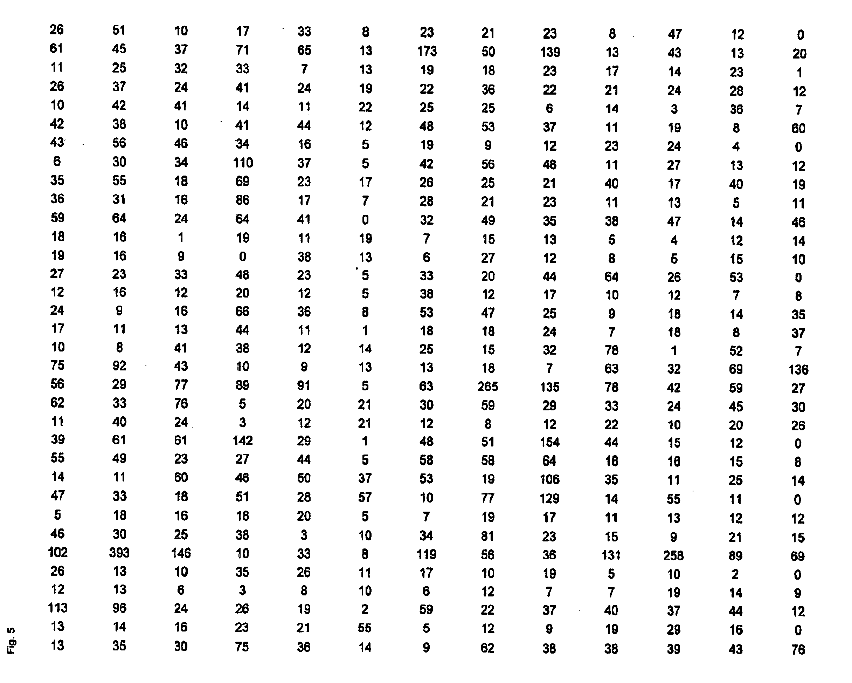

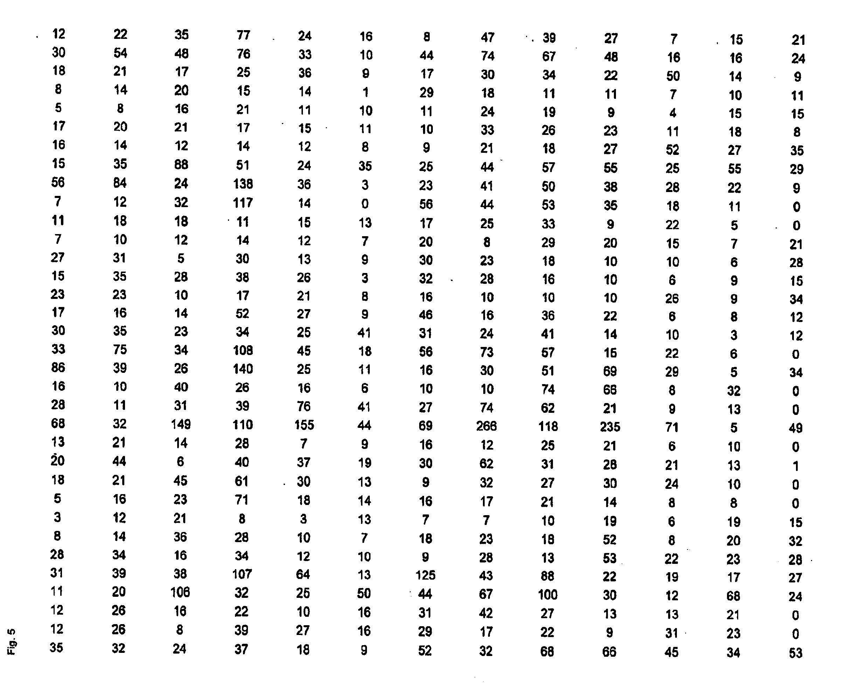

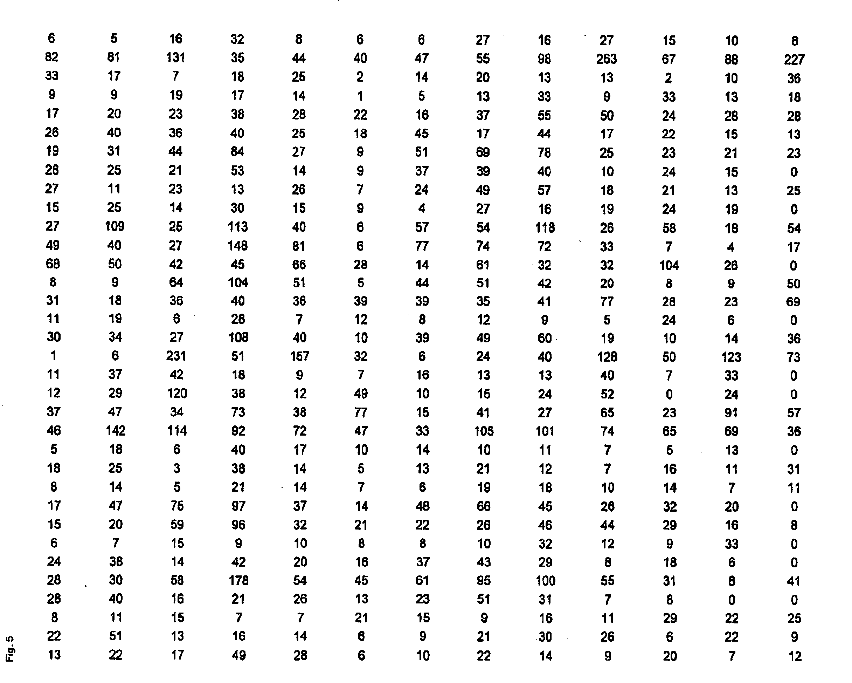

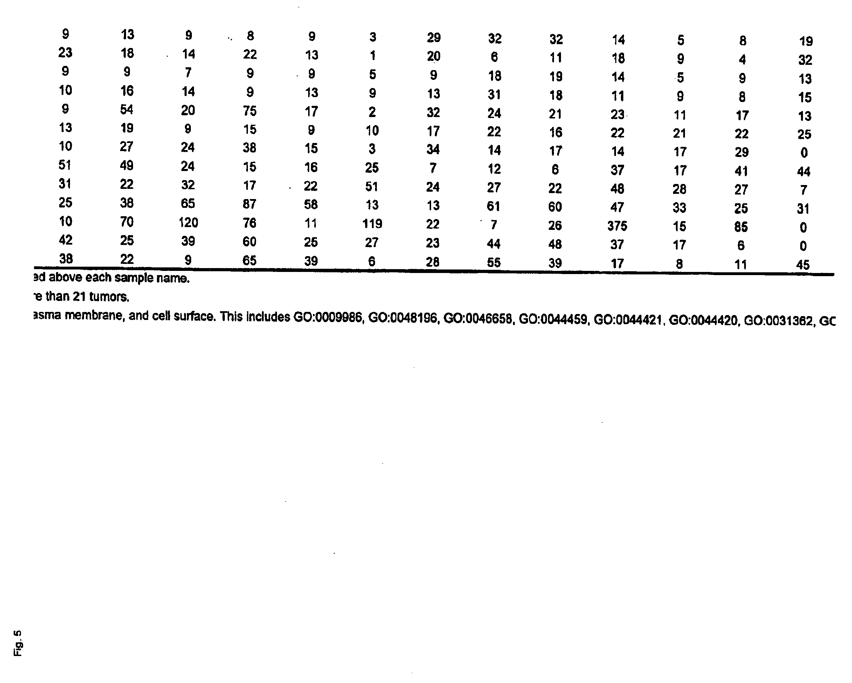

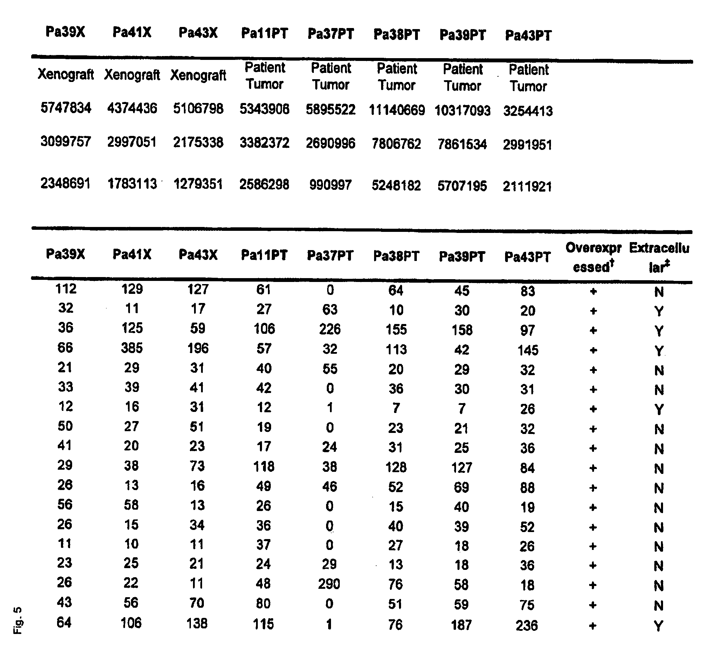

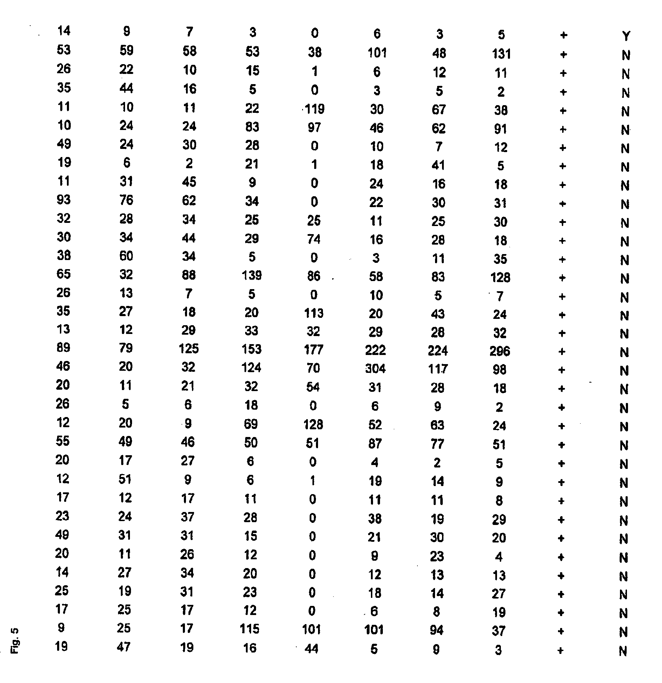

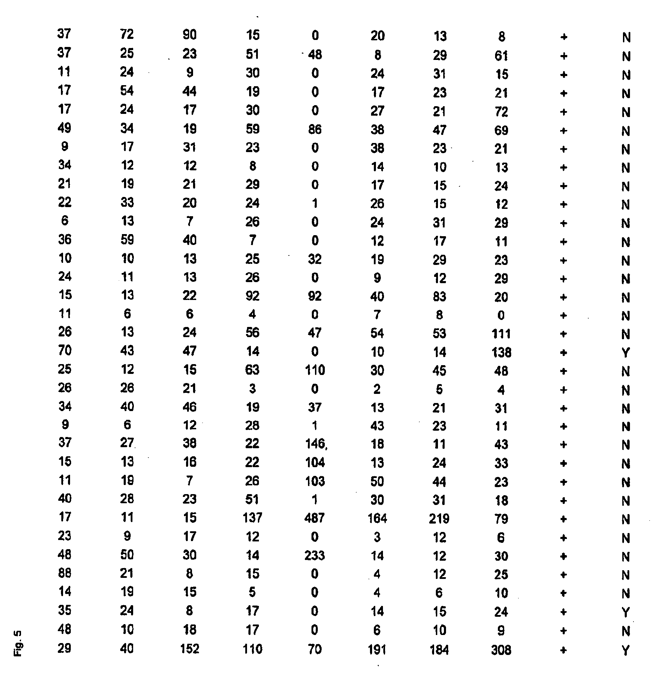

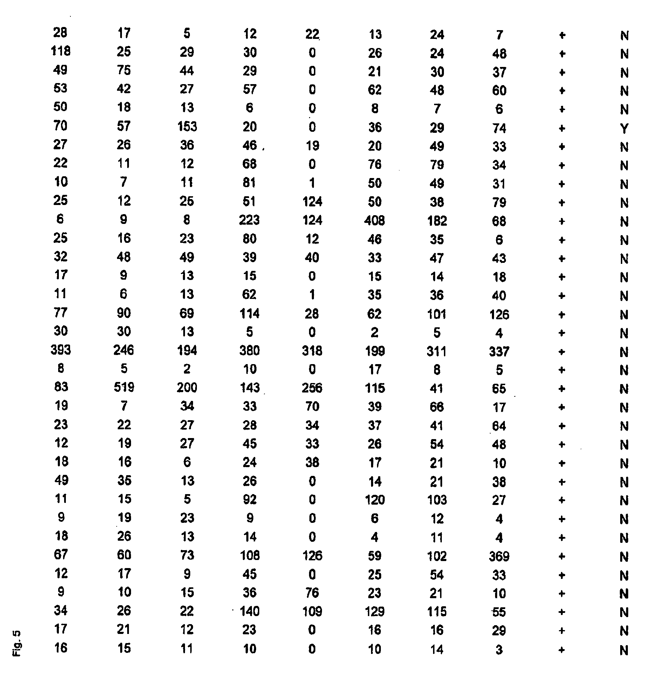

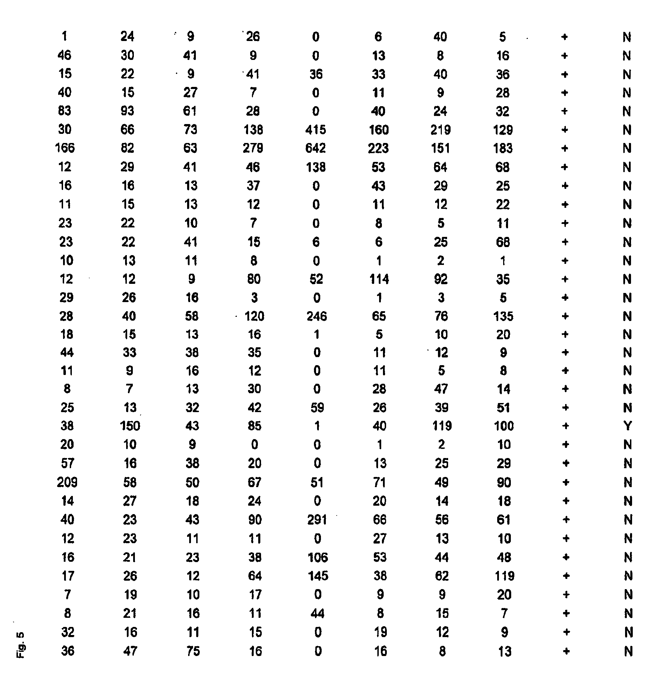

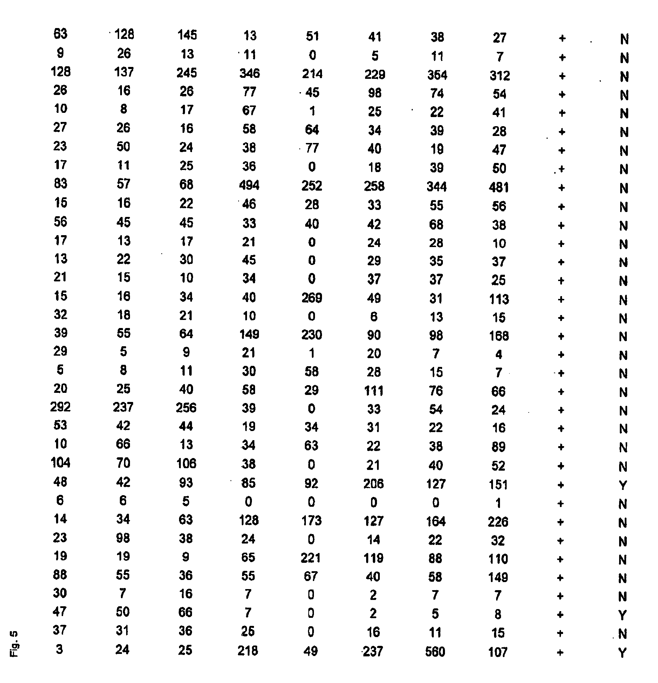

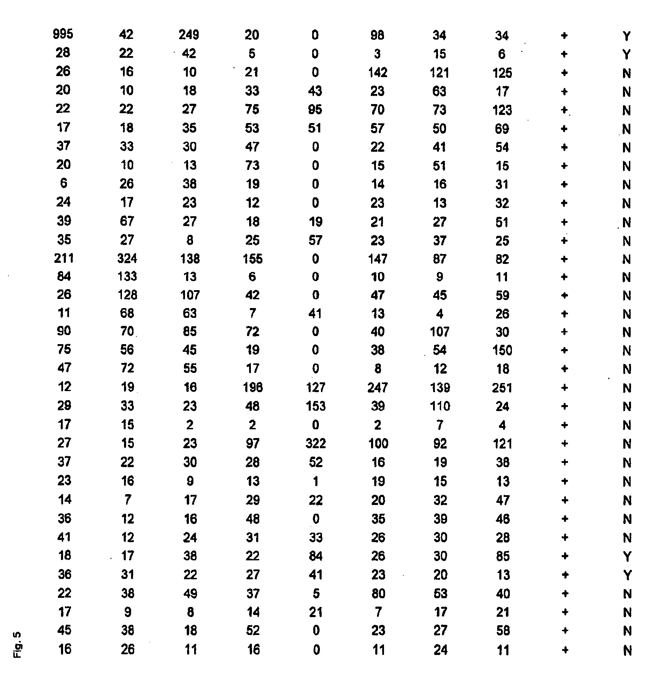

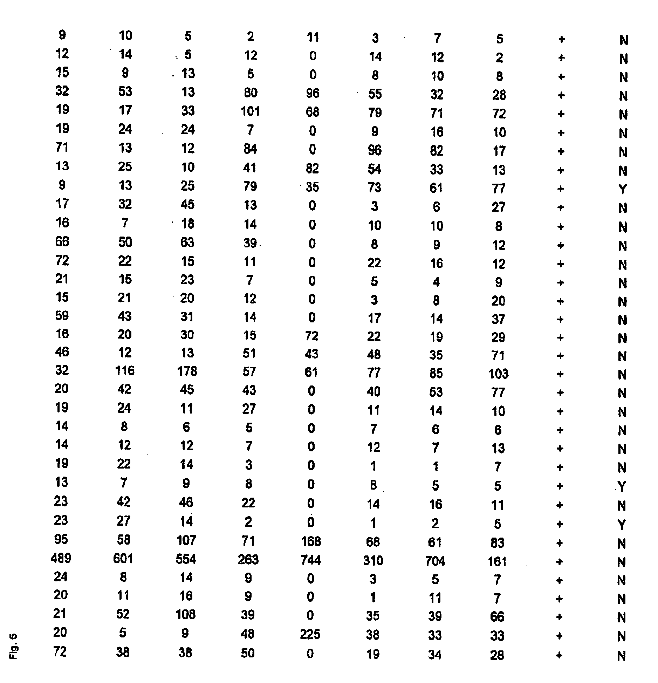

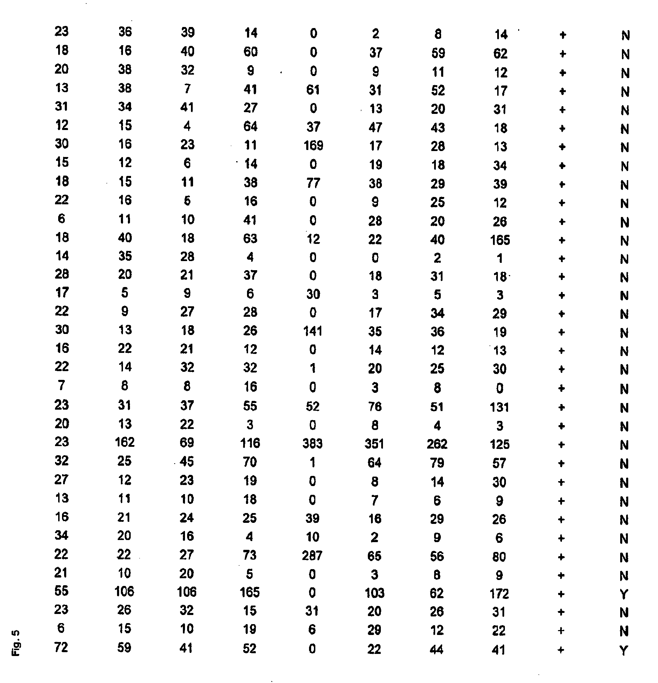

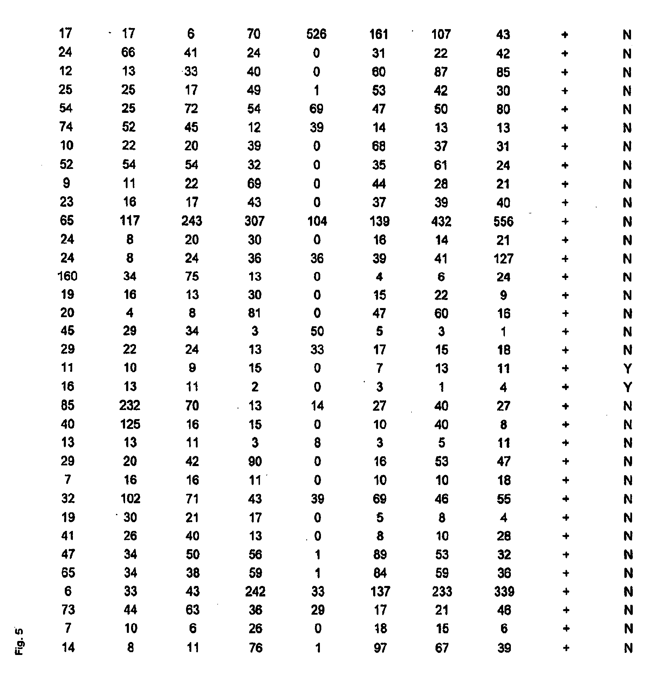

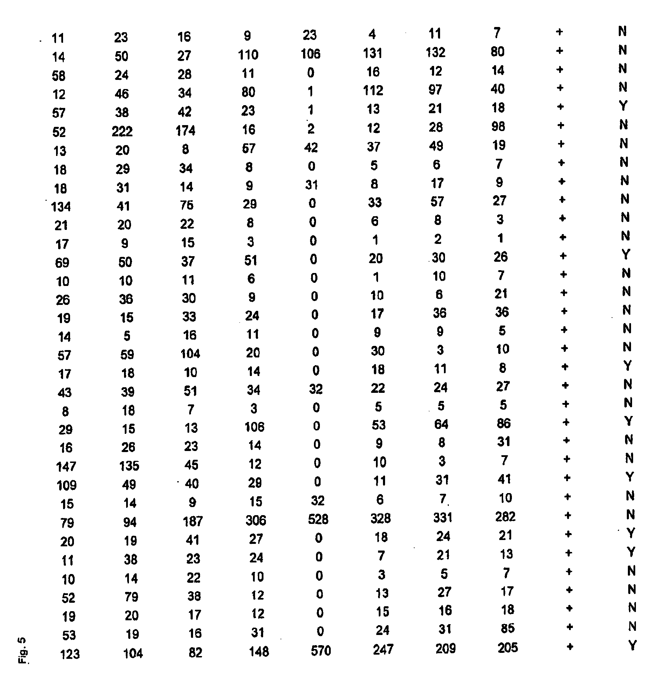

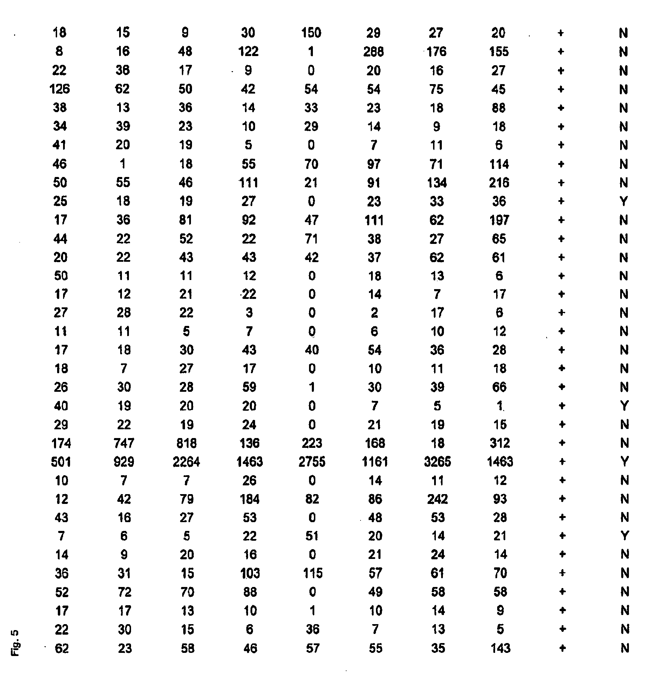

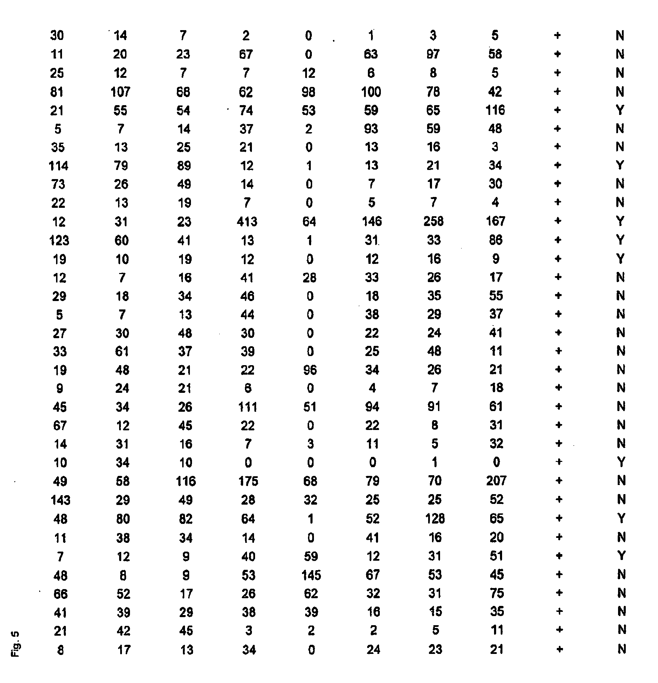

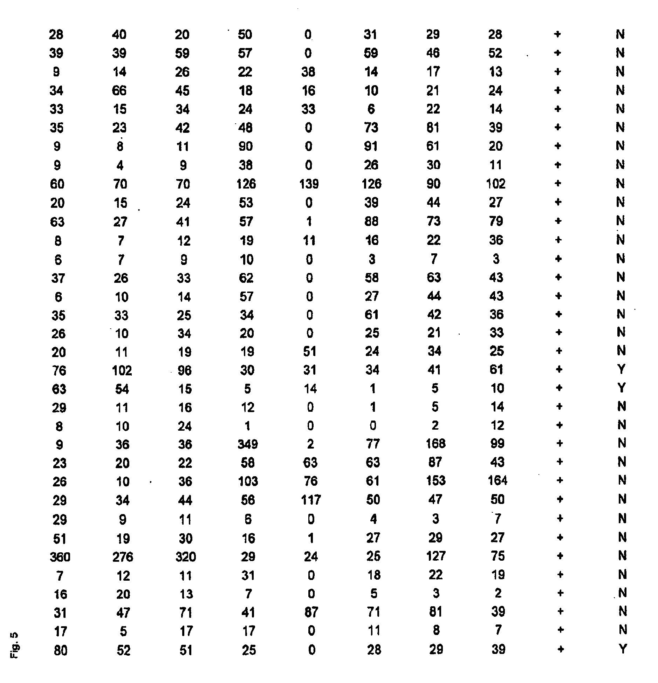

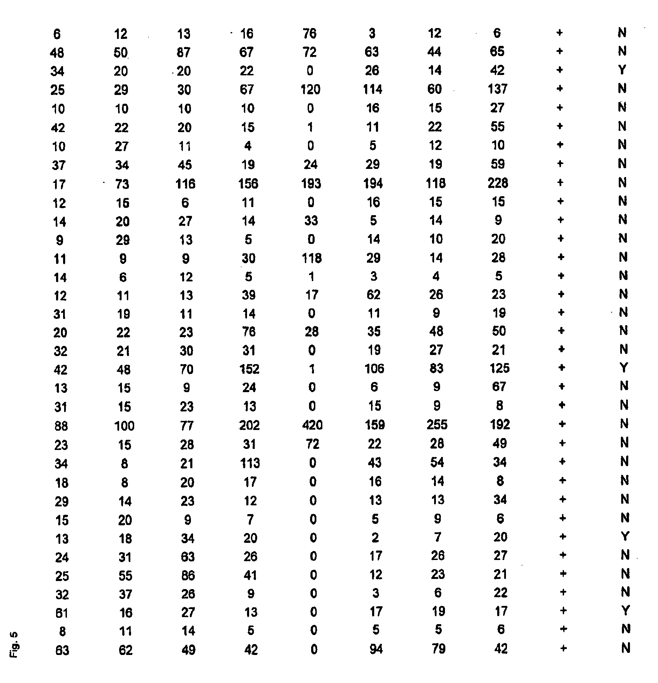

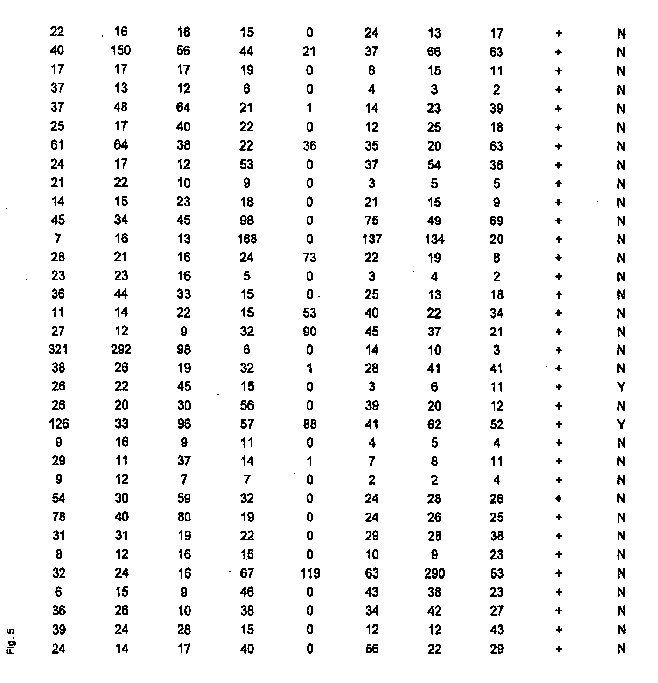

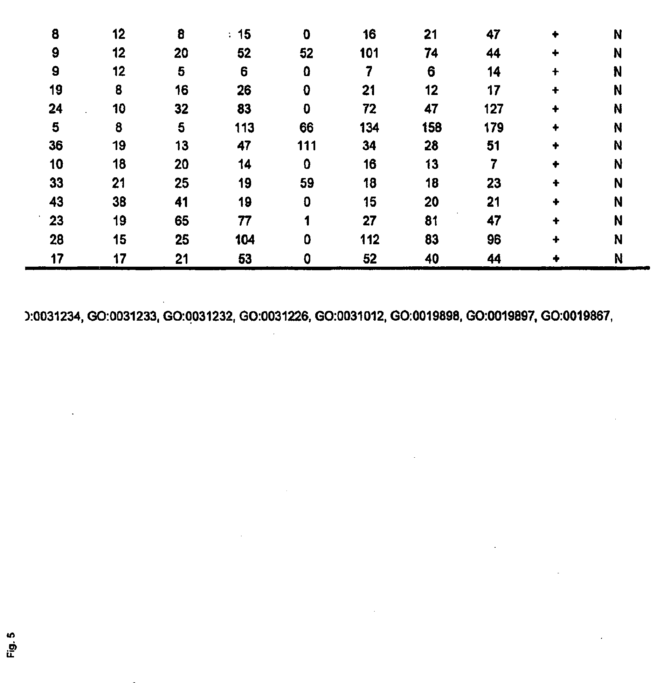

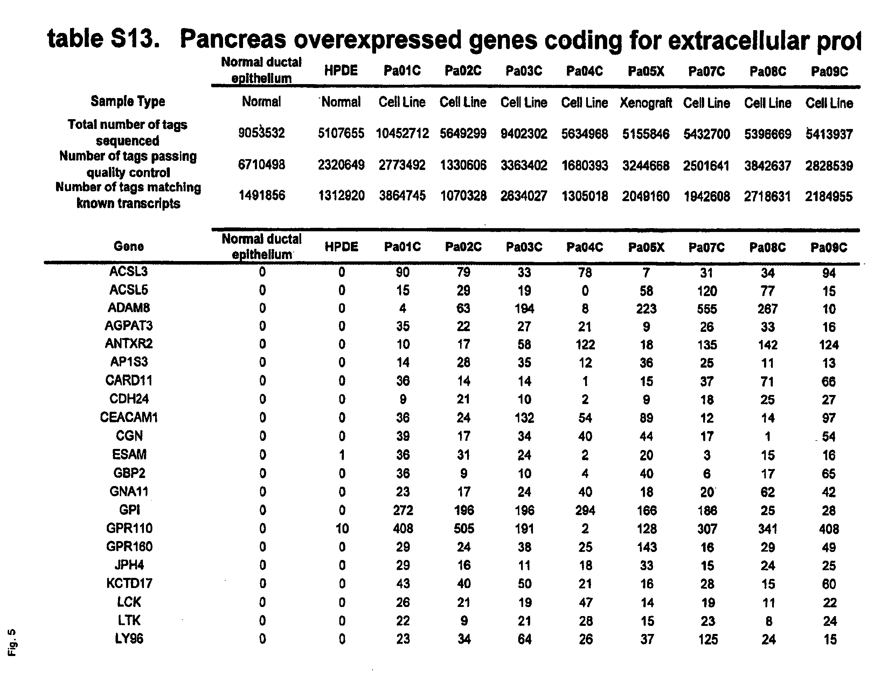

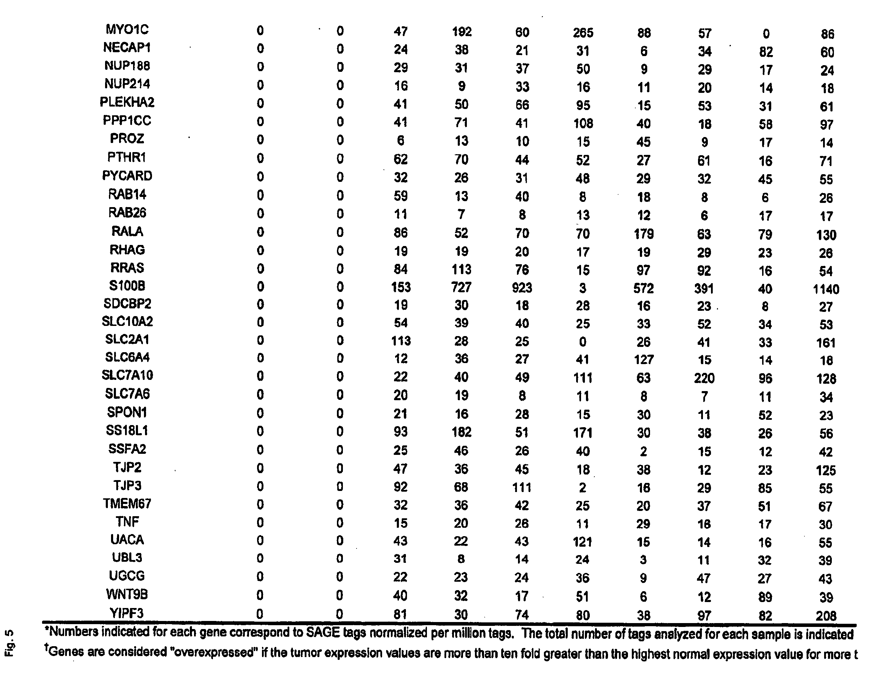

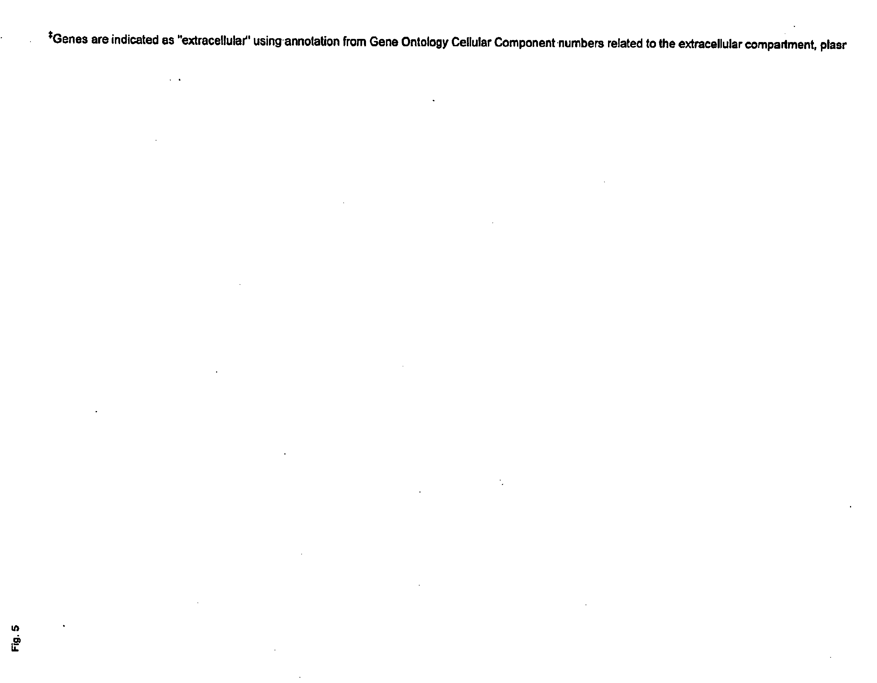

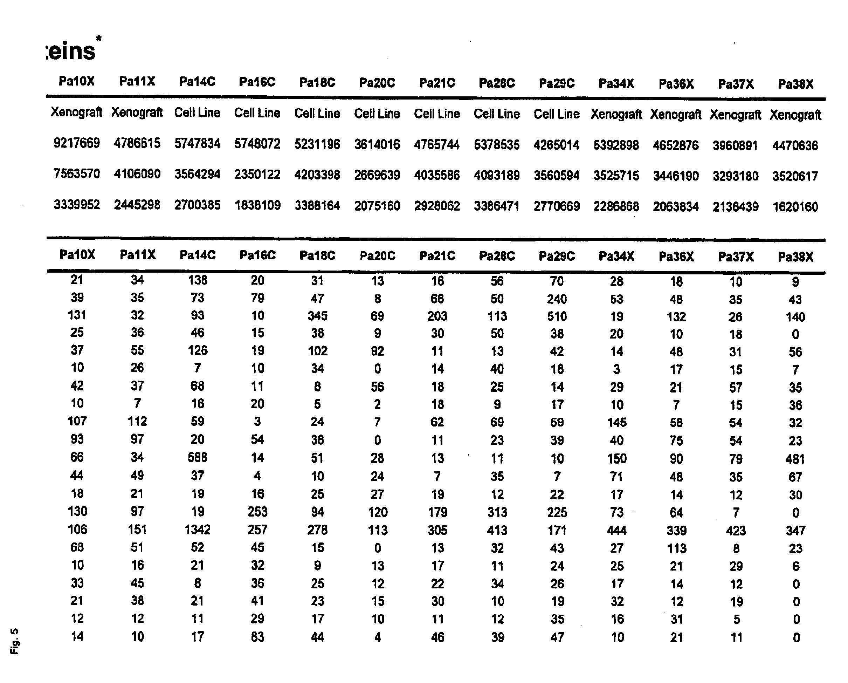

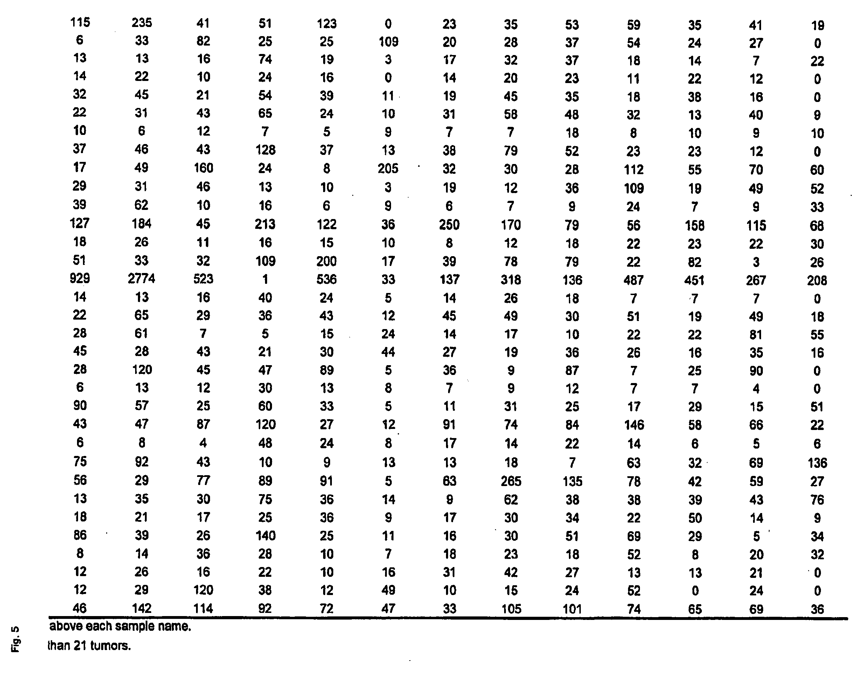

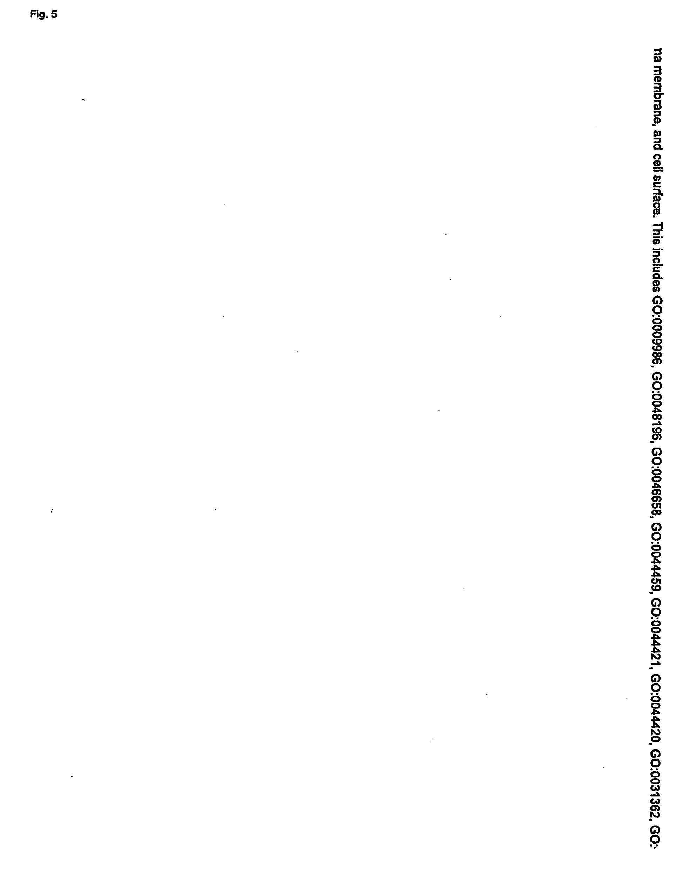

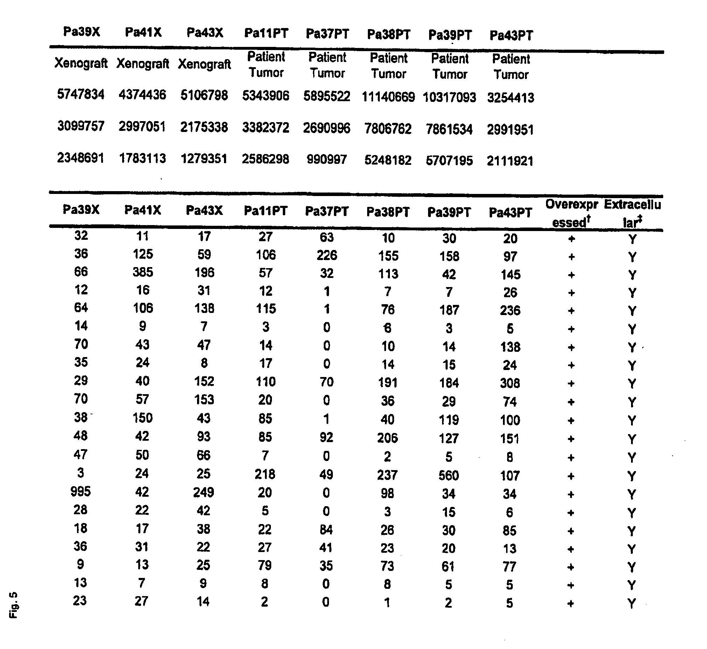

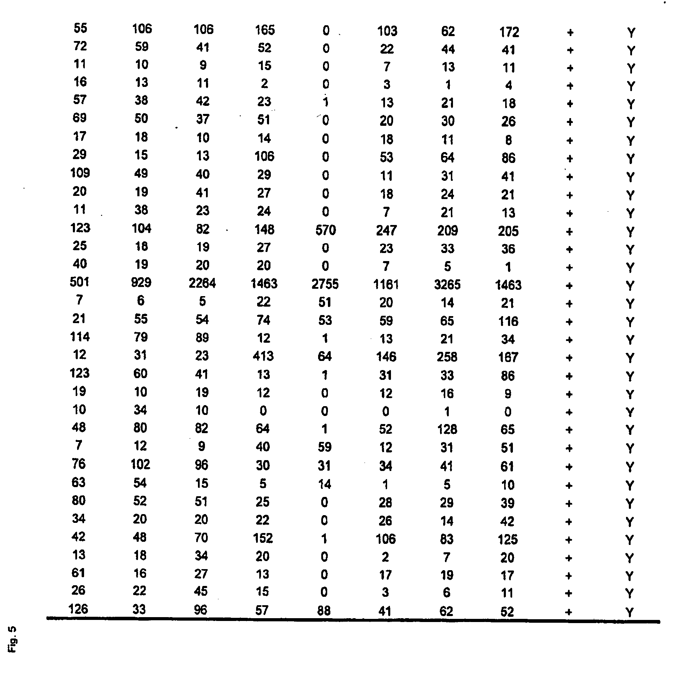

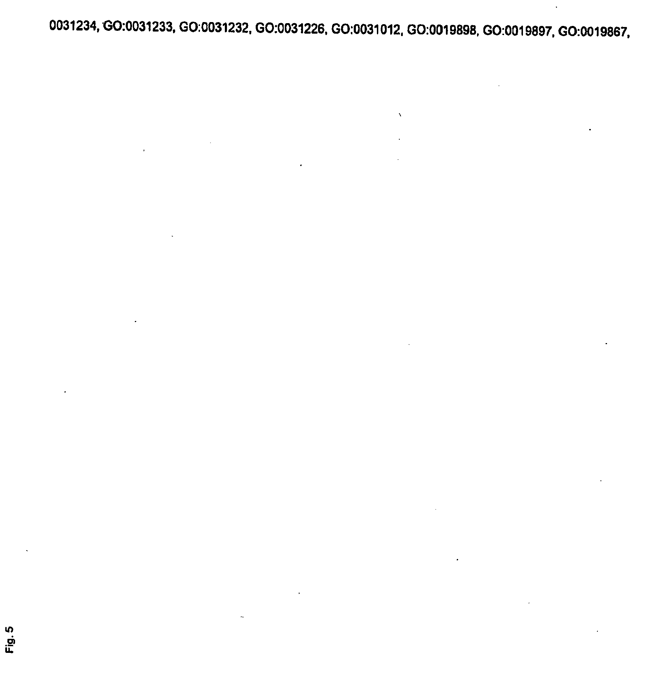

Fig. 5. Supplementary tables S3 (Mutations in Discovery Screen), S4 (Mutation Prevalence Screening), S5 (Homozygous deletions), S6 (amplified genes), S7 (CAN genes), S8 (Pathways frequently mutated), S12 (SAGE overexpressed genes), and S13 (overexpressed, extracellular genes).

### DETAILED DESCRIPTION OF THE INVENTION

The inventors have deeply analyzed pancreatic tumors and developed new therapies, prognosticators, tools, and stratifiers based on the resulting analyses. Using a number of distinct approaches, such as sequencing for mutation, amplification, and deletion detection, and expression quantitation, the inventors have identified key genes, pathways, and mutations. Despite the great genetic heterogeneity between individual pancreatic tumors, patterns of often-mutated genes and pathways have been detected.

Somatic mutations are mutations which occur in a particular clone of somatic cells during the lifetime of the individual organism. The mutation is thus not inherited from parents or passed onto progeny. The mutation will appear as a difference relative to other cells, tissues, organs. When testing for a somatic mutation in a pancreatic tissue suspected of being cancerous, a comparison can be made to normal pancreatic tissue that appears to be non-neoplastic, or to a non-pancreatic sample, such as blood cells, or to a sample from an unaffected individual.

Mutations that have been found in pancreatic tumors are shown in Table S7 or Table 2. These mutations can be detected in test samples, such as suspected tumor tissue samples, blood, pancreatic duct juice, urine, saliva, lymph etc. A somatic mutation is typically determined by comparing a sequence in the test sample to a sequence in a normal control sample, such as from healthy pancreatic tissue. One or more mutations can be used for this purpose. If the patient has undergone surgery, detection of the mutation in tumor margin or remaining adjacent tissue can be used to detect minimal residual disease or molecular relapse. If pancreatic cancer has been previously undiagnosed, the mutation may serve to help diagnose, for example in conjunction with other physical findings of laboratory results, including biochemical markers and radiological findings. Mutations may be used to stratify patients, identifying patients or groups of patients who are sensitive or resistant to drugs or other treatments.

CAN-gene signatures can be determined in order to characterize a pancreatic tumor. A signature is a set of one or more somatic mutations in a CAN gene. The CAN genes for pancreatic are listed in Table S7 and Table 2. Once such a signature has been determined, a pancreatic tumor can be assigned to a group of pancreatic tumors sharing the signature. The group can be used to assign a prognosis, to assign to a clinical trial group, to assign to a treatment regimen, and/or to assign for further characterization and studies. In a clinical trial group, drugs can be assessed for the ability to differentially affect pancreatic tumors with and without the signature. Once a differential effect is determined, the signature can be used to assign patients to drug regimens, or to avoid unnecessarily treating patients in whom the drug will not have a beneficial effect. The drug in a clinical trial can be one which is previously known for another purpose, previously known for treating pancreatic cancer, or previously unknown as a therapeutic. A CAN-gene signature may comprise at least 1, at least 2, at least 3, at least 4, at least 5, at least 6, at least 7, at least 8, at least 9. at least 10 genes. The number of genes or mutations in a particular signature may vary depending on the identity of the CAN genes in the signature.

Analysis of the mutated genes in the analyzed pancreatic tumors has revealed interesting involvement of pathways. Certain pathways frequently carry mutations in pancreatic tumors. Often, a single gene mutation excludes the presence of a mutation in another gene in that pathway in a particular tumor. Frequently mutated pathways in pancreatic tumors are listed in Table S8 and Table 2. Pathways can be defined using any of the standard reference databases, such as MetaCore Gene Ontology (GO) database, MetaCore canonical gene pathway maps (MA) database, MetaCore GeneGo (GG) database, Panther, TRMP, KEGG, and SPAD databases. Groups can be formed based on the presence or absence of a mutation in a certain pathway. Such groups will be heterogeneous with respect to mutated gene but homogeneous with respect to mutated pathway. As with CAN gene signatures, these groups can be used to characterize a pancreatic. Once a mutation in a pathway has been determined, a pancreatic can be assigned to a group of pancreatic tumors sharing the mutated pathway. The group can be used to assign a prognosis, to assign to a clinical trial group, to assign to a treatment regimen, and/or to assign for further characterization and studies. In a clinical trial group, drugs can be assessed for the ability to differentially affect pancreatic tumors with and without the mutated pathway. Once a differential effect is determined, the pathway can be used to assign patients to drug regimens, or to avoid unnecessarily treating patients in whom the drug will not have a beneficial effect. The drug in a clinical trial can be one which is previously known for another purpose, previously known for treating pancreatic, or previously unknown as a therapeutic.

Expression levels can be determined and overexpression may be indicative of a new pancreatic tumor, molecular relapse, or minimal residual disease of pancreatic. Highly increased expression found in pancreatic tumors are shown in Table S6 and Table S12. These overexpressed genes can be detected in test samples, such as suspected tumor tissue samples, blood, pancreatic duct fluid, urine, saliva, lymph etc. Elevated expression is typically determined by comparing expression of a gene in the test sample to expression of a gene in a normal sample, such as from healthy pancreatic tissue. Elevated expression of one or more genes can be used for this purpose. If the patient has undergone surgery, detection of the elevated expression in tumor margin or remaining adjacent tissue can be used to detect minimal residual disease or molecular relapse. If pancreatic has been previously undiagnosed, the elevated expression may serve to help diagnose, for example in conjunction with other physical findings of laboratory results, including biochemical markers and radiological findings. For these purposes, any means known in the art for quantitating expression can be used, including SAGE or microarrays for detecting elevated mRNA, and antibodies used in various assay formats for detecting elevated protein expression. For detecting protein expression, the genes listed in Table S 13 are particularly useful.

Tumor burden can be monitored using the mutations listed in Table S7. This may be used in a watchful waiting mode, or during therapy to monitor efficacy, for example. Using a somatic mutation as a marker and assaying for level of detectable DNA, mRNA, or protein over time, can indicate tumor burden. The level of the mutation in a sample may increase, decrease or remain stable over the time of analysis. Therapeutic treatments and timing may be guided by such monitoring.

Analysis of the pancreatic tumors revealed genes which are homozygously deleted. These are listed in Table S5. Determining loss of expression of one or more of these genes can be used as a marker of pancreatic cancer. This may be done in a sample of blood or lymph node or in a pancreatic tissue sample. Expression of one or more of these genes may be tested. Techniques such as ELISA or IHC may be used to detect diminished or loss of protein expression in a sample. Similarly the homozygously deleted genes listed in Table S5 (and the amplified genes of Table S6) may be used to monitor tumor burden over time. Expression can be repeatedly monitored so that in increase, decrease, or stable level of expression can be ascertained.

The data resulting from this integrated analysis of mutations and copy number alterations have provided a different view of the genetic landscape of pancreatic tumors. The combination of different types of genetic data, including point mutations, amplifications, and deletions allows for identification of individual CAN-genes as well as groups of genes that may be preferentially affected in complex cellular pathways and processes in pancreatic tumors. Identification of virtually all genes previously shown to be affected in pancreatic tumors by mutation, amplification, or deletion validates the comprehensive genomic approach we have employed.

The extensive genetic studies described here suggest that the key to understanding pancreatic cancers lies in an appreciation of a core set of regulatory processes and pathways. We identified 12 such processes that are genetically altered in the great majority of pancreatic cancers (Fig. 3A). However, the pathway components that are altered in any individual tumor vary widely (Fig. 3B, C). For example, the two tumors depicted in Fig. 3B and C each contain a mutation of a gene involved in the TGF-β pathway (one *SMAD4*, the other *BMPR2*). Similarly, these two tumors both contain mutations of genes involved in most of the other 11 core processes/pathways but the genes altered in each tumor are largely different. Though we cannot be certain that every identified mutation plays a functional role in the pathway or process in which it is implicated, it is clear both from the current and previously published genetic data, as well as from past functional studies, that many of them are likely to impact these pathway(s).

This perspective is likely to apply to most, if not all, epithelial tumors. It is entirely consistent with the idea that genetic alterations can be classified as mountains (highfrequency mutations) or hills (low frequency mutations), with the hills predominating in terms of the total number of alterations involved (*16*). The heterogeneity among pathway components and the varied nature of mutations within individual genes can explain tumor heterogeneity, a fundamental facet of all solid tumors (*39*).

From an intellectual viewpoint, the pathway perspective helps bring order and rudimentary understanding to a very complex disease (*40-42*). Though the importance of regulatory processes and pathways in understanding neoplasia in general has been recognized (*43*, *44*), genome-wide genetic analyses such as performed in this study can identify the precise genetic alterations responsible for their dysregulation in each patient's tumor. In addition to yielding insights into tumor pathogenesis, such studies provide the data required for approaches based on personalized cancer medicine. Unlike certain forms of leukemia, in which tumorigenesis appears to be driven by a single, targetable oncogene, pancreatic cancers result from genetic alterations of a large number of genes that function through a relatively small number of pathways and processes. As the *KRAS* oncogene has so far resisted successful targeting and similar new ubiquitously altered targets are not evident, our studies suggest that the best hope for therapeutic development lies in the discovery of agents that target the physiologic effects of the altered pathways and regulatory processes rather than their individual gene components. These effects include metabolic disturbances, neoangiogenesis, misexpression of cell surface proteins, alterations of the cell cycle, cytoskeletal abnormalities, and an impaired ability to repair genomic damage (table S8).

Methods which have been employed for pancreatic cancers have broader application. The methods of identifying genes which are involved in hereditary diseases, can be used for other cancers and for other diseases.

One gene identified as involved in susceptibility to pancreatic cancer is *PALB2.* A mutation in *PALB2* is identified in a pancreatic cancer of a patient. Family members can then be tested to ascertain whether they, too, carry the mutation. If the family member(s) has the mutation, then she is at increased risk of developing pancreatic cancer. If the *PALB2* mutation of the patient is not in the family member, then she is at the same risk as the general population. Testing may be performed by any method known to those of skill in the art. Mutations can be assayed using hybridization of template nucleic acids of the family member to a nucleic acid probe or primer. The template nucleic acids may be genomic or mRNA or cDNA, as examples. The probe or primer may contain at least 14, 16, 18, 20, 22, 24, 26, or 30 nucleic acid bases. The probe or primer may include a part of the *PALB2* which contains a mutation found in a pancreatic tumor. Primers may flank the mutation site and permit the amplification and analysis of the mutation in an amplicon. Particular mutations which may be determined are del TTGT 172-175, G>T at IVS5-1, del A at 3116, and C>T at 3256.

Mutation-specific *PALB2* probes or primers may be combined in kits. The kits may comprise a divided or undivided container. The components of the kit may be separate or mixed. Other elements of the kit in addition to the container may include instructions, reagents such as buffers and enzymes, such as polymerase. Solid supports, reaction tubes, beads, etc. can be included in kits. The kits may contain at least two, three, or four different mutation-specific reagents.

The invention is defined by claims 1-8.

### EXAMPLE 1

**Sample selection.** As with any cancer genomics study, the choice of samples is critical. For this study, we chose 24 advanced adenocarcinomas, each from a different, unrelated patient (table S1). Advanced pancreatic cancers were chosen because they can be expected to contain all of the genetic alterations responsible for tumor initiation and progression while the earlier stage cancers may only contain a subset. The 24 cancers were passaged in vitro as cell lines or in nude mice as xenografts to facilitate detection of mutations. It has been shown that such passaging provides better DNA templates for Sanger sequencing or copy number analyses than primary tumors because it removes contaminating non-neoplastic cells originally present in the tumors (*12*). It has also been demonstrated that the clonal mutations present in cell lines and xenografts rarely, if ever, arise during culture ex vivo (*12-14*)*.*

### EXAMPLE 2

**Sequencing strategy.** The sequences of exons encoding proteins found in the Consensus Coding Sequence (Release 1), Reference Sequence (Release 16) and Ensembl Databases (Release 31) wee extracted and used to design primers for amplification of genomic DNA (fig. S1). In cases wherein previously-designed primers from our past studies of breast and colorectal cancer had proven successful (*15*, *16*), the same primers were used. New primer sets were designed for the 11,579 exons not studied previously as well as for the exons for which previously designed primers proved unsatisfactory (see below) (*17*). The sequence of each of these resultant exons was then determined in 24 pancreatic cancers using dye-terminator sequencing and the 416,622 primers listed in table S2. Exons containing variant sequences were re-amplified and re-sequenced from the tumor DNA to confirm the observed alterations. DNA from normal tissues of the patient harboring the mutation was additionally examined in every case. This approach determined whether the alteration was present in normal cells (and therefore a germ-line variant) or represented a somatic mutation specific to the cancer cells in that individual.

As future medical re-sequencing projects may employ next-generation sequencing-by-synthesis chemistries, it was of interest to determine the coverage obtained with the conventional dye-terminator sequencing methods used in this study. We attempted to evaluate the sequence of the protein-encoding exons of 23,962 transcripts, representing 20,735 genes. The target sequences included all protein-encoding portions, plus four bases upstream and four bases downstream, of each exon. To cover these regions, we designed primers for 219,229 amplicons, of which 208,311 (95%) resulted in PCR products that were successfully sequenced and met our quality controls for further mutational analysis (*17*). These quality-controlled amplicons covered 94.5% of the targeted coding regions and yielded high quality sequencing data for 98.5% of the target bases within the amplicons. In aggregate, we were able to successfully sequence 752,843,968 bp, representing 93.1% of the bases in the coding regions of the targeted transcripts, in these 24 patients. This yielded mutational data on 23,219 transcripts representing 20,661 genes. Note that the primers used for amplification were at minimum, "second generation" primers, with failed primers having been replaced and improved with new primers during each of the large scale sequencing projects previously performed in our laboratory. Thus, this 93.1% value represents close to the maximum achievable with dye-terminator technology. Moreover, the vast majority of the regions that could not be sequenced represented repeated elements rather than sequencing failures per se. Because repeated regions are even more problematic with methods that produce short read lengths, this sequence coverage is not likely to be increased by next-generation technologies.

### EXAMPLE 3

**Somatic mutations.** Among the 1562 somatic mutations, 25.5% were synonymous, 62.4% were missense, 3.8% were nonsense, 5.0% were small insertions and deletions, and 3.3% were at splice sites or within the UTR (Table 1). The spectra of somatic mutations can yield insights into potential carcinogens and other environmental exposure. Table 1 lists the spectra observed in the four tumors that have been subjected to large-scale sequencing analyses of the majority of protein-encoding genes. It is evident that breast tumors have a unique somatic mutation spectrum, with a preponderance of mutations at 5'-TpC sites and a relatively small number of mutations at 5'-CpG sites. However, the spectra of colorectal, brain (*18*), and pancreas tumors are similar, suggesting that breast epithelial cells are exposed to different levels or types of carcinogens, or use different repair systems than the cells giving rise to the other tumors *(19, 20).* Given that cells in the gastrointestinal tract, such as those of the pancreas and colon, are expected to be more exposed to dietary carcinogens than breast or brain cells, one interpretation of these results is that dietary components are not directly responsible for most of the mutations found in human cancers.

**Table 1. Summary of somatic mutations in four tumor types.**

| | **Pancreas*** | **Brain†** | **Colorectalt‡** | **Breast‡** |
|---|---|---|---|---|
| Number of mutated genes | 1007 | 685 | 769 | 1026 |
| Number of nonsynonymous mutations | 1163 | 748 | 849 | 1112 |
| Missense§ | 974 (83.7) | 622 (83.2) | 722(85) | 909 (81.7) |
| Nonsense§ | 60 (5.2) | 43 (5.7) | 48(5.7) | 64 (5.8) |
| Insertion§ | 4 (0.3) | 3 (0.4) | 4 (0.5) | 5 (0.4) |
| Deletion§ | 43 (3.7) | 46 (6.1) | 27(3.2) | 78 (7.0) |
| Duplication§ | 31 (2.7) | 7 (0.9) | 18(2.1) | 3 (0.3) |
| Mutations in non-coding sequences | | | | |
| Splice site or UTR§ | 51 (4.4) | 27 (3.6) | 30(3.5) | 53 (4.8) |
| Total number of substitutions** | 1486 | 937 | 893 | 1157 |
| Substitutions at C:G base pairs | | | | |
| C:G to T:A†† | 798 (53.8) | 601 (64.1) | 534 (59.8) | 422 (36.5) |
| C:G to G:C†† | 142 (9.6) | 67 (7.2) | 61 (6.8) | 325 (28.1) |
| C:G to A:T†† | 246(16.6) | 114(12.1) | 130 (14.6) | 175 (15.1) |
| Substitutions at T:A base pairs | | | | |
| T:A to C:G†† | 142 (9.6) | 87 (9.3) | 69 (7.7) | 102 (8.8) |
| T:A to G:C†† | 79 (5.3) | 24 (2.6) | 59 (6.6) | 57 (4.9) |
| T:A to A:T†† | 77 (5.2) | 44 (4.7) | 40 (4.5) | 76 (6.6) |
| Substitutions at specific dinucleotides | | | | |
| 5'-CpG-3†† | 563 (37.9) | 404 (43.1) | 427 (47.8) | 195 (16.9) |
| 5'-TpC-3'†† | 218(14.7) | 102 (10.9) | 99(11.1) | 395 (34.1) |
| *Based on 24 tumors analyzed in the current study | | | | |
| †Based on 21 nonhypermutable tumors analyzed in Parsons et al., Science, in press 2008. | | | | |
| ‡11 breast and 11 colorectal tumors analyzed in Wood et al., Science 20:1108-13 2007 | | | | |
| §Numbers in parentheses refer to percentage of total nonsynonymous mutations. | | | | |
| **Includes synonymous as well as nonsynonymous mutations identified in the indicated study. | | | | |
| ††Numbers in parentheses refer to percentage of total substitutions | | | | |

Of the 20,661 genes analyzed by sequencing, 1327 had at least one mutation and 148 had two or more mutations among the 24 cancers surveyed (table S3). In addition to the frequency of mutations in a gene, the type of mutation can provide information useful for evaluating its potential role in disease (*21*). Nonsense mutations, out-of-frarne insertions or deletions, and splice site changes generally lead to inactivation of the protein products. The likely effect of missense mutations can be assessed through evaluation of the mutated residue by evolutionary or structural means. To evaluate missense mutations, we developed a novel algorithm that employs machine learning of 58 predictive features based on the physical-chemical properties of amino acids involved in the substitution and their evolutionary conservation at equivalent positions of conserved proteins (*17*). Of the 926 missense mutations that could be scored with this algorithm, 160 (17.3%) were predicted to contribute to tumorigenesis when assessed by this method (table S3).

We were also able to make structural models of 404 of the missense mutations identified in this study (links to structural models available at *(22)).* In each case, the model was based on X-ray crystallography or nuclear magnetic resonance spectroscopy of the normal protein or a closely related homolog. This analysis showed that 55 of the 244 mutations were located close to a domain interface or ligand-binding site and were likely to impact function (examples in Fig. 1).

Our analysis of all the protein-encoding genes provides a detailed picture of the compendium of genetic alterations in an individual tumor. As shown in Fig. 2, pancreatic cancers had an average of 48 somatic mutations in protein-encoding genes per tumor. The variation in this number was remarkably small given the complexity of the tumorigenic process and the varied ages of the patients (table S1). The average number of somatic mutations in pancreatic cancers is considerably less than in breast or colorectal cancers (p < 0.001), even though fewer genes were sequenced in the latter two tumor types (*16*). One plausible explanation for this lower rate is that the cells that initiate pancreatic tumorigenesis have gone through fewer divisions than colorectal or breast cancer cells. It has been previously shown that the majority of mutations observed in colorectal cancers are likely to have occurred in the normal stem cells that gave rise to the initiating neoplastic cell (*14*). Our data is thus consistent with observations showing that pancreatic epithelial cells divide infrequently (*23*, *24*) while mammary and colorectal epithelial cells divide frequently, the former during periods of hormonal stimulation and the latter throughout life.

We further evaluated 39 genes that were mutated in more than one of the 24 Discovery Screen cancers in a Prevalence screen consisting of 90 pancreatic cancers. In this screen, we detected 255 non-silent somatic mutations among 23 genes (table S4). The non-silent mutation rate of the genes in the Prevalence screen (excluding *KRAS, TP53, CDK2NA,* and *SMAD4* was higher than that in the Discovery Screen (3.6 vs. 1.47 non-silent mutations/Mb, p < 0.0001). The fraction of non-silent mutations observed in these 19 genes was also higher than that observed in the Discovery Screen (p < 0.052). These data are consistent with the hypothesis that a greater fraction of the genes tested in the Prevalence screen were positively selected during tumorigenesis.

### EXAMPLE 4

**Deletions.** An important aspect of the design of the current study was the use of DNA from cell lines or xenografts. This DNA permits confident detection of true homozygous deletions, a task that is very difficult with the DNA from most primary tumor specimens because of the contamination of non-neoplastic stromal and inflammatory cells. Through comparisons of SNP-array data, Digital Karyotyping, and real-time PCR analysis, we have previously developed robust algorithms for confidently identifying. deletion events in such samples from SNP-array data (*25*). When these algorithms were used to analyze data from Illumina oligonucleotide arrays containing probes for 1,069,688 SNPs, we detected 198 separate homozygous deletions in the 24 pancreatic cancers used for mutational analysis (table S5). The average size of these deletions was 335,000 bp. In addition to homozygous deletions, we observed many regions that had undergone single copy losses, often manifest as losses of heterozygosity, including losses of whole chromosomes or whole chromosome arms. We did not pursue these changes as it is difficult to reliably identify target genes from such large regions unless the residual copy of the gene on the non-deleted chromosome is mutated. Such target genes would have already been called to our attention by the results of the Discovery sequencing screen and would have been scored as homozygous changes (table S3).

According to the allelic two-hit hypothesis, the presence of a homozygous deletion indicates that a tumor suppressor gene exists within the deleted region (*26*). To determine the most likely target within these deletions, we used the results from our new mutational and expression analysis as well as data from past studies. For a gene to be considered the candidate target, a portion of its coding region had to be affected by the homozygous deletion and (i) the gene had to harbor a non-silent sequence alteration in a different tumor from the Discovery Screen or (ii) had to be a well-documented tumor-suppressor gene or (iii) had to have corroborating expression data (see gene expression section below). The presumptive target genes for each of the homozygous deletions that met these criteria are listed in table S5. This list includes the classic tumor-suppressor genes *CDKN2A* (p16), *SMAD4,* and *TP53* as well as a variety of other genes that have not previously been implicated in pancreatic tumorigenesis.

To confirm the homozygous deletions found through the SNP-arrays, we reanalyzed the sequencing data. When an exon of a gene is truly deleted in a tumor, no sequencing information should be obtained from the attempted amplification of that exon. Without exception, the sequencing data thereby confirmed the deletions identified through the microarray hybridizations. Furthermore, there was only one homozygous deletion revealed by sequencing that was not evident in the microarray hybridizations (a four-exon deletion of SMAD4 in a single tumor).

The number of deletions in a tumor was more variable than the number of somatic mutations, averaging 8.2 and ranging between 2 and 20 per tumor (Fig. 2). However, it should be noted that each homozygous deletion completely abrogated the function of the target gene as well as all other genes within the deleted region, while only a fraction of the somatic mutations were predicted to alter the gene's function. In an average pancreatic cancer, a total of ~10 genes (including targets and nearby genes within the deletion) are eradicated from the tumor's genome by homozygous deletion, providing fertile grounds for therapeutic strategies that target such losses (*27*, *28*))*.*

### EXAMPLE 5

**Amplifications.** As with deletions, we have developed algorithms for confidently identifying amplifications from SNP-array data (25). Using a combination of individual fluorescence intensity ratio measurements from the Illumina arrays, as well as the minimum, maximum, and average intensity ratios over contiguous regions of copy number changes, we identified a variety of low copy number gains of entire chromosomes, chromosomal arms, or other large genomic regions. We did not pursue these copy number changes further as it is difficult to reliably identify candidate cancer genes from such large chromosomal regions. Moreover, virtually all well-documented amplifications promoting tumor growth or drug resistance involve relatively small regions of amplification (*29*). We therefore focused on focal amplifications that were clearly the result of true amplification rather than aneuploidy.

Using rigorous criteria for focal amplification, including the presence of>12 copies of the amplified region per nucleus (*17*), we identified 144 amplifications among the 24 pancreatic cancers (table S6). To determine the most likely target of these amplifications, we again used the results from our mutational and expression analyses as well as previously published data. For a gene to be considered as the target of amplification, its entire coding region had to be included in the amplified region and it (i) had to be mutated in a different tumor from the Discovery Screen or (ii) had to be a well-documented oncogene or (iii) had to have corroborating expression data (see gene expression section below). The presumptive target genes for each of the amplifications that met these criteria are listed in table S6. There were fewer amplifications than homozygous deletions or point mutations in most pancreatic tumors (Fig. 2).

### EXAMPLE 6

**Passenger mutation rates.** The primary goal of cancer genome studies is the identification of genes that play a causal role in the neoplastic process (drivers). However, many genes accumulate relatively harmless mutations (passengers) during this decades-long process. For most mutated genes, it is therefore difficult to definitively implicate a causal role for that gene on the basis of its mutations alone (*12, 15, 30*)*.* One can, however, categorize the best candidate cancer genes (*CAN*-genes) on the basis of their mutation frequencies and types. To determine which genes are most likely to drive tumorigenesis, an estimate of the passenger mutation rate is required (*16, 30*)*.*

The passenger mutation rate cannot be directly determined from mutational data because it is impossible to distinguish passenger from driver mutations a priori. However, it is reasonable to assume that most silent (synonymous or S) mutations do not lead to a positive or negative effect on cell growth. From the synonymous mutations observed in the current study, it is possible to estimate the lower bound of the passenger rate of non-synonymous (NS) mutations in the 24 cancers (*17*). The lower bound was defined as the product of the synonymous mutation rate and the NS:S ratio (1.02) observed in the HapMap database of human polymorphisms. This is likely an underestimate because selection against certain nonsynonymous mutations may be more stringent in the germline than in somatic cells. The upper bound was determined by the total number of mutations observed (after excluding the mutations in *SMAD4, CDK2NA, TP53,* and *KRAS).* This is likely an overestimate as it assumes that none of the mutations other than those in previously known genes were drivers:

For each of the genes containing somatic mutations, passenger probabilities were determined with the low and high mutation rate boundaries as well as with a mid-rate that was the average of the two. These passenger probabilities took into account the size of the gene, its nucleotide (nt) composition, and the relative frequencies of mutations at individual nucleotides and dinucleotides in pancreatic cancers (Table 1 and (*17*)). To analyze the probability that a given gene would be involved in an amplification or deletion, we made the conservative assumption that the overall frequency of all observed amplifications and deletions represented the passenger mutation rate. The number of actual copy number alterations affecting each gene in all tumors was then compared to the simulated number of expected passenger copy number alterations taking into account gene size and the distribution of SNP locations.

*CAN*-genes could then be chosen from among the list of mutated genes by their low combined passenger probabilities of point mutations, small deletions or insertions, homozygous deletion, or amplification. The top-ranking *CAN*-genes are listed in table S7 and include all genes previously known to play a significant role in pancreatic cancer (e.g., *RAS, SMAD4,* CDKN2A, and *TP53*)*.* The identification of mutations and copy number changes in these genes provided unambiguous experimental confirmation of our general approach. Importantly, the *CAN*-*genes* included numerous other genes of potential biological interest, many of which had not previously been identified to play a role in this tumor type. Examples include the transcriptional activator *MLL3,* the TGF-β receptor *TGBBR2,* cadherin homologs *CDH10, PCDH15*, *and PCDH18,* the α-catenin *CTNNA2,* the dipeptidyl-peptidase *DPP6,* the angiogenesis inhibitor *BAI3,* the G-protein coupled receptor *GPR133,* the guanylate cyclase *GUCY1A2,* the protein kinase *PRKCG,* and *Q9H5F0,* a gene of unknown function. These genes were generally mutated at much lower frequencies than those previously identified to be mutated in pancreatic cancers. This is compatible with the idea that conventional strategies were able to identify frequently mutated genes but not the bulk of the genes that are genetically altered in pancreatic cancers.

### EXAMPLE 7

**Candidate pathways promoting pancreatic tumorigenesis.** Because all of the protein-coding genes in the human genome were evaluated in the current study, the data provide a unique opportunity to investigate genetically altered pathways and processes at a genome-wide level. We developed a statistical approach that provided a combined probability that a pathway or process contained driver alterations, taking into account all types of genetic alterations evaluated in this study (*22*). We then applied the approach to groups of genes involved in cellular pathways or processes defined through three well annotated GeneGo MetaCore databases: gene ontology (GO), canonical gene pathway maps (MA), and genes participating in defined cellular processes and networks (GG) (31). For each gene group, we considered whether the component genes were more likely to be affected by a genetic alteration than predicted by the passenger rate. These analyses were based on analysis of the rankings of altered genes within each group rather than the total number of mutations within individual groups of genes.

These analyses identified pathways and regulatory processes which were not only statistically significant but also were altered in the great majority of the 24 cancers examined (Table 2 and table S8). These included pathways in which a single, frequently altered gene predominated, such as in KRAS signaling and in the regulation of the G1/S transition; pathways in which a few altered genes predominated, such as in TGF-β signaling; and pathways in which many different genes were altered, such as in integrin signaling, regulation of invasion, homophilic cell adhesion, and small GTPase-dependent signaling.

**Table 2. Core signaling pathways and processes genetically altered in most pancreatic cancers**

| **Regulatory Process or Pathways.** | **Number of genetically altered genes detected** | **Fraction of tumors with genetic alteration of at least one of the genes** | **Representative altered genes** |
|---|---|---|---|
| Apoptosis | 9 | 100% | CASP10, VCP, CAD, HIP1 |
| DNA damage control | 9 | 83% | ERCC4, ERCC6, EP300, RANBP2, TP53 |
| Regulation of G1/S phase transition | 19 | 100% | CDKN2A, FBXW7, CHD1, APC2 |
| Hedgehog signaling | 19 | 100% | TBX5, SOX3, LRP2, GLI1, GLI3, BOC, BMPR2, CREBBP |
| Homophilic cell adhesion | 30 | 79% | CDH1, CDH10, CDH2, CDH7, FAT, PCDH15, PCDH17, PCDH18, PCDH9, PCDHB16, PCDHB2, PCDHGA1, PCDHGA11, PCDHGC4 |
| Integrin signaling | 24 | 67% | ITGA4, ITGA9, ITGA11, LAMA1, LAMA4, LAMA5, FN1, ILK |
| JNK signaling | 9 | 96% | MAP4K3, TNF, ATF2, NFATC3 |
| KRAS signaling | 5 | 100% | KRAS, MAP2K4, RASGRP3 |
| Regulation of invasion | 46 | 92% | ADAM11, ADAM 12, ADAM19, ADAM5220, ADAMTS15, DPP6, MEP1A, PCSK6, APG4A, PRSS23 |
| Small GTPase-dependent signaling (other than KRAS) | 33 | 79% | AGHGEF7, ARHGEF9, CDC42BPA, DEPDC2, PLCB3, PLCB4, RP1, PLXNB1, PRKCG |
| TGF-□signaling | 37 | 100% | TGFBR2, BMPR2, SMAD4, SMAD3 |
| Wnt/Notch signaling | 29 | 100% | MYC, PPP2R3A, WNT9A, MAP2,TSC2, GATA6, TCF4 |

| | | | |
|---|---|---|---|
| * A complete listing of the gene sets defining these signaling pathways and processes and the statistical significance of each gene set are provided in table S8. | | | |

### EXAMPLE 8

**Analysis of gene expression.** Gene expression patterns can inform the analysis of pathways because they can reflect epigenetic alterations not detectable by sequencing or copy number analyses. They can also point to downstream effects on gene expression resulting from the altered pathways described above. To analyze the transcriptome of pancreatic cancers, we performed SAGE (serial analysis of gene expression, (32)) on RNA from the same 24 cancers used for mutation analysis. When combined with massively parallel sequencing-by-synthesis, SAGE provides a highly quantitative and sensitive measure of gene expression. The sequencing-by-synthesis approach used to carry out this analysis was similar to that used in recent RNA-Seq studies (*33-36*), but SAGE has the advantage that the quantification does not depend on the length of the transcript, thereby maximizing the information resulting from the sequence of a given number of tags.

As a control for the current study, we microdissected histologically normal pancreatic duct epithelial cells. Though this microdissection is technically challenging, these cells are the presumed precursors of pancreatic cancers. As an additional control, we used HPV-immortalized pancreatic duct epithelial cells (HPDE), which have been shown to have many properties in common with normal duct epithelial cells (*37*, *38*). SAGE libraries were prepared from these cells as well as the 24 pancreatic cancers; an average of 5,737,000 tags was obtained from each library, and an average of 2,268,000 tags per library matched the sequence of known transcripts.

The transcript analysis was first used to help identify target genes from the amplified and homozygously deleted regions that were identified in this study. Though a small fraction of these regions contained a known tumor-suppressor gene or oncogene, many contained more than one gene that had not previously been implicated in cancer. In tables S5 and S6, a presumptive target gene was identified within these regions through the use of the mutational as well as transcriptional data. For example, we assumed that a gene could not have been the target of an amplification event if that gene was not expressed in the tumor containing the amplification. Similarly, we assumed that a true tumor suppressor gene within a deletion should be expressed in the normal pancreatic ductal epithelium but not in the corresponding cancer.

Second, we determined whether the genes in the core signaling pathways and processes described above were differentially expressed. If the pathways and processes containing genetic alterations were indeed responsible for tumorigenesis, one might expect that many of the genes within these pathways would be aberrantly expressed. To test this hypothesis, we examined the expression of the gene sets constituting the 12 core signaling pathways and processes (Table 2 and table S8). The 31 gene sets constituting these pathways were more highly enriched for differentially expressed genes than the remaining 3041 gene sets (p<0.001). These expression data thus independently support the contribution of these signaling pathways and processes to pancreatic tumorigenesis.

Finally, we attempted to identify individual genes rather than pathways that were differentially expressed in the cancers. The data collected represent the largest compendium of digital expression data derived for any tumor type to date. There was a remarkably high number (541) of genes that were at least 10-fold overexpressed in >90% of the 24 cancers (compared to normal pancreatic duct cells or HPDE). To determine if these genes were also overexpresssed in the primary tumors from which the cell lines were made, we performed SAGE on five such primary tumors. These results confirmed these 541 genes' overexpression in situ: the genes were, on average, expressed at 75-fold higher levels in the cell lines and at 88-fold higher levels in the primary tumors compared to normal duct epithelial cells. It was notable that 54 of the overexpressed genes encoded proteins that are predicted to be secreted or expressed on the cell surface. These overexpressed genes provide leads for a variety of diagnostic and therapeutic approaches.

### References

The disclosure of each reference cited is expressly incorporated herein.

### References and Notes

1. D. M. Parkin, F. I. Bray, S. S. Devesa, Eur J Cancer 37 Suppl 8, S4 (2001).
2. A. Jemal et al., CA Cancer J Clin 58, 71 (2008).
3. J. B. Koorstra, S. R. Hustinx, G. J. Offerhaus, A. Maitra, Pancreatology 8, 110 (2008).
4. S. A. Hahn, D. K. Bartsch, Clin Lab Med 25, 117 (2005).
5. R. H. Hruban et al., Am J Surg Pathol 25, 579 (2001).
6. E. Efthimiou, T. Crnogorac-Jurcevic, N. R. Lemoine, Pancreatology 1, 571 (2001).
7. M. Mimeault, R. E. Brand, A. A. Sasson, S. K. Batra, Pancreas 31, 301 (2005).
8. D. A. Tuveson, S. R. Hingorani, Cold Spring Harb Symp Quant Biol 70, 65 (2005).
9. E. M. Jaffee, R. H. Hruban, M. Canto, S. E. Kern, Cancer Cell 2, 25 (2002).
10. A. Maitra, S. E. Kern, R. H. Hruban, Best Pract Res Clin Gastroenterol 20, 211 (2006).
11. A. Maitra, R. H. Hruban, Annu Rev Pathol 3, 157 (2008).
12. J. M. Winter, J. R. Brody, S. E. Kern, Cancer Biol Ther 5, 360 (2006).
13. B. Rubio-Viqueira et al., Clin Cancer Res 12, 4652 (2006).
14. S. Jones et al., Proc Natl Acad Sci USA 105, 4283 (2008).
15. T. Sjoblom et al., Science 314, 268 (2006).
16. L. D. Wood et al., Science 318, 1108 (2007).
17. See supporting material on *Science* Online.
18. D. W. Parsons, Co-submitted to Science (2008).
19. A. Hartmann, H. Blaszyk, J. S. Kovach, S. S. Sommer, Trends Genet 13, 27 (1997).
20. S. P. Hussain, C. C. Harris, Mutat Res 428, 23 (1999).
21. P. C. Ng, S. Henikoff, Nucleic Acids Res 31, 3812 (2003).
22. R. Karchin. (2008). Structural models of mutants identified in pancreatic cancers.http:// karchinlab.org/Mutants/ CAN-genes/pancreatic/Pancreatic_cancer.html
23. W. M. Klein, R. H. Hruban, A. J. Klein-Szanto, R. E. Wilentz, Mod Pathol 15, 441 (2002).
24. H.-P. Elsasser, G. Adler, H. F. Kern, in The Pancreas V. L. W. Go et al., Eds. (Raven Press, New York, 1993) pp. 75-86.
25. R. J. Leary et al., Submitted (2008).
26. A. G. Knudson, Am J Med Genet 111, 96 (2002).
27. S. R. Hustinx et al., Mod Pathol 18, 959 (2005).
28. A. Varshavsky, Proc Natl Acad Sci U S A 104, 14935 (2007).
29. G. M. Brodeur, M. D. Hogarty, in The genetic basis of human cancer K. W. Kinzler, B. Vogelstein, Eds. (McGraw-Hill, New York, 1998), vol. 1, pp. 161-179.
30. C. Greenman, R. Wooster, P. A. Futreal, M. R. Stratton, D. F. Easton, Genetics 173, 2187 (2006).
31. S. Ekins, Y. Nikolsky, A. Bugrim, E. Kirillov, T. Nikolskaya, Methods Mol Biol 356, 319 (2007).
32. V. E. Velculescu, L. Zhang, B. Vogelstein, K. W. Kinzler, Science 270, 484 (1995).
33. M. Sultan et al., Science (2008).
34. R. Lister et al., Cell 133, 523 (2008).
35. A. Mortazavi, B. A. Williams, K. McCue, L. Schaeffer, B. Wold, Nat Methods 5, 621 (2008).
36. R. Morin et al., Biotechniques 45, 81 (2008).
37. T. Furukawa et al., Am J Pathol 148, 1763 (1996).
38. H. Ouyang et al., Am J Pathol 157, 1623 (2000).
39. A. H. Owens, D. S. Coffey, S. B. Baylin, Tumor Cell Heterogeneity (Academic Press, New York, 1982), pp.
40. J. Lin et al., Genome Res 17, 1304 (2007).
41. T. Chittenden et al., Genomics 91, 508 (2008).
42. E. Edelman, J. Guinney, J. Chi, P. Febbo, S. Mukherjee, PLoS Computational Biology 4, e28 (2008).
43. D. Hanahan, R. A. Weinberg, Cell 100, 57 (2000).
44. B. Vogelstein, K. W. Kinzler, Nat Med 10, 789 (2004).

### EXAMPLE 9

### Materials and Methods

### Gene Selection

The protein coding exons from 23,781 transcripts representing 20,735 unique genes were targeted for sequencing. This set comprised 14,554 transcripts from the highly curated Consensus Coding Sequence (CCDS) database (http://www.ncbi.nlm.nih.gov/CCDS/), a further 6,019 transcripts from the Reference Sequence (RefSeq) database (http://www.ncbi.nlm.nih.gov/projects/RefSeq/) and an additional 3,208 transcripts with intact open reading frames from the Ensembl database (http://www.ensembl.org/). We excluded transcripts from genes that were located on the Y chromosome or were precisely duplicated within the genome. As detailed below, 23,219 transcripts representing 20,661 genes were successfully sequenced.

### Bioinformatic Resources

Consensus Coding Sequence (Release 1) RefSeq (release 16, March 2006) and Ensembl (release 31) gene coordinates and sequences were acquired from the UCSC Santa Cruz Genome Bioinformatics Site (http://genome.ucsc.edu). The positions listed in the Supplementary Tables correspond to UCSC Santa Cruz hgl7, build 35.1. The single nucleotide polymorphisms used to filter-out known SNPs were those present in dbSNP (release 125) that had been validated by the HapMap project. BLAT and In Silico PCR (http://genome.ucsc.edu/cgi-bin/hgPcr) were used to perform homology searches in the human and mouse genomes.

### Primer Design

Primer 3 software (http://frodo.wi.mit.edu/cgi-bin/primer3/primer3_www.cgi) was used to generate primers no closer than 50 bp to the target boundaries, producing products of 300 to 600 bp . Exons exceeding 350 bp were divided into several overlapping amplicons. In silico PCR and BLAT were used to select primer pairs yielding a single PCR product from a unique genomic position. Primer pairs for duplicated regions giving multiple in silico PCR or BLAT hits were redesigned at positions that were maximally different between the target and duplicated sequences. A universal primer (M13F, 5'-GTAAAACGACGGCCAGT-3'; SEQ ID NO: 1) was added to the 5' end of the primer with the smallest number of mono- or dinucleotide repeats between itself and the target region. The primer sequences used in this study are listed in table S2.

### Tumor Samples

DNA samples from xenografts and cell lines of infiltrating ductal adenocarcinomas and matched normal tissue or peripheral blood were obtained as previously described (1). The 24 samples used for the Discovery Screen included fourteen cell lines and ten xenografts. These were derived from 17 surgically resected carcinomas and seven patients who underwent a rapid autopsy as part of our Gastrointestinal Cancer Rapid Medical Donation Program (GICRMDP). Twenty-two of the carcinomas were primary ductal adenocarcinomas of the pancreas and two were infiltrating adenocarcinomas centered on the intrapancreatic bile duct. We have previously shown that these latter neoplasms are genetically similar to pancreatic adenocarcinoma. The cancers for the Discovery Screen were selected to include advanced stage carcinomas as well as carcinomas that are publically available. Specifically, the Discovery Screen included seven metastatic carcinomas and 15 late stage (stages IIb or IV) surgically resected carcinomas and three cell lines available through the ATCC (Pa14C is Panc8.13, Pa16C is Panc10.05, and Pa18C is Panc5.04). The ninety samples used in the Prevalence Screen included 79 xenografts and 11 cell lines. Cases for the Prevalence Screen were selected to enhance uniformity. Therefore, only infiltrating ductal adenocarcinomas of the pancreas were included. Variants of infiltrating ductal adenocarcinoma (such as colloid carcinoma) and infiltrating ductal adenocarcinomas arising in association with an intraductal papillary mucinous neoplasm were excluded. All samples were obtained in accordance with the Health Insurance Portability and Accountability Act (HIPAA). As previously described, tumor-normal pair matching was confirmed by typing nine STR loci using the PowerPlex 2.1 System (Promega, Madison, WI) and sample identities checked throughout the Discovery and Prevalence screens by sequencing exon 3 of the HLA-A gene. PCR and sequencing was carried out as described in (1).

### Mutation Discovery Screen

CCDS, RefSeq and Ensembl genes were amplified in 24 pancreatic cancer samples and one control samples from normal tissues of an unrelated patient. All coding sequences and the flanking 4 bp were analyzed using Mutations Surveyor (Softgenetics, State College, PA) coupled to a relational database (Microsoft SQL Server). For an amplicon to be further analyzed, at least three quarters of the tumors were required to have 90% or more of bases in the region of interest with a Phred quality score of ≥20. In the amplicons that passed this quality control, mutations identical to those observed in the normal sample as well as known single nucleotide polymorphisms were removed. The sequencing chromatogram of each detected mutation was then visually inspected to remove false positive calls by the software. Every putative mutation was re-amplified and sequenced in tumor DNA to eliminate artifacts. DNA from normal tissues of the same patient in which the mutation was identified was amplified and sequenced to determine whether the mutations were somatic. When a mutation was found, BLAT was used to search the human and mouse genomes for related exons to ensure that putative mutations were the result of amplification of homologous sequences. When there was a similar sequence with 90% identity over 90% of the target region, additional steps were performed. Mutations potentially arising from human duplications were re-amplified using primers designed to distinguish between the two sequences. Mutations not observed using the new primer pair were excluded. The remainder were included as long as the mutant base was not present in the homologous sequence identified by BLAT. Mutations originally observed in mouse xenografts were re-amplified in DNA from primary tumors and included either if the mutation was present in the primary tumors or if the mutant was not identified in the homologous mouse sequence identified by BLAT. For comparison of the number of somatic mutations identified in pancreatic cancers with those identified in breast or colorectal cancers, an independent groups t-test between means was used.

### Mutation Prevalence Screen

A subset of 39 genes which were mutated in two or more tumors in the Discovery Screen was selected for analysis in the Prevalence screen. These genes were amplified and sequenced in a further 90 pancreatic cancers using the primers described in table S2. Mutational analysis, confirmation and determination of somatic status were carried out as described for the Discovery screen using matched normal tissues from the same 90 patients.

### Copy Number Analysis

The Illumina Infinium II Whole Genome Genotyping Assay employing the BeadChip platform was used to analyze tumor samples at 1,072,820 (1M) SNP loci. All SNP positions were based on the hgl8 (NCBI Build 36, March 2006) version of the human genome reference sequence. The genotyping assay begins with hybridization to a 50 nucleotide oligo, followed by a two-color fluorescent single base extension. Fluorescence intensity image files were processed using Illumina BeadStation software to provide normalized intensity values (R) for each SNP position. For each SNP, the normalized experimental intensity value (R) was compared to the intensity values for that SNP from a training set of normal samples and represented as a ratio (called the "Log R Ratio") of log2(Rexperimental/Rtraining set).

The SNP array data were analyzed using modifications of a previously described method (2). Homozygous deletions (HDs) were defined as three or more consecutive SNPs with a Log R Ratio value of ≤ -2. The first and last SNPs of the HD region were considered to be the boundaries of the alteration for subsequent analyses. To eliminate chip artifacts and potential copy number polymorphisms, we removed all HDs that were included in copy number polymorphism databases. Adjacent homozygous deletions separated by three or fewer SNPs were considered to be part of the same deletion, as were HDs within 100,000 bp of each other. To identify the target genes affected by HDs, we compared the location of coding exons in the RefSeq, CCDS and Ensembl databases with the genomic coordinates of the observed HDs. Any gene with a portion of its coding region contained within a homozygous deletion was considered to be affected by the deletion.

As outlined in (2), amplifications were defined by regions containing ≥ three SNPs with an average LogR ratio ≥0.9, with at least one SNP having a LogR ratio ≥1.4. As with HDs, we excluded all putative amplifications that had identical boundaries in multiple samples. As focal amplifications are more likely to be useful in identifying specific target genes, a second set of criteria were used to remove complex amplifications, large chromosomal regions or entire chromosomes that showed copy number gains. Amplifications > 3Mb in size and groups of nearby amplifications (within 1 Mb) that were also ≥ 3Mb in size were considered complex. Amplifications or groups of amplifications that occurred at a frequency of ≥4 distinct amplifications in a 10 Mb region or ≥5 amplifications per chromosome were deemed to be complex. The amplifications remaining after these filtering steps were considered to be focal amplifications and were the only ones included in subsequent statistical analyses. To identify protein coding genes affected by amplifications, we compared the location of the start and stop positions of each gene within the RefSeq, CCDS and Ensmbl databases with the genomic coordinates of the observed amplifications. As amplifications containing only a fraction of a gene are less likely to have a functional consequence, we only considered genes whose entire coding regions were included in the observed amplifications.

### Estimation of passenger mutation rates

From the synonymous mutations observed in the Discovery Screen, we estimated a lower bound of the passenger rate. The lower bound was defined as the product of the synonymous mutation rate and the NS:S ratio (1.02) observed in the HapMap database of human polymorphisms. The calculated rate of 0.54 mutations/Mb successfully sequenced is likely an underestimate because selection against nonsynonymous mutations may be more stringent in the germline than in somatic cells. An upper bound was calculated from the total observed number of non-synonymous mutations/Mb after excluding the most highly mutated genes known to be drivers from previous studies (SMAD4, CDK2NA, TP53, and KRAS). The resultant passenger mutation rate of 1.38 non-synonymous mutations/Mb represents an over-estimate of the background rate as some of the mutations in genes other than SMAD4, CDK2NA, TP53 and KRAS were likely to be drivers. A 'Mid" measure of 0.96 mutations/Mb was obtained from the average of the lower and upper bound rates. For comparisons of the number and type of somatic mutations identified in the Discovery and Prevalence Screens, we used binomial tests for comparison of two proportions as implemented by the function prop.test in the R statistical package.

### Expression Analysis

SAGE tags were generated using a Digital Gene Expression-Tag Profiling preparation kit (Illumina, San Diego, CA) as recommended by the manufacturer. In brief, RNA was purified using guianidine isothiocyanate and reverse transcription with oligo-dT magnetic beads was performed on ~1 ug of total RNA from each sample. Second strand synthesis was accomplished through RNAse H nicking and DNA polymerase I extension. The double-stranded cDNA was digested with the restriction enonuclease Nla III and ligated to an adapter containing a Mme I restriction site. After Mme I digestion, a second adapter was ligated, and the adapter-ligated cDNA construct was enriched by 18 cycles of PCR and fragments of 85 bp were purified from a polyacrylamide gel. The library size was estimated using real-time PCR and the tags sequenced on a Genome Analyzer System (Illumina, San Diego, CA).

### Statistical Analysis

### Overview of Statistical Analysis

The statistical analyses focused on quantifying the evidence that the mutations in a gene or a biologically defined set of genes reflect an underlying mutation rate that is higher than the passenger rate. In both cases, the analysis integrates data on point mutations with data on copy number alterations (CNA). The methodology for the analysis of point mutations is based on that described in (3) while, the methodology for integration across point mutations and CNA's is based on (2). We provide a self-contained summary herein, as several modifications to the previously described methods were required.

### Statistical Analyses of CAN-genes

The mutation profile of a gene refers to the number of each of the twenty-five context-specific types of mutations defined earlier (3). The evidence on mutation profiles is evaluated using an Empirical Bayes analysis (4) comparing the experimental results to a reference distribution representing a genome composed only of passenger genes. This is obtained by simulating mutations at the passenger rate in a way that precisely replicates the experimental plan. Specifically, we consider each gene in turn and simulate the number of mutations of each type from a binomial distribution with success probability equal to the context-specific passenger rate. The number of available nucleotides in each context is the number of successfully sequenced nucleotides for that particular context and gene in the samples studied. When considering nonsynonymous mutations other than indels, we focus on nucleotides at risk, as defined previously (3).

Using these simulated datasets, we evaluated the passenger probabilities for each of the genes that were analyzed in this study. These passenger probabilities represent statements about specific genes rather than about groups of genes. Each passenger probability is obtained via a logic related to that of likelihood ratios: the likelihood of observing a particular score in a gene if that gene is a passenger is compared to the likelihood of observing it in the real data. The gene-specific score used in our analysis is based on the Likelihood Ratio Test (LRT) for the null hypothesis that, for the gene under consideration, the mutation rate is the same as the passenger mutation rate. To obtain a score, we simply transform the LRT to s = log(LRT). Higher scores indicate evidence of mutation rates above the passenger rates. This general approach for evaluating passenger probabilities follows that described by Efron and Tibshirani (4). Specifically, for any given score s, F(s) represents the proportion of simulated genes with scores higher than s in the experimental data, F0 is the corresponding proportion in the simulated data, and p0 is the estimated overall proportion of passenger genes (discussed below). The variation across simulations is small but nonetheless we generated and collated 100 datasets to estimate F0. We then numerically estimated the density functions f and f0 corresponding to F and F0 and calculated, for each score s, the ratio p0·fb(s)/f(s), also known as "local false discovery rate" (4). Density estimation was performed using the function "density" in the R statistical programming language with default settings. The passenger probability calculations depend on an estimate of p0, the proportion of true passengers. Our implementation seeks to give an upper bound to p0 and thus provide conservatively high estimates of the passenger probability. To this end we set p0=1. We also constrained the passenger probability to change monotonically with the score by starting with the lowest values and recursively setting values that decrease in the next value to their right. We similarly constrain passenger probabilities to change monotonically with the passenger rate.

An open source package for performing these calculations in the R statistical environment, named CancerMutationAnalysis, is available at hrip://astor.som.jhmi.edu/∼gp/software/CancerMutationAnalysis/cma.htm. A detailed mathematical account of our specific implementation is provided in (5) and general analytic issues are discussed in (6).

Statistical Analysis of CNA. For each of the genes involved in amplifications or deletions, we further quantified the strength of the evidence that they drive tumorigenesis through estimations of their passenger probabilities. In each case, we obtain the passenger probability as an a posteriori probability that integrates information from the somatic mutation analysis of (3) with the data presented in this article. The passenger probabilities derived from the point mutation analysis serve as a priori probabilities. These are available for three different scenarios of passenger mutation rates and results are presented separately for each in table S3. Then, a likelihood ratio for "driver" versus "passenger" was evaluated using as evidence the number of samples in which a gene was found to be amplified (or deleted). The passenger term is the probability that the gene in question is amplified (or deleted) at the frequency observed. For each sample, we begin by computing the probability that the observed amplifications (and deletions) will include the gene in question by chance. Inclusion of all available SNPs is required for amplification, while any overlap of SNPs is sufficient for deletions. Specifically, if in a specific sample N SNPs are typed, and K amplifications are found, whose sizes, in terms of SNPs involved, are A1 ... AK, a gene with G SNPs will be included at random with probability (A1-G+1)/N + .... + (AK-G+1)/N for amplifications and (A1+G-1)/N + .... + (AK+G-1)/N for deletions. We then compute the probability of the observed number of amplifications (or deletions) assuming that the samples are independent but not identically distributed Bernoulli random variables, using the Thomas and Traub algorithm (7). Our approach to evaluating the likelihood under the null hypothesis is highly conservative, as it assumes that all the deletions and amplifications observed only include passengers. The driver term of the likelihood ratio was approximated as for the passenger term, after multiplying the sample-specific passenger rates above by a gene-specific factor reflecting the increase (alternative hypothesis) of interest. This increase is estimated by the ratio between the empirical deletion rate of the gene and the overall deletion rate.

This combination approach makes an approximating assumption of independence of amplifications and deletions. In reality, amplified genes cannot be deleted, so independence is technically violated. However, because of the relatively small number of amplification and deletion events, this assumption is tenable for the purposes of our analysis. Inspection of the likelihood, in a logarithmic scale, suggests that it is roughly linear in the overall number of events, supporting the validity of this approximation as a scoring system.

### Analysis of mutated gene pathways and groups

Four types of data were obtained from the MetaCore database (GeneGo, Inc., St. Joseph, MI): pathway maps, Gene Ontology (GO) processes, GeneGo process networks, and protein-protein interactions. The memberships of each of the 23,781 transcripts in these categories were retrieved from the databases using RefSeq identifiers. In GeneGo pathway maps, 22,622 relations were identified, involving 4,175 transcripts and 509 pathways. For Gene Ontology processes, a total of 66,397 pairwise relations were identified, involving 12,373 transcripts and 4,426 GO groups. For GeneGo process networks, a total of 23,356 pairwise relationships, involving 6,158 transcripts and 127 processes, were identified. The predicted protein products of each mutated gene were also evaluated with respect to their physical interactions with proteins encoded by other mutated genes as inferred from the MetaCore database.

For each of the gene sets considered, we quantified the strength of the evidence that they included a higher-than-average proportion of drivers of carcinogenesis after consideration of set size. For this purpose, we sorted the genes by a score based on the combined passenger probability described above (taking into account mutations, homozygous deletions, and amplifications). We compared the ranking of the genes contained in the set with the ranking of those outside, using the Wilcoxon test, as implemented by the Limma package in Bioconductor (8), then corrected for multiplicity by the q-value method with an alpha of 0.2 (9). We similarly quantified the strength of the evidence that gene sets included a higher-than-average proportion of genes that were expressed differentially, compared to normal pancreatic duct cells, from the SAGE data. For comparison of the expression q-values of gene sets enriched for combined genetic alterations vs. other gene sets, we used an independent groups t-test between means.

### Bioinformatic Analysis

### Overview of Bioinformatic Analysis

We have developed a novel bioinformatics software pipeline (depicted below) to compute a score (LS-Mut) for ranking somatic missense mutations by the likelihood that they are passengers. The scores are based on properties derived from protein sequences, amino acid residue changes and positions within the proteins. As part of this pipeline, we have also developed qualitative annotations of each mutation based on protein structure homology models.

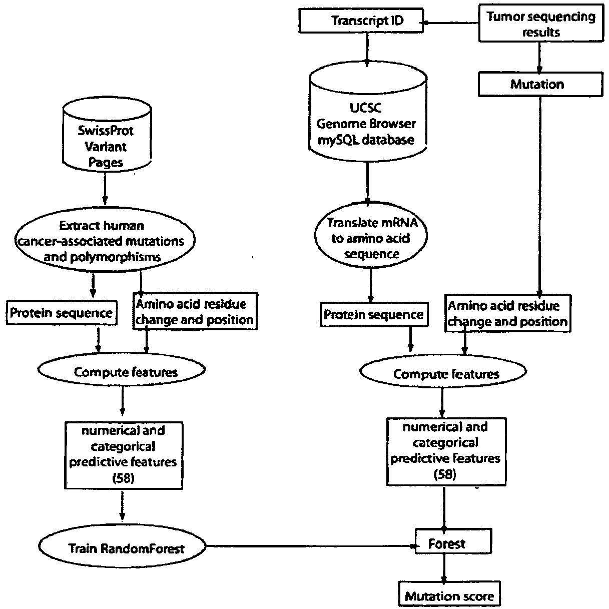

### Mutation Scores

We tested several supervised machine learning algorithms to identify one that would reliably distinguish between presumably neutral polymorphisms and cancer-associated mutations. The best algorithm was a Random Forest (11), which we trained on 2,840 cancer-associated mutations and 19,503 polymorphisms from the SwissProt Variant Pages (12) using parallel Random Forest software (PARF) [http://www.irb.hr/en/cir/projects/info/parf]. Cancer-associated mutations were identified by parsing for the keywords "cancer", "carcinoma", "sarcoma", "blastoma", "melanoma", "lymphoma", "adenoma" and "glioma". For each mutation or polymorphism, we computed 58 numerical and categorical features (see table below). Because the training set contained ~7 times as many polymorphisms as cancer- associated mutations, we used class weights to up-weight the minority class (cancer-associated mutation weight was 5.0 and polymorphism weight was 1.0). The mtry parameter was set to 8 and the forest size to 500 trees. Missing feature values were filled in using the Random Forest proximity-based imputation algorithm (13) with six iterations. Full parameter settings and all data used to build the RandomForest are available upon request.

We then applied the trained forest to 906 different pancreatic missense mutations and to a control set of 142 randomly generated missense mutations in transcripts of 78 genes that were found to be non-mutated in 11 colorectal cancers (2). For each mutation, the 58 predictive features were computed as described above and the trained forest was used to compute a predictive score for ranking the mutations. Specifically, the scores used are the fraction of trees that voted in favor of the "Polymorphic" class for each mutation.

To test the hypothesis that the scores of missense mutations in top-ranked CAN-genes in pancreatic cancers were distributed differently than random missense mutations, we applied a modified Kolmogorov-Smirnov (KS) test, in which ties are broken by adding a very small random number to each score. The scores of missense mutations in the top 32 pancreatic CAN- genes were found to be significantly different from the mutations in the control set (P<0.001).

Based on these comparisons, we estimate that mutations with scores £0.7 (~17% of the missense mutations in pancreatic cancers) are unlikely to be passengers. The threshold is based on the putative similarity of passengers to the neutral polymorphisms in the SwissProt Variant set, of which only ~2% have scores £0.7. To compute unbiased scores for the SwissProt variants that could be used to threshold the pancreatic cancer mutation scores, we randomly partitioned the 22,343 variants into two folds and trained a RandomForest on each (as described above). The variants in each fold were then scored by the RandomForest trained on the other fold.

### Homology Models

The protein translations of mRNA transcripts found to have somatic missense mutations were input into ModPipe 1.0/MODELLER 9.1 homology model building software (13). For each mutation, we identified all models that included the mutated position. If more than one model was produced for a mutation, we selected the model having the highest sequence identity with its template structure. The resulting model was used to compute the solvent accessibility of the wild type residue at the mutated position, using DSSP software (*14*). Accessibility values were normalized by dividing by the maximum residue solvent accessibility for each side chain type in a Gly-X-Gly tri-peptide (*15*). Solvent accessibilities greater than 36% were considered to be "exposed", those between 9% and 35% were considered "intermediate", and those < 9% were considered "buried". DSSP was also used to compute the secondary structure of the mutated position. We used the LigBase (*15*) and PiBase (16) databases to identify mutated residue positions in the homology models that were close to ligands or domain interfaces in the equivalent positions of their template structures. Finally, for each mutation, we generated an image of the mutation mapped onto its homology model with UCSF Chimera (*17*). The images and associated information for each mutation are available at http://karchinlab.org/Mutants/CAN-genes/pancreatic/Pancreatic cancer.html. Model coordinates are available on request.

**The 56 numerical and categorical features used to train the Random Forest**

| **#** | **Feature** | **Description** |
|---|---|---|
| **1** | residue charge change Net | The change in formal charge resulting from the mutation. |
| **2** | residue volume change | The change in residue volume resulting from the mutation (*20*). |
| **3** | Net residue hydrophobicity change | The change in residue hydrophobicity resulting from the substitution (*21*) |
| **4** | Positional Hidden Markov model (HMM) conservation score | This feature is calculated based on the degree of conservation of the residue estimated from a multiple sequence alignment built with SAM-T2K software (*22*), using the protein in which the mutation occurred as the seed sequence (*23*). The SAM-T2K alignments are large, superfamily-level alignments that include distantly related homologs (as well as close homologs and orthologs) of the protein of interest. |
| **5** | Entropy of HMM alignment | The Shannon entropy calculated for the column of the SAM-T2K multiple sequence alignment, corresponding to the location of the mutation (*24*). |
| **6** | Relative entropy of HMM alignment | Difference in Shannon entropy calculated for the column of the SAM-T2K multiple sequence alignment (corresponding to the location of the mutation) and that of a background distribution of amino acid residues computed from a large sample of multiple sequence alignments (*24*) |
| **7** | Compatibility score for amino acid substitution in the column of a multiple sequence alignment of orthologs. | These multiple sequence alignments are calculated using groups of orthologous proteins from the OMA database (*25*), which are aligned with T-Coffee software (*26*). The compatibility score for the mutation in the column of interest is computed as: (P(most frequent residue in the column) - 2*P(wild type) + P(mutant) + P(Deletion) - 1) / (5 * number of unique amino acid residues in the column) |
| **8** | Grantham score | The Grantham substitution score for the wild type => mutant transition (*27*). |
| **9-11** | Predicted residue solvent accessibility | These features consist of the probability of the wild type residue being buried, intermediate or exposed as predicted by a neural network trained with Predict-2nd software (*22*) on a set of 1763 proteins with high-resolution X-ray crystal structures sharing less than 30% homology (*28*). |
| **12-14** | Predicted contribution to protein stability | These features consist of the probability that the wild type residue contributes to overall protein stability in a manner that is highly stabilizing, average or destabilizing, as predicted by a neural network trained with Predict-2nd software (*22*) on a set of 1763 proteins with less than 30% homology. Stability estimates for the neural net training data were calculated using the FoldX force field (*29*). |
| **15-17** | Predicted flexibility (Bfactor) | These features consist of the probability that the wild type residue backbone is stiff, intermediate or flexible as predicted by a neural network trained with Predict-2nd software (*22*) on a set of 1763 proteins with less than 30% homology. Flexibilities for the neural net training data were estimated based on normalized temperature factors, computed using the method of (*30*) from the X-ray crystal structure files. |
| **18-20** | Predicted secondary structure | These features consist of the probability that the secondary structure of the region in which the wild type residue exists is helix, loop or strand as predicted by a neural net trained with Predict-2nd software (*22*)on a set of 1763 proteins with crystal structures and with less than 30% homology. |
| **21** | Change in hydrophobicity | Change in residue hydrophobicity due to the wild type → mutant transition. |
| **22** | Change in volume | Change in residue volume due to the wildtype → mutant transition. |
| **23** | Change in charge | Change in residue formal charge due to the wild type -> mutant transition. |
| **24** | Change in polarity | Change in residue polarity due to the wildtype → mutant transition. |
| **25** | EX substitution score | Amino acid substitution score from the EX matrix (*31*) |
| **26** | PAM250 substitution score | Amino acid substitution score from the PAM250 matrix (32) |
| **27** | BLOSUM 62 substitution score | Amino acid substitution score from the BLOSUM 62 matrix (33) |
| **28** | MJ substitution score | Amino acid substitution score from the Miyazawa-Jernigan contact energy matrix (*31*, *34*) |
| **29** | HGMD2003 mutation count | Number of times that the wild type → mutant substitution occurs in the Human Gene Mutation Database, 2003 version (31, 35). |
| **30** | VB mutation count | Amino acid substitution score from the VB (Venkatarajan and Braun) matrix (*31, 36*) |
| **31-34** | Probability of seeing the wildtype residue in the first, middle, or last position of an amino acid triple | Calculated by joint frequencies of amino acid triples in human proteins found in UniProtKB (*12*) |
| **35-37** | Probability of seeing the mutant residue in the first, middle, or last position of an amino acid triple | Calculated by joint frequencies of amino acid triples in human proteins found in UniProtKB (*12*) |
| **38-40** | Difference in probability of seeing the wildtype vs. the mutant residue in the first, middle, or last position of an amino acid triple | Calculated by joint frequencies of amino acid triples in human proteins found in UniProtKB (*12*) |
| **41** | Probability of seeing the wildtype at the center of a window of 5 amino acid residues | Calculated by a Markov chain of amino acid quintuples in human proteins found in UniProtKB (*12*). |
| **42** | Probability of seeing the mutant at the center of a window of 5 amino acid residues | Calculated by a Markov chain of amino acid quintuples in human proteins found in UniProtKB (*12*). |
| **43-56** | Binary categorical features from the UniProt KnowledgeBase (*12*) feature table for the protein product of the transcript | These features give annotations, curated from the literature, of general binding sites, general active sites, lipid, metal, carbohydrate, DNA, phosphate and calcium binding sites, disulfides, seleno-cysteines, modified residues, propeptide residues, signal peptide residues, known mutagenic sites, transmembrane regions, compositionally biased regions, repeat regions, known motifs, and zinc fingers. The integer 1 indicates that a feature is present and the integer 0 indicates that it is absent at a mutated position. |
| **57** | Count of missense changes at or close to the mutated position | Count of missense changes seen in a window of ±5 residues in linear sequence around (and including) the mutated position. For mutants from the SwissProt Variant Pages, counts taken from the SwissProt variant pages. For mutants in potential CAN-genes, counts taken from somatic mutations in colorectal, glioblastoma and pancreatic tumors (*1*, *2*, *37*)*.* |
| **58** | Frequency of missense change type in the Catalogue of Somatic Mutations in Cancer (COSMIC) database (38), | Frequency that missense change type (amino acid type X to amino acid type Y, e.g. ALANINE to GLYCINE) is seen in COSMIC. These frequencies were calculated during the week of August 14, 2008, using COSMIC release 38. |

### References for Example 9 only

1. T. Sjoblom et al., Science 314, 268 (2006).
2. R. J. Leary et al., Submitted (2008).
3. L. D. Wood et al., Science 318, 1108 (2007).
4. B. Efron, R. Tibshirani, Genet Epidemiol 23, 70 (2002).
5. G. Parmigiani et al., "Statistical Methods for the Analysis of Cancer Genome Sequencing Data" (Johns Hopkins University, 2006).
6. G. Parmigiani et al., Genomics in press (2008).
7. M. A. Thomas, A. E. Taub, Journal of Statistical Computation and Simulation 14, 125 (1982).
8. G. K. Smyth, in Bioinformatics and Computational Biology Solutions using R and Bioconductor V. Gentleman, S. Carey, R. Dudoit, W. H. Irizarry, Eds. (Springer, New York, 2005) pp. 397-420.
9. Y. Benjamini, Y. Hochberg, Journal of the Royal Statistical Society. Series B (Methodological) 57 289-300 (1995).
10. L. Breiman, Machine Learning, 5 (2001).
11. C. H. Wu et al., Nucleic Acids Res 34, D 187 (2006).
12. R. Karchin et al., Bioinformatics 21, 2814 (2005).
13. A. Sali, T. L. Blundell, Journal of Molecular Biology 234, 779 (1993).
14. G. D. Rose, A. R. Geselowitz, G. J. Lesser, R. H. Lee, M. H. Zehfus, Science 229, 834 (1985).
15. A. C. Stuart, V. A. Ilyin, A. Sali, Bioinformatics 18, 200 (2002).
16. F. P. Davis, A. Sali, Bioinformatics 21, 1901 (2005).
17. E. F. Pettersen et al., J Comput Chem 25, 1605 (2004).
18. A. A. Zamyatnin, Prog Biophys Mol Biol, 107 (1972).
19. D. M. Engelman, T. A. Steitz, A. Goldman, Annu Rev Biophys Biophys Chem 15,321 (1986).
20. K. Karplus et al., Proteins Suppl 5, 86 (2001).
21. S. Kullback, Information theory and statistics (Wiley, New York, 1959), pp.
22. A. Schneider, C. Dessimoz, G. H. Gonnet, Bioinformatics 23, 2180 (2007).
23. C. Notredame, D. G. Higgins, J. Heringa, J Mol Biol 302,205 (2000).
24. R. Grantham, Science 185, 862 (1974).
25. G. Wang, R. L. Dunbrack, Jr., Bioinformatics 19, 1589 (2003).
26. J. Schymkowitz et al., Nucleic Acids Res 33, W382 (2005).
27. D. K. Smith, P. Radivojac, Z. Obradovic, A. K. Dunker, G. Zhu, Protein Sci 12, 1060 (2003).
28. L. Y. Yampolsky, A. Stoltzfus, Pac Symp Biocomput, 433 (2005).
29. R. M. Schwartz, M. O. Dayhoff, Science 199,395 (1978).
30. S. Henikoff, J. G. Henikoff, Proc Natl Acad Sci U S A 89, 10915 (1992).
31. S. Miyazawa, and Jernigan, R. L., Macromolecules, 534 (1985).
32. P. D. Stenson et al., Hum Mutat 21, 577 (2003).
33. M. S. Venkatarajan, and Braun, W., Journal of Molecular Modeling, 445 (2001).

### EXAMPLE 10

There is considerable debate about the value of personal genome sequencing (1). In addition to the five individuals whose genomes have been sequenced in their entirety, 68 patients have been evaluated for tumor-specific mutations in all exons of protein coding genes (exomic sequencing). This coincidentally yielded information about germline sequence variations in these individuals (2-4). To explore the utility of such information, we evaluated a pancreatic cancer patient (Pa10) whose tumor DNA had been sequenced in (4). This patient had familial pancreatic cancer, as defined by the fact that his sister also had developed the disease.

Among the 20,661 coding genes analyzed, we identified 15,461 germline variants in Pa10 not found in the reference human genome. Of these, 7318 were synonymous, 7721 were missense, 64 were nonsense, 108 were at splice sites, and 250 were small deletions or insertions (54% in-frame). Past studies have shown that tumors arising in patients with a hereditary predisposition harbor no normal alleles of the responsible gene: one allele is inherited in mutant form, often producing a stop codon, and the other (wild type) allele is inactivated by somatic mutation during tumorigenesis. In Pa10, only three genes met these criteria: SERPINB12, RAGE and PALB2. Of these, we considered PALB2 to be the best candidate because germline stop codons in SERPINB12 and RAGE, but not in PALB2, are relatively common in healthy individuals and because germline PALB2 mutations have previously been associated with breast cancer predisposition and Fanconi anemia(5) although its function is not well understood. Pa10 harbored a germline deletion of 4 bp (TTGT at c.172-175) producing a frameshift at codon 58; the pancreatic cancer that developed in Pa10 had also somatically acquired a transition mutation (C to T) at a canonical splice site for exon 10 (IVS10+2).

To determine whether PALB2 mutations occur in other patients with familial pancreatic cancer, we sequenced this gene in a cohort of 96 familial pancreatic cancer patients, 90 of which were of Caucasian ancestry. Sixteen of these patients had one first degree relative with pancreatic cancer and 80 had at least two additional relatives, at least one of which was first degree, with the disease. Truncating mutations were identified in three of the 96 patients, each producing a different stop codon (Fig. 1). The average age-of-onset of pancreatic cancer in these families was 66.7 years, similar to the mean age of onset of 65.3 years in the families without PALB2 mutations. We determined the germ-line sequence of an affected brother in one of these kindreds, and he harbored the same stop codon. Truncating mutations in PALB2 are rare in individuals without cancer; none have been reported among 1,084 normal individuals in a previous study using a cohort of similar ethnicity to ours (6). While some families we identified with a PALB2 stop mutation had a history of both breast and pancreatic cancer, breast cancer was not observed in all families. From these data, PALB2 appears to be the second most commonly mutated gene for hereditary pancreatic cancer. Interestingly, the most commonly mutated gene is BRCA2 (7), whose protein product is a binding partner for the PALB2 protein (8).

In summary, through complete, unbiased sequencing of protein-coding genes, we have discovered a gene responsible for a hereditary disease. We note that this approach is independent of classical methods for gene discovery, such as linkage analysis, which can be challenging in the absence of large families with monogenic diseases. We predict that variations of the approach described here will soon become a standard tool for the discovery of disease-related genes.

### References (for EXAMPLE 10 only)

1. A. L. McGuire, M. K. Cho, S. E. McGuire, T. Caulfield, Science 317, 1687 (2007).
2. L. D. Wood et al., Science 318, 1108 (2007).
3. D. W. Parsons et al., Science 321, 1807 (2008).
4. S. Jones et al., Science 321, 1801 (2008).
5. C. Turnbull, N. Rahman, Annu Rev Genomics Hum Genet 9, 321 (2008).
6. N. Rahman et al., Nat Genet 39, 165 (2007).
7. A. Maitra, R. H. Hruban, Annu Rev Pathol 3, 157 (2008).
8. B. Xia et al., Mol Cell 22, 719 (2006).

## Claims

1. A method of determining susceptibility to pancreatic cancer, comprising:
testing a sample obtained from an individual for the presence of a mutation in the *PALB2* gene found in a family member of the individual;
identifying the individual as being at increased risk of developing pancreatic cancer when the mutation is present and identifying the individual as being at normal risk when the mutation is not present, and wherein the mutation is a truncating mutation.

2. The method of claim 1 wherein the testing comprises a step of hybridization of a nucleic acid probe or primer to a *PALB2* gene or transcript.

3. A method of determining susceptibility to pancreatic cancer in an individual, comprising:
performing sequencing reactions of *PALB2* gene sequences on template nucleic acid obtained from the individual;
identifying a mutation in the *PALB2* sequences, whereby the individual is identified as at increased susceptibility to pancreatic cancer, and wherein the mutation is a truncating mutation.

4. A method of determining susceptibility to pancreatic cancer in an individual, comprising:
hybridizing a nucleic acid primer or probe comprising a *PALB2* sequence of at least 18 nucleotides wherein the sequence comprises a mutation selected from the group consisting of: del TTGT 172-175, G>T at IVS5-1, del A at 3116, and C>T at 3256, to *PALB2* gene sequences on nucleic acid obtained from the individual;
identifying one of said mutations in the *PALB2* sequences, whereby the individual is identified as at increased susceptibility to pancreatic cancer.

5. A method comprising:
detecting in a pancreatic cancer sample obtained from a patient a mutation in the *PALB2* gene, wherein the mutation is a truncating mutation.

6. A method of diagnosing a predisposition to pancreatic cancer in patient, comprising:
detecting in a sample obtained from an individual presence of a germline mutation in the PALB2 gene, wherein the presence of the germline mutation indicates a predisposition to pancreatic cancer, and wherein the mutation is a truncating mutation.

7. The method of claim 6, wherein said detecting step comprises analyzing a genomic DNA in said sample.

8. The method of claim 6, wherein said individual is diagnosed with or suspected of having pancreatic cancer, or has a family history of pancreatic cancer.

## Patentansprüche

1. Ein Verfahren zum Bestimmen der Empfindlichkeit auf Pankreaskrebs, umfassend:
Testen einer aus einem Individuum erhaltenen Probe auf das Vorliegen einer Mutation in dem *PALB2*-Gen, welches in einem Familienmitglied des Individuums gefunden wird;
Identifizieren des Individuums als einem erhöhten Risiko für das Entwickeln eines Pankreaskrebses unterliegend, wenn die Mutation vorliegt, und Identifizieren des Individuums als einem Normalrisiko unterliegend, wenn die Mutation nicht vorliegt und wenn die Mutation eine trunkierende Mutation ist.

2. Das Verfahren nach Anspruch 1, wobei das Testen einen Hybridisierungsschritt einer Nukleinsäuresonde oder eines Primers an ein *PALB2*-Gen oder -Transkript umfasst.

3. Ein Verfahren zum Bestimmen der Empfindlichkeit auf Pankreaskrebs in einem Individuum, umfassend:
Durchführen von Sequenzierungsreaktionen von *PALB2*-Gensequenzen an Matrizennukleinsäure, welche von dem Individuum erhalten wird;
Identifizieren einer Mutation in den *PALB2*-Sequenzen, wodurch das Individuum identifiziert wird als einer erhöhten Empfindlichkeit auf Pankreaskrebs unterliegend und wobei die Mutation eine trunkierende Mutation ist.

4. Ein Verfahren zum Bestimmen der Empfindlichkeit auf Pankreaskrebs in einem Individuum, umfassend:
Hybridisieren eines Nukleinsäureprimers oder einer Sonde, welche eine *PALB2-*Sequenz von wenigstens 18 Nukleotiden umfasst, wobei die Sequenz eine Mutation umfasst, welche ausgewählt ist aus der Gruppe bestehend aus: del TTGT 172-175, G>T bei IVS5-1, del A bei 3116 und C>T bei 3256 an *PALB2*-Gensequenzen von Nukleinsäure, welche aus dem Individuum erhalten wird;
Identifizieren einer der Mutationen in den *PALB2*-Sequenzen, wodurch das Individuum als erhöhte Empfindlichkeit auf Pankreaskrebs identifiziert wird.

5. Ein Verfahren, umfassend:
Erfassen einer Mutation in dem *PALB2*-Gen in einer Pankreaskrebsprobe, welche aus einem Patienten erhalten wird, wobei die Mutation eine trunkierende Mutation ist.

6. Ein Verfahren zum Diagnostizieren einer Prädisposition für Pankreaskrebs in einem Patienten, umfassend:
Erfassen des Vorliegens einer Keimbahnmutation in dem *PALB2*-Gen in einer Probe, welche aus einem Individuum erhalten wird, wobei das Vorliegen der Keimbahnmutation eine Prädisposition für Pankreaskrebs anzeigt und wobei die Mutation eine trunkierende Mutation ist.

7. Das Verfahren nach Anspruch 6, wobei der Erfassungsschritt das Analysieren einer genomischen DNA in der Probe umfasst.

8. Das Verfahren nach Anspruch 6, wobei das Individuum mit Pankreaskrebs diagnostiziert wird oder Verdacht auf Pankreaskrebs diagnostiziert wird oder Pankreaskrebs in der Familienanamnese zeigt.

## Revendications

1. Procédé permettant de déterminer la susceptibilité au cancer pancréatique, comprenant :
l'analyse d'un échantillon obtenu d'un individu pour la présence d'une mutation dans le gène *PALB2* trouvée chez un membre de la famille de l'individu ;
l'identification de l'individu comme présentant un risque accru de développer un cancer pancréatique lorsque la mutation est présente et l'identification de l'individu comme présentant un risque normal lorsque la mutation n'est pas présente, et dans lequel la mutation est une mutation de troncature.

2. Procédé selon la revendication 1, dans lequel l'analyse comprend une étape d'hybridation d'une sonde ou d'une amorce d'acide nucléique à un gène *PALB2* ou à un transcrit du gène *PALB2.*

3. Procédé permettant de déterminer la susceptibilité au cancer pancréatique chez un individu, comprenant :
la réalisation de réactions de séquençage des séquences du gène *PALB2* sur l'acide nucléique matrice obtenu de l'individu ;
l'identification d'une mutation dans les séquences de *PALB2,* grâce à laquelle l'individu est identifié comme présentant une susceptibilité accrue au cancer pancréatique, et dans lequel la mutation est une mutation de troncature.

4. Procédé permettant de déterminer la susceptibilité au cancer pancréatique chez un individu, comprenant :
l'hybridation d'une amorce ou d'une sonde d'acide nucléique comprenant une séquence de *PALB2* d'au moins 18 nucléotides dans lequel la séquence comprend une mutation choisie dans le groupe constitué de : del TTGT 172-175, G>T en IVS5-1, del A en 3116, et C>T en 3256, aux séquences du gène *PALB2* sur l'acide nucléique obtenu de l'individu ;
l'identification de l'une desdites mutations dans les séquences de *PALB2,* grâce à laquelle l'individu est identifié comme présentant une susceptibilité accrue au cancer pancréatique.

5. Procédé comprenant :
la détection dans un échantillon de cancer pancréatique obtenu d'un patient d'une mutation dans le gène *PALB2,* dans lequel la mutation est une mutation de troncature.

6. Procédé de diagnostic d'une prédisposition au cancer pancréatique chez un patient, comprenant :
la détection dans un échantillon obtenu d'un individu de la présence d'une mutation de la lignée germinale dans le gène *PALB2,* dans lequel la présence de la mutation de la lignée germinale indique une prédisposition au cancer pancréatique, et dans lequel la mutation est une mutation de troncature.

7. Procédé selon la revendication 6, dans lequel ladite étape de détection comprend l'analyse d'un ADN génomique dans ledit échantillon.

8. Procédé selon la revendication 6, dans lequel ledit individu est diagnostiqué avec ou suspecté d'avoir un cancer pancréatique, ou a des antécédents familiaux de cancer pancréatique.
